# EUROPEAN PATENT APPLICATION

(11) **EP 3 929 292 A1**
(43) Date of publication of application: **29.12.2021**
(21) Application number: 18938930.7
(22) Date of filing: 30.11.2018
(51) Int. Cl.: C12N 15/113, C12N 15/85, C12N 15/90, A61K 48/00, A61P 35/00, A61P 29/00, A61P 25/16, A61P 9/00, A61P 25/28, A61P 25/00, A61P 25/34, A61P 27/02, A61P 25/18

(54) **SYSTEM AND METHOD FOR GENOME EDITING BASED ON C2C1 NUCLEASES**

(30) Priority: 02.11.2018 CN 201811300251
(71) Applicant: Institute Of Zoology, Chinese Academy Of Sciences, Beijing 100101 (CN)
(72) Inventor: LI, Wei, Beijing 100101 (CN); ZHOU, Qi, Beijing 100101 (CN); TENG, Fei, Beijing 100101 (CN)
(74) Representative: Michalski Hüttermann & Partner Patentanwälte mbB
(86) International application number: PCT/CN2018/118458
(87) International publication number: WO 2020/087631

(57) **Abstract**

The invention relates to the field of genetic engineering. In particular, the present invention relates to a genome editing system and method based on C2c1 nuclease. The present invention also relates to artificial guide RNA which can be combined with the C2c1 nuclease and used for genomic editing

Sequence Listing

<110>  INSTITUTE OF ZOOLOGY, CHINESE ACADEMY OF SCIENCES

<120>  System and method for genome editing based on C2c1 nuclease

<130>  P2018TC640

<160>  80

<170>  PatentIn version 3.5

<210>  1
<211>  1129
<212>  PRT
<213>  Alicyclobacillus acidiphilus

<400>  1

Met Ala Val Lys Ser Met Lys Val Lys Leu Arg Leu Asp Asn Met Pro
1               5                   10                  15

Glu Ile Arg Ala Gly Leu Trp Lys Leu His Thr Glu Val Asn Ala Gly
            20                  25                  30

Val Arg Tyr Tyr Thr Glu Trp Leu Ser Leu Leu Arg Gln Glu Asn Leu
        35                  40                  45

Tyr Arg Arg Ser Pro Asn Gly Asp Gly Glu Gln Glu Cys Tyr Lys Thr
    50                  55                  60

Ala Glu Glu Cys Lys Ala Glu Leu Leu Glu Arg Leu Arg Ala Arg Gln
65                  70                  75                  80

Val Glu Asn Gly His Cys Gly Pro Ala Gly Ser Asp Asp Glu Leu Leu
                85                  90                  95

Gln Leu Ala Arg Gln Leu Tyr Glu Leu Leu Val Pro Gln Ala Ile Gly
            100                 105                 110

Ala Lys Gly Asp Ala Gln Gln Ile Ala Arg Lys Phe Leu Ser Pro Leu
        115                 120                 125

Ala Asp Lys Asp Ala Val Gly Gly Leu Gly Ile Ala Lys Ala Gly Asn
    130                 135                 140

Lys Pro Arg Trp Val Arg Met Arg Glu Ala Gly Glu Pro Gly Trp Glu
145                 150                 155                 160

Glu Glu Lys Ala Lys Ala Glu Ala Arg Lys Ser Thr Asp Arg Thr Ala
                165                 170                 175

Asp Val Leu Arg Ala Leu Ala Asp Phe Gly Leu Lys Pro Leu Met Arg
        180                 185                 190

Val Tyr Thr Asp Ser Asp Met Ser Ser Val Gln Trp Lys Pro Leu Arg
    195                 200                 205

Lys Gly Gln Ala Val Arg Thr Trp Asp Arg Asp Met Phe Gln Gln Ala
    210                 215                 220

Ile Glu Arg Met Met Ser Trp Glu Ser Trp Asn Gln Arg Val Gly Glu
225                 230                 235                 240

Ala Tyr Ala Lys Leu Val Glu Gln Lys Ser Arg Phe Glu Gln Lys Asn
                245                 250                 255

Phe Val Gly Gln Glu His Leu Val Gln Leu Val Asn Gln Leu Gln Gln
            260                 265                 270

Asp Met Lys Glu Ala Ser His Gly Leu Glu Ser Lys Glu Gln Thr Ala
        275                 280                 285

His Tyr Leu Thr Gly Arg Ala Leu Arg Gly Ser Asp Lys Val Phe Glu
    290                 295                 300

Lys Trp Glu Lys Leu Asp Pro Asp Ala Pro Phe Asp Leu Tyr Asp Thr
305                 310                 315                 320

Glu Ile Lys Asn Val Gln Arg Arg Asn Thr Arg Arg Phe Gly Ser His
                325                 330                 335

Asp Leu Phe Ala Lys Leu Ala Glu Pro Lys Tyr Gln Ala Leu Trp Arg
            340                 345                 350

Glu Asp Ala Ser Phe Leu Thr Arg Tyr Ala Val Tyr Asn Ser Ile Val
        355                 360                 365

Arg Lys Leu Asn His Ala Lys Met Phe Ala Thr Phe Thr Leu Pro Asp
    370                 375                 380

Ala Thr Ala His Pro Ile Trp Thr Arg Phe Asp Lys Leu Gly Gly Asn
385                 390                 395                 400

Leu His Gln Tyr Thr Phe Leu Phe Asn Glu Phe Gly Glu Gly Arg His
                405                 410                 415

Ala Ile Arg Phe Gln Lys Leu Leu Thr Val Glu Asp Gly Val Ala Lys
            420                 425                 430

Glu Val Asp Asp Val Thr Val Pro Ile Ser Met Ser Ala Gln Leu Asp
        435                 440                 445

Asp Leu Leu Pro Arg Asp Pro His Glu Leu Val Ala Leu Tyr Phe Gln
    450                 455                 460

Asp Tyr Gly Ala Glu Gln His Leu Ala Gly Glu Phe Gly Gly Ala Lys
465                 470                 475                 480

Ile Gln Tyr Arg Arg Asp Gln Leu Asn His Leu His Ala Arg Arg Gly
                485                 490                 495

Ala Arg Asp Val Tyr Leu Asn Leu Ser Val Arg Val Gln Ser Gln Ser
            500                 505                 510

Glu Ala Arg Gly Glu Arg Arg Pro Pro Tyr Ala Ala Val Phe Arg Leu
        515                 520                 525

Val Gly Asp Asn His Arg Ala Phe Val His Phe Asp Lys Leu Ser Asp
    530                 535                 540

Tyr Leu Ala Glu His Pro Asp Asp Gly Lys Leu Gly Ser Glu Gly Leu
545                 550                 555                 560

Leu Ser Gly Leu Arg Val Met Ser Val Asp Leu Gly Leu Arg Thr Ser
                565                 570                 575

Ala Ser Ile Ser Val Phe Arg Val Ala Arg Lys Asp Glu Leu Lys Pro
            580                 585                 590

Asn Ser Glu Gly Arg Val Pro Phe Cys Phe Pro Ile Glu Gly Asn Glu
        595                 600                 605

Asn Leu Val Ala Val His Glu Arg Ser Gln Leu Leu Lys Leu Pro Gly
    610                 615                 620

Glu Thr Glu Ser Lys Asp Leu Arg Ala Ile Arg Glu Glu Arg Gln Arg
625                 630                 635                 640

Thr Leu Arg Gln Leu Arg Thr Gln Leu Ala Tyr Leu Arg Leu Leu Val
                645                 650                 655

Arg Cys Gly Ser Glu Asp Val Gly Arg Arg Glu Arg Ser Trp Ala Lys
            660                 665                 670

Leu Ile Glu Gln Pro Met Asp Ala Asn Gln Met Thr Pro Asp Trp Arg
        675                 680                 685

Glu Ala Phe Glu Asp Glu Leu Gln Lys Leu Lys Ser Leu Tyr Gly Ile
    690                 695                 700

Cys Gly Asp Arg Glu Trp Thr Glu Ala Val Tyr Glu Ser Val Arg Arg
705                 710                 715                 720

Val Trp Arg His Met Gly Lys Gln Val Arg Asp Trp Arg Lys Asp Val
                725                 730                 735

Arg Ser Gly Glu Arg Pro Lys Ile Arg Gly Tyr Gln Lys Asp Val Val
            740                 745                 750

Gly Gly Asn Ser Ile Glu Gln Ile Glu Tyr Leu Glu Arg Gln Tyr Lys
        755                 760                 765

Phe Leu Lys Ser Trp Ser Phe Phe Gly Lys Val Ser Gly Gln Val Ile
    770                 775                 780

Arg Ala Glu Lys Gly Ser Arg Phe Ala Ile Thr Leu Arg Glu His Ile
785                 790                 795                 800

Asp His Ala Lys Glu Asp Arg Leu Lys Lys Leu Ala Asp Arg Ile Ile
                805                 810                 815

Met Glu Ala Leu Gly Tyr Val Tyr Ala Leu Asp Asp Glu Arg Gly Lys
            820                 825                 830

Gly Lys Trp Val Ala Lys Tyr Pro Pro Cys Gln Leu Ile Leu Leu Glu
        835                 840                 845

Glu Leu Ser Glu Tyr Gln Phe Asn Asn Asp Arg Pro Pro Ser Glu Asn
    850                 855                 860

Asn Gln Leu Met Gln Trp Ser His Arg Gly Val Phe Gln Glu Leu Leu
865                 870                 875                 880

Asn Gln Ala Gln Val His Asp Leu Leu Val Gly Thr Met Tyr Ala Ala
                885                 890                 895

Phe Ser Ser Arg Phe Asp Ala Arg Thr Gly Ala Pro Gly Ile Arg Cys
            900                 905                 910

Arg Arg Val Pro Ala Arg Cys Ala Arg Glu Gln Asn Pro Glu Pro Phe
        915                 920                 925

Pro Trp Trp Leu Asn Lys Phe Val Ala Glu His Lys Leu Asp Gly Cys

930                 935                 940

Pro Leu Arg Ala Asp Asp Leu Ile Pro Thr Gly Glu Gly Glu Phe Phe
945                 950                 955                 960

Val Ser Pro Phe Ser Ala Glu Glu Gly Asp Phe His Gln Ile His Ala
                965                 970                 975

Asp Leu Asn Ala Ala Gln Asn Leu Gln Arg Arg Leu Trp Ser Asp Phe
            980                 985                 990

Asp Ile Ser Gln Ile Arg Leu Arg  Cys Asp Trp Gly Glu  Val Asp Gly
        995                 1000                 1005

Glu Pro  Val Leu Ile Pro Arg  Thr Thr Gly Lys Arg  Thr Ala Asp
    1010                 1015                 1020

Ser Tyr  Gly Asn Lys Val Phe  Tyr Thr Lys Thr Gly  Val Thr Tyr
    1025                 1030                 1035

Tyr Glu  Arg Glu Arg Gly Lys  Lys Arg Arg Lys Val  Phe Ala Gln
    1040                 1045                 1050

Glu Glu  Leu Ser Glu Glu Glu  Ala Glu Leu Leu Val  Glu Ala Asp
    1055                 1060                 1065

Glu Ala  Arg Glu Lys Ser Val  Val Leu Met Arg Asp  Pro Ser Gly
    1070                 1075                 1080

Ile Ile  Asn Arg Gly Asp Trp  Thr Arg Gln Lys Glu  Phe Trp Ser
    1085                 1090                 1095

Met Val  Asn Gln Arg Ile Glu  Gly Tyr Leu Val Lys  Gln Ile Arg
    1100                 1105                 1110

Ser Arg  Val Arg Leu Gln Glu  Ser Ala Cys Glu Asn  Thr Gly Asp
    1115                 1120                 1125

Ile

<210>  2
<211>  1147
<212>  PRT
<213>  Alicyclobacillus kakegawensis

<400>  2

Met Ala Val Lys Ser Ile Lys Val Lys Leu Arg Leu Ser Glu Cys Pro

1               5                   10                  15

Asp Ile Leu Ala Gly Met Trp Gln Leu His Arg Ala Thr Asn Ala Gly
            20                  25                  30

Val Arg Tyr Tyr Thr Glu Trp Val Ser Leu Met Arg Gln Glu Ile Leu
        35                  40                  45

Tyr Ser Arg Gly Pro Asp Gly Gly Gln Gln Cys Tyr Met Thr Ala Glu
    50                  55                  60

Asp Cys Gln Arg Glu Leu Leu Arg Arg Leu Arg Asn Arg Gln Leu His
65                  70                  75                  80

Asn Gly Arg Gln Asp Gln Pro Gly Thr Asp Ala Asp Leu Leu Ala Ile
                85                  90                  95

Ser Arg Arg Leu Tyr Glu Ile Leu Val Leu Gln Ser Ile Gly Lys Arg
            100                 105                 110

Gly Asp Ala Gln Gln Ile Ala Ser Ser Phe Leu Ser Pro Leu Val Asp
        115                 120                 125

Pro Asn Ser Lys Gly Gly Arg Gly Glu Ala Lys Ser Gly Arg Lys Pro
    130                 135                 140

Ala Trp Gln Lys Met Arg Asp Gln Gly Asp Pro Arg Trp Val Ala Ala
145                 150                 155                 160

Arg Glu Lys Tyr Glu Gln Arg Lys Ala Val Asp Pro Ser Lys Glu Ile
                165                 170                 175

Leu Asn Ser Leu Asp Ala Leu Gly Leu Arg Pro Leu Phe Ala Val Phe
            180                 185                 190

Thr Glu Thr Tyr Arg Ser Gly Val Asp Trp Lys Pro Leu Gly Lys Ser
        195                 200                 205

Gln Gly Val Arg Thr Trp Asp Arg Asp Met Phe Gln Gln Ala Leu Glu
    210                 215                 220

Arg Leu Met Ser Trp Glu Ser Trp Asn Arg Arg Val Gly Glu Glu Tyr
225                 230                 235                 240

Ala Arg Leu Phe Gln Gln Lys Met Lys Phe Glu Gln Glu His Phe Ala
                245                 250                 255

Glu Gln Ser His Leu Val Lys Leu Ala Arg Ala Leu Glu Ala Asp Met
            260                 265                 270

Arg Ala Ala Ser Gln Gly Phe Glu Ala Lys Arg Gly Thr Ala His Gln
        275                 280                 285

Ile Thr Arg Arg Ala Leu Arg Gly Ala Asp Arg Val Phe Glu Ile Trp
    290                 295                 300

Lys Ser Ile Pro Glu Glu Ala Leu Phe Ser Gln Tyr Asp Glu Val Ile
305                 310                 315                 320

Arg Gln Val Gln Ala Glu Lys Arg Arg Asp Phe Gly Ser His Asp Leu
                325                 330                 335

Phe Ala Lys Leu Ala Glu Pro Lys Tyr Gln Pro Leu Trp Arg Ala Asp
            340                 345                 350

Glu Thr Phe Leu Thr Arg Tyr Ala Leu Tyr Asn Gly Val Leu Arg Asp
        355                 360                 365

Leu Glu Lys Ala Arg Gln Phe Ala Thr Phe Thr Leu Pro Asp Ala Cys
    370                 375                 380

Val Asn Pro Ile Trp Thr Arg Phe Glu Ser Ser Gln Gly Ser Asn Leu
385                 390                 395                 400

His Lys Tyr Glu Phe Leu Phe Asp His Leu Gly Pro Gly Arg His Ala
                405                 410                 415

Val Arg Phe Gln Arg Leu Leu Val Val Glu Ser Glu Gly Ala Lys Glu
            420                 425                 430

Arg Asp Ser Val Val Val Pro Val Ala Pro Ser Gly Gln Leu Asp Lys
        435                 440                 445

Leu Val Leu Arg Glu Glu Glu Lys Ser Ser Val Ala Leu His Leu His
    450                 455                 460

Asp Thr Ala Arg Pro Asp Gly Phe Met Ala Glu Trp Ala Gly Ala Lys
465                 470                 475                 480

Leu Gln Tyr Glu Arg Ser Thr Leu Ala Arg Lys Ala Arg Arg Asp Lys
                485                 490                 495

Gln Gly Met Arg Ser Trp Arg Arg Gln Pro Ser Met Leu Met Ser Ala
            500                 505                 510

Ala Gln Met Leu Glu Asp Ala Lys Gln Ala Gly Asp Val Tyr Leu Asn
        515                 520                 525

Ile Ser Val Arg Val Lys Ser Pro Ser Glu Val Arg Gly Gln Arg Arg
    530                 535                 540

Pro Pro Tyr Ala Ala Leu Phe Arg Ile Asp Asp Lys Gln Arg Arg Val
545                 550                 555                 560

Thr Val Asn Tyr Asn Lys Leu Ser Ala Tyr Leu Glu Glu His Pro Asp
                565                 570                 575

Lys Gln Ile Pro Gly Ala Pro Gly Leu Leu Ser Gly Leu Arg Val Met
            580                 585                 590

Ser Val Asp Leu Gly Leu Arg Thr Ser Ala Ser Ile Ser Val Phe Arg
        595                 600                 605

Val Ala Lys Lys Glu Glu Val Glu Ala Leu Gly Asp Gly Arg Pro Pro
    610                 615                 620

His Tyr Tyr Pro Ile His Gly Thr Asp Asp Leu Val Ala Val His Glu
625                 630                 635                 640

Arg Ser His Leu Ile Gln Met Pro Gly Glu Thr Glu Thr Lys Gln Leu
                645                 650                 655

Arg Lys Leu Arg Glu Glu Arg Gln Ala Val Leu Arg Pro Leu Phe Ala
            660                 665                 670

Gln Leu Ala Leu Leu Arg Leu Leu Val Arg Cys Gly Ala Ala Asp Glu
        675                 680                 685

Arg Ile Arg Thr Arg Ser Trp Gln Arg Leu Thr Lys Gln Gly Arg Glu
    690                 695                 700

Phe Thr Lys Arg Leu Thr Pro Ser Trp Arg Glu Ala Leu Glu Leu Glu
705                 710                 715                 720

Leu Thr Arg Leu Glu Ala Tyr Cys Gly Arg Val Pro Asp Asp Glu Trp
                725                 730                 735

Ser Arg Ile Val Asp Arg Thr Val Ile Ala Leu Trp Arg Arg Met Gly
            740                 745                 750

Lys Gln Val Arg Asp Trp Arg Lys Gln Val Lys Ser Gly Ala Lys Val
        755                 760                 765

Lys Val Lys Gly Tyr Gln Leu Asp Val Val Gly Gly Asn Ser Leu Ala
    770                 775                 780

Gln Ile Asp Tyr Leu Glu Gln Gln Tyr Lys Phe Leu Arg Arg Trp Ser
785                 790                 795                 800

Phe Phe Ala Arg Ala Ser Gly Leu Val Val Arg Ala Asp Arg Glu Ser
                805                 810                 815

His Phe Ala Val Ala Leu Arg Gln His Ile Glu Asn Ala Lys Arg Asp
            820                 825                 830

Arg Leu Lys Lys Leu Ala Asp Arg Ile Leu Met Glu Ala Leu Gly Tyr
        835                 840                 845

Val Tyr Glu Ala Ser Gly Pro Arg Glu Gly Gln Trp Thr Ala Gln His
    850                 855                 860

Pro Pro Cys Gln Leu Ile Ile Leu Glu Glu Leu Ser Ala Tyr Arg Phe
865                 870                 875                 880

Ser Asp Asp Arg Pro Pro Ser Glu Asn Ser Lys Leu Met Ala Trp Gly
                885                 890                 895

His Arg Gly Ile Leu Glu Glu Leu Val Asn Gln Ala Gln Val His Asp
            900                 905                 910

Val Leu Val Gly Thr Val Tyr Ala Ala Phe Ser Ser Arg Phe Asp Ala
        915                 920                 925

Arg Thr Gly Ala Pro Gly Val Arg Cys Arg Arg Val Pro Ala Arg Phe
    930                 935                 940

Val Gly Ala Thr Val Asp Asp Ser Leu Pro Leu Trp Leu Thr Glu Phe
945                 950                 955                 960

Leu Asp Lys His Arg Leu Asp Lys Asn Leu Leu Arg Pro Asp Asp Val
                965                 970                 975

Ile Pro Thr Gly Glu Gly Glu Phe Leu Val Ser Pro Cys Gly Glu Glu
            980                 985                 990

Ala Ala Arg Val Arg Gln Val His  Ala Asp Ile Asn Ala  Ala Gln Asn
        995                 1000                 1005

Leu Gln  Arg Arg Leu Trp Gln  Asn Phe Asp Ile Thr  Glu Leu Arg

1010                    1015                    1020

Leu Arg  Cys Asp Val Lys Met  Gly Gly Glu Gly Thr  Val Leu Val
    1025                    1030                    1035

Pro Arg  Val Asn Asn Ala Arg  Ala Lys Gln Leu Phe  Gly Lys Lys
    1040                    1045                    1050

Val Leu  Val Ser Gln Asp Gly  Val Thr Phe Phe Glu  Arg Ser Gln
    1055                    1060                    1065

Thr Gly  Gly Lys Pro His Ser  Glu Lys Gln Thr Asp  Leu Thr Asp
    1070                    1075                    1080

Lys Glu  Leu Glu Leu Ile Ala  Glu Ala Asp Glu Ala  Arg Ala Lys
    1085                    1090                    1095

Ser Val  Val Leu Phe Arg Asp  Pro Ser Gly His Ile  Gly Lys Gly
    1100                    1105                    1110

His Trp  Ile Arg Gln Arg Glu  Phe Trp Ser Leu Val  Lys Gln Arg
    1115                    1120                    1125

Ile Glu  Ser His Thr Ala Glu  Arg Ile Arg Val Arg  Gly Val Gly
    1130                    1135                    1140

Ser Ser  Leu Asp
    1145

<210>  3
<211>  1146
<212>  PRT
<213>  Alicyclobacillus macrosporangiidus

<400>  3

Met Asn Val Ala Val Lys Ser Ile Lys Val Lys Leu Met Leu Gly His
1               5                   10                  15

Leu Pro Glu Ile Arg Glu Gly Leu Trp His Leu His Glu Ala Val Asn
            20                  25                  30

Leu Gly Val Arg Tyr Tyr Thr Glu Trp Leu Ala Leu Leu Arg Gln Gly
        35                  40                  45

Asn Leu Tyr Arg Arg Gly Lys Asp Gly Ala Gln Glu Cys Tyr Met Thr
    50                  55                  60

Ala Glu Gln Cys Arg Gln Glu Leu Leu Val Arg Leu Arg Asp Arg Gln

65                  70                  75                  80

Lys Arg Asn Gly His Thr Gly Asp Pro Gly Thr Asp Glu Glu Leu Leu
                85                  90                  95

Gly Val Ala Arg Arg Leu Tyr Glu Leu Leu Val Pro Gln Ser Val Gly
            100                 105                 110

Lys Lys Gly Gln Ala Gln Met Leu Ala Ser Gly Phe Leu Ser Pro Leu
        115                 120                 125

Ala Asp Pro Lys Ser Glu Gly Gly Lys Gly Thr Ser Lys Ser Gly Arg
    130                 135                 140

Lys Pro Ala Trp Met Gly Met Lys Glu Ala Gly Asp Ser Arg Trp Val
145                 150                 155                 160

Glu Ala Lys Ala Arg Tyr Glu Ala Asn Lys Ala Lys Asp Pro Thr Lys
                165                 170                 175

Gln Val Ile Ala Ser Leu Glu Met Tyr Gly Leu Arg Pro Leu Phe Asp
            180                 185                 190

Val Phe Thr Glu Thr Tyr Lys Thr Ile Arg Trp Met Pro Leu Gly Lys
        195                 200                 205

His Gln Gly Val Arg Ala Trp Asp Arg Asp Met Phe Gln Gln Ser Leu
    210                 215                 220

Glu Arg Leu Met Ser Trp Glu Ser Trp Asn Glu Arg Val Gly Ala Glu
225                 230                 235                 240

Phe Ala Arg Leu Val Asp Arg Arg Asp Arg Phe Arg Glu Lys His Phe
                245                 250                 255

Thr Gly Gln Glu His Leu Val Ala Leu Ala Gln Arg Leu Glu Gln Glu
            260                 265                 270

Met Lys Glu Ala Ser Pro Gly Phe Glu Ser Lys Ser Ser Gln Ala His
        275                 280                 285

Arg Ile Thr Lys Arg Ala Leu Arg Gly Ala Asp Gly Ile Ile Asp Asp
    290                 295                 300

Trp Leu Lys Leu Ser Glu Gly Glu Pro Val Asp Arg Phe Asp Glu Ile
305                 310                 315                 320

Leu Arg Lys Arg Gln Ala Gln Asn Pro Arg Arg Phe Gly Ser His Asp
                325                 330                 335

Leu Phe Leu Lys Leu Ala Glu Pro Val Phe Gln Pro Leu Trp Arg Glu
            340                 345                 350

Asp Pro Ser Phe Leu Ser Arg Trp Ala Ser Tyr Asn Glu Val Leu Asn
        355                 360                 365

Lys Leu Glu Asp Ala Lys Gln Phe Ala Thr Phe Thr Leu Pro Ser Pro
    370                 375                 380

Cys Ser Asn Pro Val Trp Ala Arg Phe Glu Asn Ala Glu Gly Thr Asn
385                 390                 395                 400

Ile Phe Lys Tyr Asp Phe Leu Phe Asp His Phe Gly Lys Gly Arg His
                405                 410                 415

Gly Val Arg Phe Gln Arg Met Ile Val Met Arg Asp Gly Val Pro Thr
            420                 425                 430

Glu Val Glu Gly Ile Val Val Pro Ile Ala Pro Ser Arg Gln Leu Asp
        435                 440                 445

Ala Leu Ala Pro Asn Asp Ala Ala Ser Pro Ile Asp Val Phe Val Gly
    450                 455                 460

Asp Pro Ala Ala Pro Gly Ala Phe Arg Gly Gln Phe Gly Gly Ala Lys
465                 470                 475                 480

Ile Gln Tyr Arg Arg Ser Ala Leu Val Arg Lys Gly Arg Arg Glu Glu
                485                 490                 495

Lys Ala Tyr Leu Cys Gly Phe Arg Leu Pro Ser Gln Arg Arg Thr Gly
            500                 505                 510

Thr Pro Ala Asp Asp Ala Gly Glu Val Phe Leu Asn Leu Ser Leu Arg
        515                 520                 525

Val Glu Ser Gln Ser Glu Gln Ala Gly Arg Arg Asn Pro Pro Tyr Ala
    530                 535                 540

Ala Val Phe His Ile Ser Asp Gln Thr Arg Arg Val Ile Val Arg Tyr
545                 550                 555                 560

Gly Glu Ile Glu Arg Tyr Leu Ala Glu His Pro Asp Thr Gly Ile Pro
                565                 570                 575

Gly Ser Arg Gly Leu Thr Ser Gly Leu Arg Val Met Ser Val Asp Leu
        580                 585                 590

Gly Leu Arg Thr Ser Ala Ala Ile Ser Val Phe Arg Val Ala His Arg
    595                 600                 605

Asp Glu Leu Thr Pro Asp Ala His Gly Arg Gln Pro Phe Phe Phe Pro
    610                 615                 620

Ile His Gly Met Asp His Leu Val Ala Leu His Glu Arg Ser His Leu
625                 630                 635                 640

Ile Arg Leu Pro Gly Glu Thr Glu Ser Lys Lys Val Arg Ser Ile Arg
                645                 650                 655

Glu Gln Arg Leu Asp Arg Leu Asn Arg Leu Arg Ser Gln Met Ala Ser
            660                 665                 670

Leu Arg Leu Leu Val Arg Thr Gly Val Leu Asp Glu Gln Lys Arg Asp
        675                 680                 685

Arg Asn Trp Glu Arg Leu Gln Ser Ser Met Glu Arg Gly Gly Glu Arg
    690                 695                 700

Met Pro Ser Asp Trp Trp Asp Leu Phe Gln Ala Gln Val Arg Tyr Leu
705                 710                 715                 720

Ala Gln His Arg Asp Ala Ser Gly Glu Ala Trp Gly Arg Met Val Gln
                725                 730                 735

Ala Ala Val Arg Thr Leu Trp Arg Gln Leu Ala Lys Gln Val Arg Asp
            740                 745                 750

Trp Arg Lys Glu Val Arg Arg Asn Ala Asp Lys Val Lys Ile Arg Gly
        755                 760                 765

Ile Ala Arg Asp Val Pro Gly Gly His Ser Leu Ala Gln Leu Asp Tyr
    770                 775                 780

Leu Glu Arg Gln Tyr Arg Phe Leu Arg Ser Trp Ser Ala Phe Ser Val
785                 790                 795                 800

Gln Ala Gly Gln Val Val Arg Ala Glu Arg Asp Ser Arg Phe Ala Val
                805                 810                 815

Ala Leu Arg Glu His Ile Asp Asn Gly Lys Lys Asp Arg Leu Lys Lys
            820                 825                 830

Leu Ala Asp Arg Ile Leu Met Glu Ala Leu Gly Tyr Val Tyr Val Thr
        835                 840                 845

Asp Gly Arg Arg Ala Gly Gln Trp Gln Ala Val Tyr Pro Pro Cys Gln
    850                 855                 860

Leu Val Leu Leu Glu Glu Leu Ser Glu Tyr Arg Phe Ser Asn Asp Arg
865                 870                 875                 880

Pro Pro Ser Glu Asn Ser Gln Leu Met Val Trp Ser His Arg Gly Val
                885                 890                 895

Leu Glu Glu Leu Ile His Gln Ala Gln Val His Asp Val Leu Val Gly
            900                 905                 910

Thr Ile Pro Ala Ala Phe Ser Ser Arg Phe Asp Ala Arg Thr Gly Ala
        915                 920                 925

Pro Gly Ile Arg Cys Arg Arg Val Pro Ser Ile Pro Leu Lys Asp Ala
    930                 935                 940

Pro Ser Ile Pro Ile Trp Leu Ser His Tyr Leu Lys Gln Thr Glu Arg
945                 950                 955                 960

Asp Ala Ala Ala Leu Arg Pro Gly Glu Leu Ile Pro Thr Gly Asp Gly
                965                 970                 975

Glu Phe Leu Val Thr Pro Ala Gly Arg Gly Ala Ser Gly Val Arg Val
            980                 985                 990

Val His Ala Asp Ile Asn Ala Ala His Asn Leu Gln Arg Arg Leu Trp
        995                 1000                1005

Glu Asn Phe Asp Leu Ser Asp Ile Arg Val Arg Cys Asp Arg Arg
    1010                1015                1020

Glu Gly Lys Asp Gly Thr Val Val Leu Ile Pro Arg Leu Thr Asn
    1025                1030                1035

Gln Arg Val Lys Glu Arg Tyr Ser Gly Val Ile Phe Thr Ser Glu
    1040                1045                1050

Asp Gly Val Ser Phe Thr Val Gly Asp Ala Lys Thr Arg Arg Arg
    1055                1060                1065

Ser Ser Ala Ser Gln Gly Glu Gly Asp Asp Leu Ser Asp Glu Glu

1070                    1075                    1080

Gln Glu  Leu Leu Ala Glu Ala  Asp Asp Ala Arg Glu  Arg Ser Val
    1085                    1090                    1095

Val Leu  Phe Arg Asp Pro Ser  Gly Phe Val Asn Gly  Gly Arg Trp
    1100                    1105                    1110

Thr Ala  Gln Arg Ala Phe Trp  Gly Met Val His Asn  Arg Ile Glu
    1115                    1120                    1125

Thr Leu  Leu Ala Glu Arg Phe  Ser Val Ser Gly Ala  Ala Glu Lys
    1130                    1135                    1140

Val Arg  Gly
    1145

<210>  4
<211>  1108
<212>  PRT
<213>  Bacillus hisashii

<400>  4

Met Ala Thr Arg Ser Phe Ile Leu Lys Ile Glu Pro Asn Glu Glu Val
1               5                   10                  15

Lys Lys Gly Leu Trp Lys Thr His Glu Val Leu Asn His Gly Ile Ala
            20                  25                  30

Tyr Tyr Met Asn Ile Leu Lys Leu Ile Arg Gln Glu Ala Ile Tyr Glu
        35                  40                  45

His His Glu Gln Asp Pro Lys Asn Pro Lys Lys Val Ser Lys Ala Glu
    50                  55                  60

Ile Gln Ala Glu Leu Trp Asp Phe Val Leu Lys Met Gln Lys Cys Asn
65                  70                  75                  80

Ser Phe Thr His Glu Val Asp Lys Asp Glu Val Phe Asn Ile Leu Arg
                85                  90                  95

Glu Leu Tyr Glu Glu Leu Val Pro Ser Ser Val Glu Lys Lys Gly Glu
            100                 105                 110

Ala Asn Gln Leu Ser Asn Lys Phe Leu Tyr Pro Leu Val Asp Pro Asn
        115                 120                 125

Ser Gln Ser Gly Lys Gly Thr Ala Ser Ser Gly Arg Lys Pro Arg Trp

130                 135                 140

Tyr Asn Leu Lys Ile Ala Gly Asp Pro Ser Trp Glu Glu Glu Lys Lys
145                 150                 155                 160

Lys Trp Glu Glu Asp Lys Lys Lys Asp Pro Leu Ala Lys Ile Leu Gly
                165                 170                 175

Lys Leu Ala Glu Tyr Gly Leu Ile Pro Leu Phe Ile Pro Tyr Thr Asp
            180                 185                 190

Ser Asn Glu Pro Ile Val Lys Glu Ile Lys Trp Met Glu Lys Ser Arg
        195                 200                 205

Asn Gln Ser Val Arg Arg Leu Asp Lys Asp Met Phe Ile Gln Ala Leu
    210                 215                 220

Glu Arg Phe Leu Ser Trp Glu Ser Trp Asn Leu Lys Val Lys Glu Glu
225                 230                 235                 240

Tyr Glu Lys Val Glu Lys Glu Tyr Lys Thr Leu Glu Glu Arg Ile Lys
                245                 250                 255

Glu Asp Ile Gln Ala Leu Lys Ala Leu Glu Gln Tyr Glu Lys Glu Arg
            260                 265                 270

Gln Glu Gln Leu Leu Arg Asp Thr Leu Asn Thr Asn Glu Tyr Arg Leu
        275                 280                 285

Ser Lys Arg Gly Leu Arg Gly Trp Arg Glu Ile Ile Gln Lys Trp Leu
    290                 295                 300

Lys Met Asp Glu Asn Glu Pro Ser Glu Lys Tyr Leu Glu Val Phe Lys
305                 310                 315                 320

Asp Tyr Gln Arg Lys His Pro Arg Glu Ala Gly Asp Tyr Ser Val Tyr
                325                 330                 335

Glu Phe Leu Ser Lys Lys Glu Asn His Phe Ile Trp Arg Asn His Pro
            340                 345                 350

Glu Tyr Pro Tyr Leu Tyr Ala Thr Phe Cys Glu Ile Asp Lys Lys Lys
        355                 360                 365

Lys Asp Ala Lys Gln Gln Ala Thr Phe Thr Leu Ala Asp Pro Ile Asn
    370                 375                 380

His Pro Leu Trp Val Arg Phe Glu Glu Arg Ser Gly Ser Asn Leu Asn
385                 390                 395                 400

Lys Tyr Arg Ile Leu Thr Glu Gln Leu His Thr Glu Lys Leu Lys Lys
                405                 410                 415

Lys Leu Thr Val Gln Leu Asp Arg Leu Ile Tyr Pro Thr Glu Ser Gly
            420                 425                 430

Gly Trp Glu Glu Lys Gly Lys Val Asp Ile Val Leu Leu Pro Ser Arg
        435                 440                 445

Gln Phe Tyr Asn Gln Ile Phe Leu Asp Ile Glu Glu Lys Gly Lys His
    450                 455                 460

Ala Phe Thr Tyr Lys Asp Glu Ser Ile Lys Phe Pro Leu Lys Gly Thr
465                 470                 475                 480

Leu Gly Gly Ala Arg Val Gln Phe Asp Arg Asp His Leu Arg Arg Tyr
                485                 490                 495

Pro His Lys Val Glu Ser Gly Asn Val Gly Arg Ile Tyr Phe Asn Met
            500                 505                 510

Thr Val Asn Ile Glu Pro Thr Glu Ser Pro Val Ser Lys Ser Leu Lys
        515                 520                 525

Ile His Arg Asp Asp Phe Pro Lys Val Val Asn Phe Lys Pro Lys Glu
    530                 535                 540

Leu Thr Glu Trp Ile Lys Asp Ser Lys Gly Lys Lys Leu Lys Ser Gly
545                 550                 555                 560

Ile Glu Ser Leu Glu Ile Gly Leu Arg Val Met Ser Ile Asp Leu Gly
                565                 570                 575

Gln Arg Gln Ala Ala Ala Ala Ser Ile Phe Glu Val Val Asp Gln Lys
            580                 585                 590

Pro Asp Ile Glu Gly Lys Leu Phe Phe Pro Ile Lys Gly Thr Glu Leu
        595                 600                 605

Tyr Ala Val His Arg Ala Ser Phe Asn Ile Lys Leu Pro Gly Glu Thr
    610                 615                 620

Leu Val Lys Ser Arg Glu Val Leu Arg Lys Ala Arg Glu Asp Asn Leu
625                 630                 635                 640

Lys Leu Met Asn Gln Lys Leu Asn Phe Leu Arg Asn Val Leu His Phe
                645                 650                 655

Gln Gln Phe Glu Asp Ile Thr Glu Arg Glu Lys Arg Val Thr Lys Trp
            660                 665                 670

Ile Ser Arg Gln Glu Asn Ser Asp Val Pro Leu Val Tyr Gln Asp Glu
        675                 680                 685

Leu Ile Gln Ile Arg Glu Leu Met Tyr Lys Pro Tyr Lys Asp Trp Val
    690                 695                 700

Ala Phe Leu Lys Gln Leu His Lys Arg Leu Glu Val Glu Ile Gly Lys
705                 710                 715                 720

Glu Val Lys His Trp Arg Lys Ser Leu Ser Asp Gly Arg Lys Gly Leu
                725                 730                 735

Tyr Gly Ile Ser Leu Lys Asn Ile Asp Glu Ile Asp Arg Thr Arg Lys
            740                 745                 750

Phe Leu Leu Arg Trp Ser Leu Arg Pro Thr Glu Pro Gly Glu Val Arg
        755                 760                 765

Arg Leu Glu Pro Gly Gln Arg Phe Ala Ile Asp Gln Leu Asn His Leu
    770                 775                 780

Asn Ala Leu Lys Glu Asp Arg Leu Lys Lys Met Ala Asn Thr Ile Ile
785                 790                 795                 800

Met His Ala Leu Gly Tyr Cys Tyr Asp Val Arg Lys Lys Lys Trp Gln
                805                 810                 815

Ala Lys Asn Pro Ala Cys Gln Ile Ile Leu Phe Glu Asp Leu Ser Asn
            820                 825                 830

Tyr Asn Pro Tyr Glu Glu Arg Ser Arg Phe Glu Asn Ser Lys Leu Met
        835                 840                 845

Lys Trp Ser Arg Arg Glu Ile Pro Arg Gln Val Ala Leu Gln Gly Glu
    850                 855                 860

Ile Tyr Gly Leu Gln Val Gly Glu Val Gly Ala Gln Phe Ser Ser Arg
865                 870                 875                 880

Phe His Ala Lys Thr Gly Ser Pro Gly Ile Arg Cys Ser Val Val Thr
                885                 890                 895

Lys Glu Lys Leu Gln Asp Asn Arg Phe Phe Lys Asn Leu Gln Arg Glu
            900                 905                 910

Gly Arg Leu Thr Leu Asp Lys Ile Ala Val Leu Lys Glu Gly Asp Leu
        915                 920                 925

Tyr Pro Asp Lys Gly Gly Glu Lys Phe Ile Ser Leu Ser Lys Asp Arg
    930                 935                 940

Lys Cys Val Thr Thr His Ala Asp Ile Asn Ala Ala Gln Asn Leu Gln
945                 950                 955                 960

Lys Arg Phe Trp Thr Arg Thr His Gly Phe Tyr Lys Val Tyr Cys Lys
                965                 970                 975

Ala Tyr Gln Val Asp Gly Gln Thr Val Tyr Ile Pro Glu Ser Lys Asp
            980                 985                 990

Gln Lys Gln Lys Ile Ile Glu Glu Phe Gly Glu Gly Tyr Phe Ile Leu
        995                 1000                1005

Lys Asp Gly Val Tyr Glu Trp Val Asn Ala Gly Lys Leu Lys Ile
    1010                1015                1020

Lys Lys Gly Ser Ser Lys Gln Ser Ser Ser Glu Leu Val Asp Ser
    1025                1030                1035

Asp Ile Leu Lys Asp Ser Phe Asp Leu Ala Ser Glu Leu Lys Gly
    1040                1045                1050

Glu Lys Leu Met Leu Tyr Arg Asp Pro Ser Gly Asn Val Phe Pro
    1055                1060                1065

Ser Asp Lys Trp Met Ala Ala Gly Val Phe Phe Gly Lys Leu Glu
    1070                1075                1080

Arg Ile Leu Ile Ser Lys Leu Thr Asn Gln Tyr Ser Ile Ser Thr
    1085                1090                1095

Ile Glu Asp Asp Ser Ser Lys Gln Ser Met
    1100                1105

<210> 5
<211> 1108
<212> PRT
<213> Bacillus

<400>  5

Met Ala Ile Arg Ser Ile Lys Leu Lys Leu Lys Thr His Thr Gly Pro
1               5                   10                  15

Glu Ala Gln Asn Leu Arg Lys Gly Ile Trp Arg Thr His Arg Leu Leu
            20                  25                  30

Asn Glu Gly Val Ala Tyr Tyr Met Lys Met Leu Leu Leu Phe Arg Gln
        35                  40                  45

Glu Ser Thr Gly Glu Arg Pro Lys Glu Glu Leu Gln Glu Glu Leu Ile
    50                  55                  60

Cys His Ile Arg Glu Gln Gln Gln Arg Asn Gln Ala Asp Lys Asn Thr
65                  70                  75                  80

Gln Ala Leu Pro Leu Asp Lys Ala Leu Glu Ala Leu Arg Gln Leu Tyr
                85                  90                  95

Glu Leu Leu Val Pro Ser Ser Val Gly Gln Ser Gly Asp Ala Gln Ile
            100                 105                 110

Ile Ser Arg Lys Phe Leu Ser Pro Leu Val Asp Pro Asn Ser Glu Gly
        115                 120                 125

Gly Lys Gly Thr Ser Lys Ala Gly Ala Lys Pro Thr Trp Gln Lys Lys
    130                 135                 140

Lys Glu Ala Asn Asp Pro Thr Trp Glu Gln Asp Tyr Glu Lys Trp Lys
145                 150                 155                 160

Lys Arg Arg Glu Glu Asp Pro Thr Ala Ser Val Ile Thr Thr Leu Glu
                165                 170                 175

Glu Tyr Gly Ile Arg Pro Ile Phe Pro Leu Tyr Thr Asn Thr Val Thr
            180                 185                 190

Asp Ile Ala Trp Leu Pro Leu Gln Ser Asn Gln Phe Val Arg Thr Trp
        195                 200                 205

Asp Arg Asp Met Leu Gln Gln Ala Ile Glu Arg Leu Leu Ser Trp Glu
    210                 215                 220

Ser Trp Asn Lys Arg Val Gln Glu Glu Tyr Ala Lys Leu Lys Glu Lys
225                 230                 235                 240

Met Ala Gln Leu Asn Glu Gln Leu Glu Gly Gly Gln Glu Trp Ile Ser

245                 250                 255

Leu Leu Glu Gln Tyr Glu Glu Asn Arg Glu Arg Glu Leu Arg Glu Asn
            260                 265                 270

Met Thr Ala Ala Asn Asp Lys Tyr Arg Ile Thr Lys Arg Gln Met Lys
        275                 280                 285

Gly Trp Asn Glu Leu Tyr Glu Leu Trp Ser Thr Phe Pro Ala Ser Ala
    290                 295                 300

Ser His Glu Gln Tyr Lys Glu Ala Leu Lys Arg Val Gln Gln Arg Leu
305                 310                 315                 320

Arg Gly Arg Phe Gly Asp Ala His Phe Phe Gln Tyr Leu Met Glu Glu
                325                 330                 335

Lys Asn Arg Leu Ile Trp Lys Gly Asn Pro Gln Arg Ile His Tyr Phe
            340                 345                 350

Val Ala Arg Asn Glu Leu Thr Lys Arg Leu Glu Glu Ala Lys Gln Ser
        355                 360                 365

Ala Thr Met Thr Leu Pro Asn Ala Arg Lys His Pro Leu Trp Val Arg
    370                 375                 380

Phe Asp Ala Arg Gly Gly Asn Leu Gln Asp Tyr Tyr Leu Thr Ala Glu
385                 390                 395                 400

Ala Asp Lys Pro Arg Ser Arg Arg Phe Val Thr Phe Ser Gln Leu Ile
                405                 410                 415

Trp Pro Ser Glu Ser Gly Trp Met Glu Lys Lys Asp Val Glu Val Glu
            420                 425                 430

Leu Ala Leu Ser Arg Gln Phe Tyr Gln Gln Val Lys Leu Leu Lys Asn
        435                 440                 445

Asp Lys Gly Lys Gln Lys Ile Glu Phe Lys Asp Lys Gly Ser Gly Ser
    450                 455                 460

Thr Phe Asn Gly His Leu Gly Gly Ala Lys Leu Gln Leu Glu Arg Gly
465                 470                 475                 480

Asp Leu Glu Lys Glu Glu Lys Asn Phe Glu Asp Gly Glu Ile Gly Ser
                485                 490                 495

Val Tyr Leu Asn Val Val Ile Asp Phe Glu Pro Leu Gln Glu Val Lys
        500                 505                 510

Asn Gly Arg Val Gln Ala Pro Tyr Gly Gln Val Leu Gln Leu Ile Arg
    515                 520                 525

Arg Pro Asn Glu Phe Pro Lys Val Thr Thr Tyr Lys Ser Glu Gln Leu
    530                 535                 540

Val Glu Trp Ile Lys Ala Ser Pro Gln His Ser Ala Gly Val Glu Ser
545                 550                 555                 560

Leu Ala Ser Gly Phe Arg Val Met Ser Ile Asp Leu Gly Leu Arg Ala
                565                 570                 575

Ala Ala Ala Thr Ser Ile Phe Ser Val Glu Glu Ser Ser Asp Lys Asn
            580                 585                 590

Ala Ala Asp Phe Ser Tyr Trp Ile Glu Gly Thr Pro Leu Val Ala Val
        595                 600                 605

His Gln Arg Ser Tyr Met Leu Arg Leu Pro Gly Glu Gln Val Glu Lys
    610                 615                 620

Gln Val Met Glu Lys Arg Asp Glu Arg Phe Gln Leu His Gln Arg Val
625                 630                 635                 640

Lys Phe Gln Ile Arg Val Leu Ala Gln Ile Met Arg Met Ala Asn Lys
                645                 650                 655

Gln Tyr Gly Asp Arg Trp Asp Glu Leu Asp Ser Leu Lys Gln Ala Val
            660                 665                 670

Glu Gln Lys Lys Ser Pro Leu Asp Gln Thr Asp Arg Thr Phe Trp Glu
        675                 680                 685

Gly Ile Val Cys Asp Leu Thr Lys Val Leu Pro Arg Asn Glu Ala Asp
    690                 695                 700

Trp Glu Gln Ala Val Val Gln Ile His Arg Lys Ala Glu Glu Tyr Val
705                 710                 715                 720

Gly Lys Ala Val Gln Ala Trp Arg Lys Arg Phe Ala Ala Asp Glu Arg
                725                 730                 735

Lys Gly Ile Ala Gly Leu Ser Met Trp Asn Ile Glu Glu Leu Glu Gly
            740                 745                 750

Leu Arg Lys Leu Leu Ile Ser Trp Ser Arg Arg Thr Arg Asn Pro Gln
        755                 760                 765

Glu Val Asn Arg Phe Glu Arg Gly His Thr Ser His Gln Arg Leu Leu
    770                 775                 780

Thr His Ile Gln Asn Val Lys Glu Asp Arg Leu Lys Gln Leu Ser His
785                 790                 795                 800

Ala Ile Val Met Thr Ala Leu Gly Tyr Val Tyr Asp Glu Arg Lys Gln
                805                 810                 815

Glu Trp Cys Ala Glu Tyr Pro Ala Cys Gln Val Ile Leu Phe Glu Asn
            820                 825                 830

Leu Ser Gln Tyr Arg Ser Asn Leu Asp Arg Ser Thr Lys Glu Asn Ser
        835                 840                 845

Thr Leu Met Lys Trp Ala His Arg Ser Ile Pro Lys Tyr Val His Met
    850                 855                 860

Gln Ala Glu Pro Tyr Gly Ile Gln Ile Gly Asp Val Arg Ala Glu Tyr
865                 870                 875                 880

Ser Ser Arg Phe Tyr Ala Lys Thr Gly Thr Pro Gly Ile Arg Cys Lys
                885                 890                 895

Lys Val Arg Gly Gln Asp Leu Gln Gly Arg Arg Phe Glu Asn Leu Gln
            900                 905                 910

Lys Arg Leu Val Asn Glu Gln Phe Leu Thr Glu Glu Gln Val Lys Gln
        915                 920                 925

Leu Arg Pro Gly Asp Ile Val Pro Asp Asp Ser Gly Glu Leu Phe Met
    930                 935                 940

Thr Leu Thr Asp Gly Ser Gly Ser Lys Glu Val Val Phe Leu Gln Ala
945                 950                 955                 960

Asp Ile Asn Ala Ala His Asn Leu Gln Lys Arg Phe Trp Gln Arg Tyr
                965                 970                 975

Asn Glu Leu Phe Lys Val Ser Cys Arg Val Ile Val Arg Asp Glu Glu
            980                 985                 990

Glu Tyr Leu Val Pro Lys Thr Lys  Ser Val Gln Ala Lys  Leu Gly Lys
        995                 1000                1005

Gly Leu Phe Val Lys Lys Ser Asp Thr Ala Trp Lys Asp Val Tyr
    1010                1015                1020

Val Trp Asp Ser Gln Ala Lys Leu Lys Gly Lys Thr Thr Phe Thr
    1025                1030                1035

Glu Glu Ser Glu Ser Pro Glu Gln Leu Glu Asp Phe Gln Glu Ile
    1040                1045                1050

Ile Glu Glu Ala Glu Glu Ala Lys Gly Thr Tyr Arg Thr Leu Phe
    1055                1060                1065

Arg Asp Pro Ser Gly Val Phe Phe Pro Glu Ser Val Trp Tyr Pro
    1070                1075                1080

Gln Lys Asp Phe Trp Gly Glu Val Lys Arg Lys Leu Tyr Gly Lys
    1085                1090                1095

Leu Arg Glu Arg Phe Leu Thr Lys Ala Arg
    1100                1105

<210> 6
<211> 1112
<212> PRT
<213> Bacillus

<400> 6

Met Ala Ile Arg Ser Ile Lys Leu Lys Met Lys Thr Asn Ser Gly Thr
1               5                   10                  15

Asp Ser Ile Tyr Leu Arg Lys Ala Leu Trp Arg Thr His Gln Leu Ile
            20                  25                  30

Asn Glu Gly Ile Ala Tyr Tyr Met Asn Leu Leu Thr Leu Tyr Arg Gln
        35                  40                  45

Glu Ala Ile Gly Asp Lys Thr Lys Glu Ala Tyr Gln Ala Glu Leu Ile
    50                  55                  60

Asn Ile Ile Arg Asn Gln Gln Arg Asn Asn Gly Ser Ser Glu Glu His
65                  70                  75                  80

Gly Ser Asp Gln Glu Ile Leu Ala Leu Leu Arg Gln Leu Tyr Glu Leu
                85                  90                  95

Ile Ile Pro Ser Ser Ile Gly Glu Ser Gly Asp Ala Asn Gln Leu Gly
            100                 105                 110

Asn Lys Phe Leu Tyr Pro Leu Val Asp Pro Asn Ser Gln Ser Gly Lys
        115                 120                 125

Gly Thr Ser Asn Ala Gly Arg Lys Pro Arg Trp Lys Arg Leu Lys Glu
    130                 135                 140

Glu Gly Asn Pro Asp Trp Glu Leu Glu Lys Lys Lys Asp Glu Glu Arg
145                 150                 155                 160

Lys Ala Lys Asp Pro Thr Val Lys Ile Phe Asp Asn Leu Asn Lys Tyr
                165                 170                 175

Gly Leu Leu Pro Leu Phe Pro Leu Phe Thr Asn Ile Gln Lys Asp Ile
            180                 185                 190

Glu Trp Leu Pro Leu Gly Lys Arg Gln Ser Val Arg Lys Trp Asp Lys
        195                 200                 205

Asp Met Phe Ile Gln Ala Ile Glu Arg Leu Leu Ser Trp Glu Ser Trp
    210                 215                 220

Asn Arg Arg Val Ala Asp Glu Tyr Lys Gln Leu Lys Glu Lys Thr Glu
225                 230                 235                 240

Ser Tyr Tyr Lys Glu His Leu Thr Gly Gly Glu Glu Trp Ile Glu Lys
                245                 250                 255

Ile Arg Lys Phe Glu Lys Glu Arg Asn Met Glu Leu Glu Lys Asn Ala
            260                 265                 270

Phe Ala Pro Asn Asp Gly Tyr Phe Ile Thr Ser Arg Gln Ile Arg Gly
        275                 280                 285

Trp Asp Arg Val Tyr Glu Lys Trp Ser Lys Leu Pro Glu Ser Ala Ser
    290                 295                 300

Pro Glu Glu Leu Trp Lys Val Val Ala Glu Gln Gln Asn Lys Met Ser
305                 310                 315                 320

Glu Gly Phe Gly Asp Pro Lys Val Phe Ser Phe Leu Ala Asn Arg Glu
                325                 330                 335

Asn Arg Asp Ile Trp Arg Gly His Ser Glu Arg Ile Tyr His Ile Ala
            340                 345                 350

Ala Tyr Asn Gly Leu Gln Lys Lys Leu Ser Arg Thr Lys Glu Gln Ala

355                 360                 365

Thr Phe Thr Leu Pro Asp Ala Ile Glu His Pro Leu Trp Ile Arg Tyr
    370                 375                 380

Glu Ser Pro Gly Gly Thr Asn Leu Asn Leu Phe Lys Leu Glu Glu Lys
385                 390                 395                 400

Gln Lys Lys Asn Tyr Tyr Val Thr Leu Ser Lys Ile Ile Trp Pro Ser
                405                 410                 415

Glu Glu Lys Trp Ile Glu Lys Glu Asn Ile Glu Ile Pro Leu Ala Pro
            420                 425                 430

Ser Ile Gln Phe Asn Arg Gln Ile Lys Leu Lys Gln His Val Lys Gly
        435                 440                 445

Lys Gln Glu Ile Ser Phe Ser Asp Tyr Ser Ser Arg Ile Ser Leu Asp
    450                 455                 460

Gly Val Leu Gly Gly Ser Arg Ile Gln Phe Asn Arg Lys Tyr Ile Lys
465                 470                 475                 480

Asn His Lys Glu Leu Leu Gly Glu Gly Asp Ile Gly Pro Val Phe Phe
                485                 490                 495

Asn Leu Val Val Asp Val Ala Pro Leu Gln Glu Thr Arg Asn Gly Arg
            500                 505                 510

Leu Gln Ser Pro Ile Gly Lys Ala Leu Lys Val Ile Ser Ser Asp Phe
        515                 520                 525

Ser Lys Val Ile Asp Tyr Lys Pro Lys Glu Leu Met Asp Trp Met Asn
    530                 535                 540

Thr Gly Ser Ala Ser Asn Ser Phe Gly Val Ala Ser Leu Leu Glu Gly
545                 550                 555                 560

Met Arg Val Met Ser Ile Asp Met Gly Gln Arg Thr Ser Ala Ser Val
                565                 570                 575

Ser Ile Phe Glu Val Val Lys Glu Leu Pro Lys Asp Gln Glu Gln Lys
            580                 585                 590

Leu Phe Tyr Ser Ile Asn Asp Thr Glu Leu Phe Ala Ile His Lys Arg
        595                 600                 605

Ser Phe Leu Leu Asn Leu Pro Gly Glu Val Val Thr Lys Asn Asn Lys
    610                 615                 620

Gln Gln Arg Gln Glu Arg Arg Lys Lys Arg Gln Phe Val Arg Ser Gln
625                 630                 635                 640

Ile Arg Met Leu Ala Asn Val Leu Arg Leu Glu Thr Lys Lys Thr Pro
                645                 650                 655

Asp Glu Arg Lys Lys Ala Ile His Lys Leu Met Glu Ile Val Gln Ser
            660                 665                 670

Tyr Asp Ser Trp Thr Ala Ser Gln Lys Glu Val Trp Glu Lys Glu Leu
        675                 680                 685

Asn Leu Leu Thr Asn Met Ala Ala Phe Asn Asp Glu Ile Trp Lys Glu
    690                 695                 700

Ser Leu Val Glu Leu His His Arg Ile Glu Pro Tyr Val Gly Gln Ile
705                 710                 715                 720

Val Ser Lys Trp Arg Lys Gly Leu Ser Glu Gly Arg Lys Asn Leu Ala
                725                 730                 735

Gly Ile Ser Met Trp Asn Ile Asp Glu Leu Glu Asp Thr Arg Arg Leu
            740                 745                 750

Leu Ile Ser Trp Ser Lys Arg Ser Arg Thr Pro Gly Glu Ala Asn Arg
        755                 760                 765

Ile Glu Thr Asp Glu Pro Phe Gly Ser Ser Leu Leu Gln His Ile Gln
    770                 775                 780

Asn Val Lys Asp Asp Arg Leu Lys Gln Met Ala Asn Leu Ile Ile Met
785                 790                 795                 800

Thr Ala Leu Gly Phe Lys Tyr Asp Lys Glu Glu Lys Asp Arg Tyr Lys
                805                 810                 815

Arg Trp Lys Glu Thr Tyr Pro Ala Cys Gln Ile Ile Leu Phe Glu Asn
            820                 825                 830

Leu Asn Arg Tyr Leu Phe Asn Leu Asp Arg Ser Arg Arg Glu Asn Ser
        835                 840                 845

Arg Leu Met Lys Trp Ala His Arg Ser Ile Pro Arg Thr Val Ser Met
    850                 855                 860

Gln Gly Glu Met Phe Gly Leu Gln Val Gly Asp Val Arg Ser Glu Tyr
865                 870                 875                 880

Ser Ser Arg Phe His Ala Lys Thr Gly Ala Pro Gly Ile Arg Cys His
                885                 890                 895

Ala Leu Thr Glu Glu Asp Leu Lys Ala Gly Ser Asn Thr Leu Lys Arg
            900                 905                 910

Leu Ile Glu Asp Gly Phe Ile Asn Glu Ser Glu Leu Ala Tyr Leu Lys
        915                 920                 925

Lys Gly Asp Ile Ile Pro Ser Gln Gly Gly Glu Leu Phe Val Thr Leu
    930                 935                 940

Ser Lys Arg Tyr Lys Lys Asp Ser Asp Asn Asn Glu Leu Thr Val Ile
945                 950                 955                 960

His Ala Asp Ile Asn Ala Ala Gln Asn Leu Gln Lys Arg Phe Trp Gln
                965                 970                 975

Gln Asn Ser Glu Val Tyr Arg Val Pro Cys Gln Leu Ala Arg Met Gly
            980                 985                 990

Glu Asp Lys Leu Tyr Ile Pro Lys  Ser Gln Thr Glu Thr  Ile Lys Lys
        995                 1000                1005

Tyr Phe  Gly Lys Gly Ser Phe  Val Lys Asn Asn Thr  Glu Gln Glu
    1010                1015                1020

Val Tyr  Lys Trp Glu Lys Ser  Glu Lys Met Lys Ile  Lys Thr Asp
    1025                1030                1035

Thr Thr  Phe Asp Leu Gln Asp  Leu Asp Gly Phe Glu  Asp Ile Ser
    1040                1045                1050

Lys Thr  Ile Glu Leu Ala Gln  Glu Gln Gln Lys Lys  Tyr Leu Thr
    1055                1060                1065

Met Phe  Arg Asp Pro Ser Gly  Tyr Phe Phe Asn Asn  Glu Thr Trp
    1070                1075                1080

Arg Pro  Gln Lys Glu Tyr Trp  Ser Ile Val Asn Asn  Ile Ile Lys
    1085                1090                1095

Ser Cys  Leu Lys Lys Lys Ile  Leu Ser Asn Lys Val  Glu Leu
    1100                1105                1110

<210>  7
<211>  1149
<212>  PRT
<213>  Desulfovibrio inopinatus

<400>  7

Met Pro Thr Arg Thr Ile Asn Leu Lys Leu Val Leu Gly Lys Asn Pro
1               5                   10                  15

Glu Asn Ala Thr Leu Arg Arg Ala Leu Phe Ser Thr His Arg Leu Val
            20                  25                  30

Asn Gln Ala Thr Lys Arg Ile Glu Glu Phe Leu Leu Leu Cys Arg Gly
        35                  40                  45

Glu Ala Tyr Arg Thr Val Asp Asn Glu Gly Lys Glu Ala Glu Ile Pro
    50                  55                  60

Arg His Ala Val Gln Glu Glu Ala Leu Ala Phe Ala Lys Ala Ala Gln
65                  70                  75                  80

Arg His Asn Gly Cys Ile Ser Thr Tyr Glu Asp Gln Glu Ile Leu Asp
                85                  90                  95

Val Leu Arg Gln Leu Tyr Glu Arg Leu Val Pro Ser Val Asn Glu Asn
            100                 105                 110

Asn Glu Ala Gly Asp Ala Gln Ala Ala Asn Ala Trp Val Ser Pro Leu
        115                 120                 125

Met Ser Ala Glu Ser Glu Gly Gly Leu Ser Val Tyr Asp Lys Val Leu
    130                 135                 140

Asp Pro Pro Pro Val Trp Met Lys Leu Lys Glu Glu Lys Ala Pro Gly
145                 150                 155                 160

Trp Glu Ala Ala Ser Gln Ile Trp Ile Gln Ser Asp Glu Gly Gln Ser
                165                 170                 175

Leu Leu Asn Lys Pro Gly Ser Pro Pro Arg Trp Ile Arg Lys Leu Arg
            180                 185                 190

Ser Gly Gln Pro Trp Gln Asp Asp Phe Val Ser Asp Gln Lys Lys Lys
        195                 200                 205

Gln Asp Glu Leu Thr Lys Gly Asn Ala Pro Leu Ile Lys Gln Leu Lys
    210                 215                 220

Glu Met Gly Leu Leu Pro Leu Val Asn Pro Phe Phe Arg His Leu Leu
225                 230                 235                 240

Asp Pro Glu Gly Lys Gly Val Ser Pro Trp Asp Arg Leu Ala Val Arg
                245                 250                 255

Ala Ala Val Ala His Phe Ile Ser Trp Glu Ser Trp Asn His Arg Thr
            260                 265                 270

Arg Ala Glu Tyr Asn Ser Leu Lys Leu Arg Arg Asp Glu Phe Glu Ala
        275                 280                 285

Ala Ser Asp Glu Phe Lys Asp Asp Phe Thr Leu Leu Arg Gln Tyr Glu
    290                 295                 300

Ala Lys Arg His Ser Thr Leu Lys Ser Ile Ala Leu Ala Asp Asp Ser
305                 310                 315                 320

Asn Pro Tyr Arg Ile Gly Val Arg Ser Leu Arg Ala Trp Asn Arg Val
                325                 330                 335

Arg Glu Glu Trp Ile Asp Lys Gly Ala Thr Glu Glu Gln Arg Val Thr
            340                 345                 350

Ile Leu Ser Lys Leu Gln Thr Gln Leu Arg Gly Lys Phe Gly Asp Pro
        355                 360                 365

Asp Leu Phe Asn Trp Leu Ala Gln Asp Arg His Val His Leu Trp Ser
    370                 375                 380

Pro Arg Asp Ser Val Thr Pro Leu Val Arg Ile Asn Ala Val Asp Lys
385                 390                 395                 400

Val Leu Arg Arg Arg Lys Pro Tyr Ala Leu Met Thr Phe Ala His Pro
                405                 410                 415

Arg Phe His Pro Arg Trp Ile Leu Tyr Glu Ala Pro Gly Gly Ser Asn
            420                 425                 430

Leu Arg Gln Tyr Ala Leu Asp Cys Thr Glu Asn Ala Leu His Ile Thr
        435                 440                 445

Leu Pro Leu Leu Val Asp Asp Ala His Gly Thr Trp Ile Glu Lys Lys
    450                 455                 460

Ile Arg Val Pro Leu Ala Pro Ser Gly Gln Ile Gln Asp Leu Thr Leu

465                 470                 475                 480

Glu Lys Leu Glu Lys Lys Lys Asn Arg Leu Tyr Tyr Arg Ser Gly Phe
                485                 490                 495

Gln Gln Phe Ala Gly Leu Ala Gly Gly Ala Glu Val Leu Phe His Arg
            500                 505                 510

Pro Tyr Met Glu His Asp Glu Arg Ser Glu Glu Ser Leu Leu Glu Arg
        515                 520                 525

Pro Gly Ala Val Trp Phe Lys Leu Thr Leu Asp Val Ala Thr Gln Ala
    530                 535                 540

Pro Pro Asn Trp Leu Asp Gly Lys Gly Arg Val Arg Thr Pro Pro Glu
545                 550                 555                 560

Val His His Phe Lys Thr Ala Leu Ser Asn Lys Ser Lys His Thr Arg
                565                 570                 575

Thr Leu Gln Pro Gly Leu Arg Val Leu Ser Val Asp Leu Gly Met Arg
            580                 585                 590

Thr Phe Ala Ser Cys Ser Val Phe Glu Leu Ile Glu Gly Lys Pro Glu
        595                 600                 605

Thr Gly Arg Ala Phe Pro Val Ala Asp Glu Arg Ser Met Asp Ser Pro
    610                 615                 620

Asn Lys Leu Trp Ala Lys His Glu Arg Ser Phe Lys Leu Thr Leu Pro
625                 630                 635                 640

Gly Glu Thr Pro Ser Arg Lys Glu Glu Glu Glu Arg Ser Ile Ala Arg
                645                 650                 655

Ala Glu Ile Tyr Ala Leu Lys Arg Asp Ile Gln Arg Leu Lys Ser Leu
            660                 665                 670

Leu Arg Leu Gly Glu Glu Asp Asn Asp Asn Arg Arg Asp Ala Leu Leu
        675                 680                 685

Glu Gln Phe Phe Lys Gly Trp Gly Glu Glu Asp Val Val Pro Gly Gln
    690                 695                 700

Ala Phe Pro Arg Ser Leu Phe Gln Gly Leu Gly Ala Ala Pro Phe Arg
705                 710                 715                 720

Ser Thr Pro Glu Leu Trp Arg Gln His Cys Gln Thr Tyr Tyr Asp Lys
                725                 730                 735

Ala Glu Ala Cys Leu Ala Lys His Ile Ser Asp Trp Arg Lys Arg Thr
            740                 745                 750

Arg Pro Arg Pro Thr Ser Arg Glu Met Trp Tyr Lys Thr Arg Ser Tyr
        755                 760                 765

His Gly Gly Lys Ser Ile Trp Met Leu Glu Tyr Leu Asp Ala Val Arg
    770                 775                 780

Lys Leu Leu Leu Ser Trp Ser Leu Arg Gly Arg Thr Tyr Gly Ala Ile
785                 790                 795                 800

Asn Arg Gln Asp Thr Ala Arg Phe Gly Ser Leu Ala Ser Arg Leu Leu
                805                 810                 815

His His Ile Asn Ser Leu Lys Glu Asp Arg Ile Lys Thr Gly Ala Asp
            820                 825                 830

Ser Ile Val Gln Ala Ala Arg Gly Tyr Ile Pro Leu Pro His Gly Lys
        835                 840                 845

Gly Trp Glu Gln Arg Tyr Glu Pro Cys Gln Leu Ile Leu Phe Glu Asp
    850                 855                 860

Leu Ala Arg Tyr Arg Phe Arg Val Asp Arg Pro Arg Arg Glu Asn Ser
865                 870                 875                 880

Gln Leu Met Gln Trp Asn His Arg Ala Ile Val Ala Glu Thr Thr Met
                885                 890                 895

Gln Ala Glu Leu Tyr Gly Gln Ile Val Glu Asn Thr Ala Ala Gly Phe
            900                 905                 910

Ser Ser Arg Phe His Ala Ala Thr Gly Ala Pro Gly Val Arg Cys Arg
        915                 920                 925

Phe Leu Leu Glu Arg Asp Phe Asp Asn Asp Leu Pro Lys Pro Tyr Leu
    930                 935                 940

Leu Arg Glu Leu Ser Trp Met Leu Gly Asn Thr Lys Val Glu Ser Glu
945                 950                 955                 960

Glu Glu Lys Leu Arg Leu Leu Ser Glu Lys Ile Arg Pro Gly Ser Leu
                965                 970                 975

Val Pro Trp Asp Gly Gly Glu Gln Phe Ala Thr Leu His Pro Lys Arg
            980                 985                 990

Gln Thr Leu Cys Val Ile His Ala  Asp Met Asn Ala Ala  Gln Asn Leu
        995                 1000                1005

Gln Arg  Arg Phe Phe Gly Arg  Cys Gly Glu Ala Phe  Arg Leu Val
    1010                 1015                 1020

Cys Gln  Pro His Gly Asp Asp  Val Leu Arg Leu Ala  Ser Thr Pro
    1025                 1030                 1035

Gly Ala  Arg Leu Leu Gly Ala  Leu Gln Gln Leu Glu  Asn Gly Gln
    1040                 1045                 1050

Gly Ala  Phe Glu Leu Val Arg  Asp Met Gly Ser Thr  Ser Gln Met
    1055                 1060                 1065

Asn Arg  Phe Val Met Lys Ser  Leu Gly Lys Lys Lys  Ile Lys Pro
    1070                 1075                 1080

Leu Gln  Asp Asn Asn Gly Asp  Asp Glu Leu Glu Asp  Val Leu Ser
    1085                 1090                 1095

Val Leu  Pro Glu Glu Asp Asp  Thr Gly Arg Ile Thr  Val Phe Arg
    1100                 1105                 1110

Asp Ser  Ser Gly Ile Phe Phe  Pro Cys Asn Val Trp  Ile Pro Ala
    1115                 1120                 1125

Lys Gln  Phe Trp Pro Ala Val  Arg Ala Met Ile Trp  Lys Val Met
    1130                 1135                 1140

Ala Ser  His Ser Leu Gly
    1145

<210>  8
<211>  1090
<212>  PRT
<213>  Laceyella sediminis

<400>  8

Met Ser Ile Arg Ser Phe Lys Leu Lys Ile Lys Thr Lys Ser Gly Val
1               5                   10                  15

Asn Ala Glu Glu Leu Arg Arg Gly Leu Trp Arg Thr His Gln Leu Ile
            20                  25                  30

Asn Asp Gly Ile Ala Tyr Tyr Met Asn Trp Leu Val Leu Leu Arg Gln
        35                  40                  45

Glu Asp Leu Phe Ile Arg Asn Glu Glu Thr Asn Glu Ile Glu Lys Arg
    50                  55                  60

Ser Lys Glu Glu Ile Gln Gly Glu Leu Leu Glu Arg Val His Lys Gln
65                  70                  75                  80

Gln Gln Arg Asn Gln Trp Ser Gly Glu Val Asp Asp Gln Thr Leu Leu
                85                  90                  95

Gln Thr Leu Arg His Leu Tyr Glu Glu Ile Val Pro Ser Val Ile Gly
            100                 105                 110

Lys Ser Gly Asn Ala Ser Leu Lys Ala Arg Phe Phe Leu Gly Pro Leu
        115                 120                 125

Val Asp Pro Asn Asn Lys Thr Thr Lys Asp Val Ser Lys Ser Gly Pro
    130                 135                 140

Thr Pro Lys Trp Lys Lys Met Lys Asp Ala Gly Asp Pro Asn Trp Val
145                 150                 155                 160

Gln Glu Tyr Glu Lys Tyr Met Ala Glu Arg Gln Thr Leu Val Arg Leu
                165                 170                 175

Glu Glu Met Gly Leu Ile Pro Leu Phe Pro Met Tyr Thr Asp Glu Val
            180                 185                 190

Gly Asp Ile His Trp Leu Pro Gln Ala Ser Gly Tyr Thr Arg Thr Trp
        195                 200                 205

Asp Arg Asp Met Phe Gln Gln Ala Ile Glu Arg Leu Leu Ser Trp Glu
    210                 215                 220

Ser Trp Asn Arg Arg Val Arg Glu Arg Arg Ala Gln Phe Glu Lys Lys
225                 230                 235                 240

Thr His Asp Phe Ala Ser Arg Phe Ser Glu Ser Asp Val Gln Trp Met
                245                 250                 255

Asn Lys Leu Arg Glu Tyr Glu Ala Gln Gln Glu Lys Ser Leu Glu Glu
            260                 265                 270

Asn Ala Phe Ala Pro Asn Glu Pro Tyr Ala Leu Thr Lys Lys Ala Leu
        275                 280                 285

Arg Gly Trp Glu Arg Val Tyr His Ser Trp Met Arg Leu Asp Ser Ala
    290                 295                 300

Ala Ser Glu Glu Ala Tyr Trp Gln Glu Val Ala Thr Cys Gln Thr Ala
305                 310                 315                 320

Met Arg Gly Glu Phe Gly Asp Pro Ala Ile Tyr Gln Phe Leu Ala Gln
                325                 330                 335

Lys Glu Asn His Asp Ile Trp Arg Gly Tyr Pro Glu Arg Val Ile Asp
            340                 345                 350

Phe Ala Glu Leu Asn His Leu Gln Arg Glu Leu Arg Arg Ala Lys Glu
        355                 360                 365

Asp Ala Thr Phe Thr Leu Pro Asp Ser Val Asp His Pro Leu Trp Val
    370                 375                 380

Arg Tyr Glu Ala Pro Gly Gly Thr Asn Ile His Gly Tyr Asp Leu Val
385                 390                 395                 400

Gln Asp Thr Lys Arg Asn Leu Thr Leu Ile Leu Asp Lys Phe Ile Leu
                405                 410                 415

Pro Asp Glu Asn Gly Ser Trp His Glu Val Lys Lys Val Pro Phe Ser
            420                 425                 430

Leu Ala Lys Ser Lys Gln Phe His Arg Gln Val Trp Leu Gln Glu Glu
        435                 440                 445

Gln Lys Gln Lys Lys Arg Glu Val Val Phe Tyr Asp Tyr Ser Thr Asn
    450                 455                 460

Leu Pro His Leu Gly Thr Leu Ala Gly Ala Lys Leu Gln Trp Asp Arg
465                 470                 475                 480

Asn Phe Leu Asn Lys Arg Thr Gln Gln Gln Ile Glu Glu Thr Gly Glu
                485                 490                 495

Ile Gly Lys Val Phe Phe Asn Ile Ser Val Asp Val Arg Pro Ala Val
            500                 505                 510

Glu Val Lys Asn Gly Arg Leu Gln Asn Gly Leu Gly Lys Ala Leu Thr
        515                 520                 525

Val Leu Thr His Pro Asp Gly Thr Lys Ile Val Thr Gly Trp Lys Ala

530                 535                 540

Glu Gln Leu Glu Lys Trp Val Gly Glu Ser Gly Arg Val Ser Ser Leu
545                 550                 555                 560

Gly Leu Asp Ser Leu Ser Glu Gly Leu Arg Val Met Ser Ile Asp Leu
                565                 570                 575

Gly Gln Arg Thr Ser Ala Thr Val Ser Val Phe Glu Ile Thr Lys Glu
            580                 585                 590

Ala Pro Asp Asn Pro Tyr Lys Phe Phe Tyr Gln Leu Glu Gly Thr Glu
        595                 600                 605

Leu Phe Ala Val His Gln Arg Ser Phe Leu Leu Ala Leu Pro Gly Glu
    610                 615                 620

Asn Pro Pro Gln Lys Ile Lys Gln Met Arg Glu Ile Arg Trp Lys Glu
625                 630                 635                 640

Arg Asn Arg Ile Lys Gln Gln Val Asp Gln Leu Ser Ala Ile Leu Arg
                645                 650                 655

Leu His Lys Lys Val Asn Glu Asp Glu Arg Ile Gln Ala Ile Asp Lys
            660                 665                 670

Leu Leu Gln Lys Val Ala Ser Trp Gln Leu Asn Glu Glu Ile Ala Thr
        675                 680                 685

Ala Trp Asn Gln Ala Leu Ser Gln Leu Tyr Ser Lys Ala Lys Glu Asn
    690                 695                 700

Asp Leu Gln Trp Asn Gln Ala Ile Lys Asn Ala His His Gln Leu Glu
705                 710                 715                 720

Pro Val Val Gly Lys Gln Ile Ser Leu Trp Arg Lys Asp Leu Ser Thr
                725                 730                 735

Gly Arg Gln Gly Ile Ala Gly Leu Ser Leu Trp Ser Ile Glu Glu Leu
            740                 745                 750

Glu Ala Thr Lys Lys Leu Leu Thr Arg Trp Ser Lys Arg Ser Arg Glu
        755                 760                 765

Pro Gly Val Val Lys Arg Ile Glu Arg Phe Glu Thr Phe Ala Lys Gln
    770                 775                 780

Ile Gln His His Ile Asn Gln Val Lys Glu Asn Arg Leu Lys Gln Leu
785                 790                 795                 800

Ala Asn Leu Ile Val Met Thr Ala Leu Gly Tyr Lys Tyr Asp Gln Glu
                805                 810                 815

Gln Lys Lys Trp Ile Glu Val Tyr Pro Ala Cys Gln Val Val Leu Phe
            820                 825                 830

Glu Asn Leu Arg Ser Tyr Arg Phe Ser Tyr Glu Arg Ser Arg Arg Glu
        835                 840                 845

Asn Lys Lys Leu Met Glu Trp Ser His Arg Ser Ile Pro Lys Leu Val
    850                 855                 860

Gln Met Gln Gly Glu Leu Phe Gly Leu Gln Val Ala Asp Val Tyr Ala
865                 870                 875                 880

Ala Tyr Ser Ser Arg Tyr His Gly Arg Thr Gly Ala Pro Gly Ile Arg
                885                 890                 895

Cys His Ala Leu Thr Glu Ala Asp Leu Arg Asn Glu Thr Asn Ile Ile
            900                 905                 910

His Glu Leu Ile Glu Ala Gly Phe Ile Lys Glu Glu His Arg Pro Tyr
        915                 920                 925

Leu Gln Gln Gly Asp Leu Val Pro Trp Ser Gly Gly Glu Leu Phe Ala
    930                 935                 940

Thr Leu Gln Lys Pro Tyr Asp Asn Pro Arg Ile Leu Thr Leu His Ala
945                 950                 955                 960

Asp Ile Asn Ala Ala Gln Asn Ile Gln Lys Arg Phe Trp His Pro Ser
                965                 970                 975

Met Trp Phe Arg Val Asn Cys Glu Ser Val Met Glu Gly Glu Ile Val
            980                 985                 990

Thr Tyr Val Pro Lys Asn Lys Thr  Val His Lys Lys Gln  Gly Lys Thr
        995                 1000                 1005

Phe Arg  Phe Val Lys Val Glu  Gly Ser Asp Val Tyr  Glu Trp Ala
    1010                 1015                 1020

Lys Trp  Ser Lys Asn Arg Asn  Lys Asn Thr Phe Ser  Ser Ile Thr
    1025                 1030                 1035

Glu Arg Lys Pro Pro Ser Ser Met Ile Leu Phe Arg Asp Pro Ser
    1040                1045                1050

Gly Thr Phe Phe Lys Glu Gln Glu Trp Val Glu Gln Lys Thr Phe
    1055                1060                1065

Trp Gly Lys Val Gln Ser Met Ile Gln Ala Tyr Met Lys Lys Thr
    1070                1075                1080

Ile Val Gln Arg Met Glu Glu
    1085                1090

<210> 9
<211> 1119
<212> PRT
<213> Spirochaetes

<400> 9

Met Ser Phe Thr Ile Ser Tyr Pro Phe Lys Leu Ile Ile Lys Asn Lys
1               5                   10                  15

Asp Glu Ala Lys Ala Leu Leu Asp Thr His Gln Tyr Met Asn Glu Gly
            20                  25                  30

Val Lys Tyr Tyr Leu Glu Lys Leu Leu Met Phe Arg Gln Glu Lys Ile
        35                  40                  45

Phe Ile Gly Glu Asp Glu Thr Gly Lys Arg Ile Tyr Ile Glu Glu Thr
    50                  55                  60

Glu Tyr Lys Lys Gln Ile Glu Glu Phe Tyr Leu Ile Lys Lys Thr Glu
65                  70                  75                  80

Leu Gly Arg Asn Leu Thr Leu Thr Leu Asp Glu Phe Lys Thr Leu Met
                85                  90                  95

Arg Glu Leu Tyr Ile Cys Leu Val Ser Ser Ser Met Glu Asn Lys Lys
            100                 105                 110

Gly Phe Pro Asn Ala Gln Gln Ala Ser Leu Asn Ile Phe Ser Pro Leu
        115                 120                 125

Phe Asp Ala Glu Ser Lys Gly Tyr Ile Leu Lys Glu Glu Asn Asn Asn
    130                 135                 140

Ile Ser Leu Ile His Lys Asp Tyr Gly Lys Ile Leu Leu Lys Arg Leu
145                 150                 155                 160

Arg Asp Asn Asn Leu Ile Pro Ile Phe Thr Lys Phe Thr Asp Ile Lys
                165                 170                 175

Lys Ile Thr Ala Lys Leu Ser Pro Thr Ala Leu Asp Arg Met Ile Phe
            180                 185                 190

Ala Gln Ala Ile Glu Lys Leu Leu Ser Tyr Glu Ser Trp Cys Lys Leu
        195                 200                 205

Met Ile Lys Glu Arg Phe Asp Lys Glu Val Lys Ile Lys Glu Leu Glu
    210                 215                 220

Asn Lys Cys Glu Asn Lys Gln Glu Arg Asp Lys Ile Phe Glu Ile Leu
225                 230                 235                 240

Glu Lys Tyr Glu Glu Glu Arg Gln Lys Thr Phe Glu Gln Asp Ser Gly
                245                 250                 255

Phe Ala Lys Lys Gly Lys Phe Tyr Ile Thr Gly Arg Met Leu Lys Gly
            260                 265                 270

Phe Asp Glu Ile Lys Glu Lys Trp Leu Lys Glu Lys Asp Arg Ser Glu
        275                 280                 285

Gln Asn Leu Ile Asn Ile Leu Asn Lys Tyr Gln Thr Asp Asn Ser Lys
    290                 295                 300

Leu Val Gly Asp Arg Asn Leu Phe Glu Phe Ile Ile Lys Leu Glu Asn
305                 310                 315                 320

Gln Cys Leu Trp Asn Gly Asp Ile Asp Tyr Leu Lys Ile Lys Arg Asp
                325                 330                 335

Ile Asn Lys Asn Gln Ile Trp Leu Asp Arg Pro Glu Met Pro Arg Phe
            340                 345                 350

Thr Met Pro Asp Phe Lys Lys His Pro Leu Trp Tyr Arg Tyr Glu Asp
        355                 360                 365

Pro Ser Asn Ser Asn Phe Arg Asn Tyr Lys Ile Glu Val Val Lys Asp
    370                 375                 380

Glu Asn Tyr Ile Thr Ile Pro Leu Ile Thr Glu Arg Asn Asn Glu Tyr
385                 390                 395                 400

Phe Glu Glu Asn Tyr Thr Phe Asn Leu Ala Lys Leu Lys Lys Leu Ser
                405                 410                 415

Glu Asn Ile Thr Phe Ile Pro Lys Ser Lys Asn Lys Glu Phe Glu Phe
            420                 425                 430

Ile Asp Ser Asn Asp Glu Glu Glu Asp Lys Lys Asp Gln Lys Lys Ser
        435                 440                 445

Lys Gln Tyr Ile Lys Tyr Cys Asp Thr Ala Lys Asn Thr Ser Tyr Gly
    450                 455                 460

Lys Ser Gly Gly Ile Arg Leu Tyr Phe Asn Arg Asn Glu Leu Glu Asn
465                 470                 475                 480

Tyr Lys Asp Gly Lys Lys Met Asp Ser Tyr Thr Val Phe Thr Leu Ser
                485                 490                 495

Ile Arg Asp Tyr Lys Ser Leu Phe Ala Lys Glu Lys Leu Gln Pro Gln
            500                 505                 510

Ile Phe Asn Thr Val Asp Asn Lys Ile Thr Ser Leu Lys Ile Gln Lys
        515                 520                 525

Lys Phe Gly Asn Glu Glu Gln Thr Asn Phe Leu Ser Tyr Phe Thr Gln
    530                 535                 540

Asn Gln Ile Thr Lys Lys Asp Trp Met Asp Glu Lys Thr Phe Gln Asn
545                 550                 555                 560

Val Lys Glu Leu Asn Glu Gly Ile Arg Val Leu Ser Val Asp Leu Gly
                565                 570                 575

Gln Arg Phe Phe Ala Ala Val Ser Cys Phe Glu Ile Met Ser Glu Ile
            580                 585                 590

Asp Asn Asn Lys Leu Phe Phe Asn Leu Asn Asp Gln Asn His Lys Ile
        595                 600                 605

Ile Arg Ile Asn Asp Lys Asn Tyr Tyr Ala Lys His Ile Tyr Ser Lys
    610                 615                 620

Thr Ile Lys Leu Ser Gly Glu Asp Asp Asp Leu Tyr Lys Glu Arg Lys
625                 630                 635                 640

Ile Asn Lys Asn Tyr Lys Leu Ser Tyr Gln Glu Arg Lys Asn Lys Ile
                645                 650                 655

Gly Ile Phe Thr Arg Gln Ile Asn Lys Leu Asn Gln Leu Leu Lys Ile

            660                 665                 670

Ile Arg Asn Asp Glu Ile Asp Lys Glu Lys Phe Lys Glu Leu Ile Glu
        675                 680                 685

Thr Thr Lys Arg Tyr Val Lys Asn Thr Tyr Asn Asp Gly Ile Ile Asp
    690                 695                 700

Trp Asn Asn Val Asp Asn Lys Ile Leu Ser Tyr Glu Asn Lys Glu Asp
705                 710                 715                 720

Val Ile Asn Leu His Lys Glu Leu Asp Lys Lys Leu Glu Ile Asp Phe
                725                 730                 735

Lys Glu Phe Ile Arg Glu Cys Arg Lys Pro Ile Phe Arg Ser Gly Gly
            740                 745                 750

Leu Ser Met Gln Arg Ile Asp Phe Leu Glu Lys Leu Asn Lys Leu Lys
        755                 760                 765

Arg Lys Trp Val Ala Arg Thr Gln Lys Ser Ala Glu Ser Ile Val Leu
    770                 775                 780

Thr Pro Lys Phe Gly Tyr Lys Leu Lys Glu His Ile Asn Glu Leu Lys
785                 790                 795                 800

Asp Asn Arg Val Lys Gln Gly Val Asn Tyr Ile Leu Met Thr Ala Leu
                805                 810                 815

Gly Tyr Ile Lys Asp Asn Glu Ile Lys Asn Asp Ser Lys Lys Lys Gln
            820                 825                 830

Lys Glu Asp Trp Val Lys Lys Asn Arg Ala Cys Gln Ile Ile Leu Met
        835                 840                 845

Glu Lys Leu Thr Glu Tyr Thr Phe Ala Glu Asp Arg Pro Arg Glu Glu
    850                 855                 860

Asn Ser Lys Leu Arg Met Trp Ser His Arg Gln Ile Phe Asn Phe Leu
865                 870                 875                 880

Gln Gln Lys Ala Ser Leu Trp Gly Ile Leu Val Gly Asp Val Phe Ala
                885                 890                 895

Pro Tyr Thr Ser Lys Cys Leu Ser Asp Asn Asn Ala Pro Gly Ile Arg
            900                 905                 910

Cys His Gln Val Thr Lys Lys Asp Leu Ile Asp Asn Ser Trp Phe Leu
        915                 920                 925

Lys Ile Val Val Lys Asp Asp Ala Phe Cys Asp Leu Ile Glu Ile Asn
    930                 935                 940

Lys Glu Asn Val Lys Asn Lys Ser Ile Lys Ile Asn Asp Ile Leu Pro
945                 950                 955                 960

Leu Arg Gly Gly Glu Leu Phe Ala Ser Ile Lys Asp Gly Lys Leu His
                965                 970                 975

Ile Val Gln Ala Asp Ile Asn Ala Ser Arg Asn Ile Ala Lys Arg Phe
            980                 985                 990

Leu Ser Gln Ile Asn Pro Phe Arg Val Val Leu Lys Lys Asp Lys Asp
        995                 1000                1005

Glu Thr Phe His Leu Lys Asn Glu Pro Asn Tyr Leu Lys Asn Tyr
    1010                1015                1020

Tyr Ser Ile Leu Asn Phe Val Pro Thr Asn Glu Glu Leu Thr Phe
    1025                1030                1035

Phe Lys Val Glu Glu Asn Lys Asp Ile Lys Pro Thr Lys Arg Ile
    1040                1045                1050

Lys Met Asp Lys His Glu Lys Glu Ser Thr Asp Glu Gly Asp Asp
    1055                1060                1065

Tyr Ser Lys Asn Gln Ile Ala Leu Phe Arg Asp Asp Ser Gly Ile
    1070                1075                1080

Phe Phe Asp Lys Ser Leu Trp Val Asp Gly Lys Ile Phe Trp Ser
    1085                1090                1095

Val Val Lys Asn Lys Met Thr Lys Leu Leu Arg Glu Arg Asn Asn
    1100                1105                1110

Lys Lys Asn Gly Ser Lys
    1115

<210> 10
<211> 1142
<212> PRT
<213> Tuberibacillus calidus

<400> 10

Met Asn Ile His Leu Lys Glu Leu Ile Arg Met Ala Thr Lys Ser Phe
1               5                   10                  15

Ile Leu Lys Met Lys Thr Lys Asn Asn Pro Gln Leu Arg Leu Ser Leu
            20                  25                  30

Trp Lys Thr His Glu Leu Phe Asn Phe Gly Val Ala Tyr Tyr Met Asp
        35                  40                  45

Leu Leu Ser Leu Phe Arg Gln Lys Asp Leu Tyr Met His Asn Asp Glu
    50                  55                  60

Asp Pro Asp His Pro Val Val Leu Lys Lys Glu Glu Ile Gln Glu Arg
65                  70                  75                  80

Leu Trp Met Lys Val Arg Glu Thr Gln Gln Lys Asn Gly Phe His Gly
                85                  90                  95

Glu Val Ser Lys Asp Glu Val Leu Glu Thr Leu Arg Ala Leu Tyr Glu
            100                 105                 110

Glu Leu Val Pro Ser Ala Val Gly Lys Ser Gly Glu Ala Asn Gln Ile
        115                 120                 125

Ser Asn Lys Tyr Leu Tyr Pro Leu Thr Asp Pro Ala Ser Gln Ser Gly
    130                 135                 140

Lys Gly Thr Ala Asn Ser Gly Arg Lys Pro Arg Trp Lys Lys Leu Lys
145                 150                 155                 160

Glu Ala Gly Asp Pro Ser Trp Lys Asp Ala Tyr Glu Lys Trp Glu Lys
                165                 170                 175

Glu Arg Gln Glu Asp Pro Lys Leu Lys Ile Leu Ala Ala Leu Gln Ser
            180                 185                 190

Phe Gly Leu Ile Pro Leu Phe Arg Pro Phe Thr Glu Asn Asp His Lys
        195                 200                 205

Ala Val Ile Ser Val Lys Trp Met Pro Lys Ser Lys Asn Gln Ser Val
    210                 215                 220

Arg Lys Phe Asp Lys Asp Met Phe Asn Gln Ala Ile Glu Arg Phe Leu
225                 230                 235                 240

Ser Trp Glu Ser Trp Asn Glu Lys Val Ala Glu Asp Tyr Glu Lys Thr
                245                 250                 255

Val Ser Ile Tyr Glu Ser Leu Gln Lys Glu Leu Lys Gly Ile Ser Thr
            260                 265                 270

Lys Ala Phe Glu Ile Met Glu Arg Val Glu Lys Ala Tyr Glu Ala His
        275                 280                 285

Leu Arg Glu Ile Thr Phe Ser Asn Ser Thr Tyr Arg Ile Gly Asn Arg
    290                 295                 300

Ala Ile Arg Gly Trp Thr Glu Ile Val Lys Lys Trp Met Lys Leu Asp
305                 310                 315                 320

Pro Ser Ala Pro Gln Gly Asn Tyr Leu Asp Val Val Lys Asp Tyr Gln
                325                 330                 335

Arg Arg His Pro Arg Glu Ser Gly Asp Phe Lys Leu Phe Glu Leu Leu
            340                 345                 350

Ser Arg Pro Glu Asn Gln Ala Ala Trp Arg Glu Tyr Pro Glu Phe Leu
        355                 360                 365

Pro Leu Tyr Val Lys Tyr Arg His Ala Glu Gln Arg Met Lys Thr Ala
    370                 375                 380

Lys Lys Gln Ala Thr Phe Thr Leu Cys Asp Pro Ile Arg His Pro Leu
385                 390                 395                 400

Trp Val Arg Tyr Glu Glu Arg Ser Gly Thr Asn Leu Asn Lys Tyr Arg
                405                 410                 415

Leu Ile Met Asn Glu Lys Glu Lys Val Val Gln Phe Asp Arg Leu Ile
            420                 425                 430

Cys Leu Asn Ala Asp Gly His Tyr Glu Glu Gln Glu Asp Val Thr Val
        435                 440                 445

Pro Leu Ala Pro Ser Gln Gln Phe Asp Asp Gln Ile Lys Phe Ser Ser
    450                 455                 460

Glu Asp Thr Gly Lys Gly Lys His Asn Phe Ser Tyr Tyr His Lys Gly
465                 470                 475                 480

Ile Asn Tyr Glu Leu Lys Gly Thr Leu Gly Gly Ala Arg Ile Gln Phe
                485                 490                 495

Asp Arg Glu His Leu Leu Arg Arg Gln Gly Val Lys Ala Gly Asn Val
            500                 505                 510

Gly Arg Ile Phe Leu Asn Val Thr Leu Asn Ile Glu Pro Met Gln Pro
        515                 520                 525

Phe Ser Arg Ser Gly Asn Leu Gln Thr Ser Val Gly Lys Ala Leu Lys
    530                 535                 540

Val Tyr Val Asp Gly Tyr Pro Lys Val Val Asn Phe Lys Pro Lys Glu
545                 550                 555                 560

Leu Thr Glu His Ile Lys Glu Ser Glu Lys Asn Thr Leu Thr Leu Gly
                565                 570                 575

Val Glu Ser Leu Pro Thr Gly Leu Arg Val Met Ser Val Asp Leu Gly
            580                 585                 590

Gln Arg Gln Ala Ala Ala Ile Ser Ile Phe Glu Val Val Ser Glu Lys
        595                 600                 605

Pro Asp Asp Asn Lys Leu Phe Tyr Pro Val Lys Asp Thr Asp Leu Phe
    610                 615                 620

Ala Val His Arg Thr Ser Phe Asn Ile Lys Leu Pro Gly Glu Lys Arg
625                 630                 635                 640

Thr Glu Arg Arg Met Leu Glu Gln Gln Lys Arg Asp Gln Ala Ile Arg
                645                 650                 655

Asp Leu Ser Arg Lys Leu Lys Phe Leu Lys Asn Val Leu Asn Met Gln
            660                 665                 670

Lys Leu Glu Lys Thr Asp Glu Arg Glu Lys Arg Val Asn Arg Trp Ile
        675                 680                 685

Lys Asp Arg Glu Arg Glu Glu Glu Asn Pro Val Tyr Val Gln Glu Phe
    690                 695                 700

Glu Met Ile Ser Lys Val Leu Tyr Ser Pro His Ser Val Trp Val Asp
705                 710                 715                 720

Gln Leu Lys Ser Ile His Arg Lys Leu Glu Glu Gln Leu Gly Lys Glu
                725                 730                 735

Ile Ser Lys Trp Arg Gln Ser Ile Ser Gln Gly Arg Gln Gly Val Tyr
            740                 745                 750

Gly Ile Ser Leu Lys Asn Ile Glu Asp Ile Glu Lys Thr Arg Arg Leu

755                 760                 765

Leu Phe Arg Trp Ser Met Arg Pro Glu Asn Pro Gly Glu Val Lys Gln
    770                 775                 780

Leu Gln Pro Gly Glu Arg Phe Ala Ile Asp Gln Gln Asn His Leu Asn
785                 790                 795                 800

His Leu Lys Asp Asp Arg Ile Lys Lys Leu Ala Asn Gln Ile Val Met
                805                 810                 815

Thr Ala Leu Gly Tyr Arg Tyr Asp Gly Lys Arg Lys Lys Trp Ile Ala
            820                 825                 830

Lys His Pro Ala Cys Gln Leu Val Leu Phe Glu Asp Leu Ser Arg Tyr
        835                 840                 845

Ala Phe Tyr Asp Glu Arg Ser Arg Leu Glu Asn Arg Asn Leu Met Arg
    850                 855                 860

Trp Ser Arg Arg Glu Ile Pro Lys Gln Val Ala Gln Ile Gly Gly Leu
865                 870                 875                 880

Tyr Gly Leu Leu Val Gly Glu Val Gly Ala Gln Tyr Ser Ser Arg Phe
                885                 890                 895

His Ala Lys Ser Gly Ala Pro Gly Ile Arg Cys Arg Val Val Lys Glu
            900                 905                 910

His Glu Leu Tyr Ile Thr Glu Gly Gly Gln Lys Val Arg Asn Gln Lys
        915                 920                 925

Phe Leu Asp Ser Leu Val Glu Asn Asn Ile Ile Glu Pro Asp Asp Ala
    930                 935                 940

Arg Arg Leu Glu Pro Gly Asp Leu Ile Arg Asp Gln Gly Gly Asp Lys
945                 950                 955                 960

Phe Ala Thr Leu Asp Glu Arg Gly Glu Leu Val Ile Thr His Ala Asp
                965                 970                 975

Ile Asn Ala Ala Gln Asn Leu Gln Lys Arg Phe Trp Thr Arg Thr His
            980                 985                 990

Gly Leu Tyr Arg Ile Arg Cys Glu  Ser Arg Glu Ile Lys  Asp Ala Val
        995                 1000                1005

Val Leu Val Pro Ser Asp Lys Asp Gln Lys Glu Lys Met Glu Asn
    1010                1015                1020

Leu Phe Gly Ile Gly Tyr Leu Gln Pro Phe Lys Gln Glu Asn Asp
    1025                1030                1035

Val Tyr Lys Trp Val Lys Gly Glu Lys Ile Lys Gly Lys Lys Thr
    1040                1045                1050

Ser Ser Gln Ser Asp Asp Lys Glu Leu Val Ser Glu Ile Leu Gln
    1055                1060                1065

Glu Ala Ser Val Met Ala Asp Glu Leu Lys Gly Asn Arg Lys Thr
    1070                1075                1080

Leu Phe Arg Asp Pro Ser Gly Tyr Val Phe Pro Lys Asp Arg Trp
    1085                1090                1095

Tyr Thr Gly Gly Arg Tyr Phe Gly Thr Leu Glu His Leu Leu Lys
    1100                1105                1110

Arg Lys Leu Ala Glu Arg Arg Leu Phe Asp Gly Gly Ser Ser Arg
    1115                1120                1125

Arg Gly Leu Phe Asn Gly Thr Asp Ser Asn Thr Asn Val Glu
    1130                1135                1140

<210>  11
<211>  3387
<212>  DNA
<213>  Alicyclobacillus acidiphilus

<400>  11
atggccgtga agagcatgaa ggtgaagctg cgcctggaca acatgcccga gatccgcgcc      60

ggcctgtgga agctgcacac cgaggtgaac gccggcgtgc gctactacac cgagtggctg     120

agcctgctgc gccaggagaa cctgtaccgc cgcagcccca acggcgacgg cgagcaggag     180

tgctacaaga ccgccgagga gtgcaaggcc gagctgctgg agcgcctgcg cgcccgccag     240

gtggagaacg gccactgcgg ccccgccggc agcgacgacg agctgctgca gctggcccgc     300

cagctgtacg agctgctggt gccccaggcc atcggcgcca agggcgacgc ccagcagatc     360

gcccgcaagt tcctgagccc cctggccgac aaggacgccg tgggcggcct gggcatcgcc     420

aaggccggca acaagccccg ctgggtgcgc atgcgcgagg ccggcgagcc cggctgggag     480

gaggagaagg ccaaggccga ggcccgcaag agcaccgacc gcaccgccga cgtgctgcgc     540

gccctggccg acttcggcct gaagcccctg atgcgcgtgt acaccgacag cgacatgagc     600

agcgtgcagt ggaagcccct gcgcaagggc caggccgtgc gcacctggga ccgcgacatg     660

ttccagcagg ccatcgagcg catgatgagc tgggagagct ggaaccagcg cgtgggcgag 720

gcctacgcca agctggtgga gcagaagagc cgcttcgagc agaagaactt cgtgggccag 780

gagcacctgg tgcagctggt gaaccagctg cagcaggaca tgaaggaggc cagccacggc 840

ctggagagca aggagcagac cgcccactac ctgaccggcc gcgccctgcg cggcagcgac 900

aaggtgttcg agaagtggga gaagctggac cccgacgccc ccttcgacct gtacgacacc 960

gagatcaaga acgtgcagcg ccgcaacacc cgccgcttcg gcagccacga cctgttcgcc 1020

aagctggccg agcccaagta ccaggccctg tggcgcgagg acgccagctt cctgacccgc 1080

tacgccgtgt acaacagcat cgtgcgcaag ctgaaccacg ccaagatgtt cgccaccttc 1140

accctgcccg acgccaccgc ccaccccatc tggacccgct tcgacaagct gggcggcaac 1200

ctgcaccagt acaccttcct gttcaacgag ttcggcgagg gccgccacgc catccgcttc 1260

cagaagctgc tgaccgtgga ggacggcgtg gccaaggagg tggacgacgt gaccgtgccc 1320

atcagcatga gcgcccagct ggacgacctg ctgccccgcg acccccacga gctggtggcc 1380

ctgtacttcc aggactacgg cgccgagcag cacctggccg gcgagttcgg cggcgccaag 1440

atccagtacc gccgcgacca gctgaaccac ctgcacgccc gccgcggcgc ccgcgacgtg 1500

tacctgaacc tgagcgtgcg cgtgcagagc cagagcgagg cccgcggcga gcgccgcccc 1560

ccctacgccg ccgtgttccg cctggtgggc gacaaccacc gcgccttcgt gcacttcgac 1620

aagctgagcg actacctggc cgagcacccc gacgacggca agctgggcag cgagggcctg 1680

ctgagcggcc tgcgcgtgat gagcgtggac ctgggcctgc gcaccagcgc cagcatcagc 1740

gtgttccgcg tggcccgcaa ggacgagctg aagcccaaca gcgagggccg cgtgcccttc 1800

tgcttcccca tcgagggcaa cgagaacctg gtggccgtgc acgagcgcag ccagctgctg 1860

aagctgcccg gcgagaccga gagcaaggac ctgcgcgcca tccgcgagga gcgccagcgc 1920

accctgcgcc agctgcgcac ccagctggcc tacctgcgcc tgctggtgcg ctgcggcagc 1980

gaggacgtgg gccgccgcga gcgcagctgg gccaagctga tcgagcagcc catggacgcc 2040

aaccagatga cccccgactg gcgcgaggcc ttcgaggacg agctgcagaa gctgaagagc 2100

ctgtacggca tctgcggcga ccgcgagtgg accgaggccg tgtacgagag cgtgcgccgc 2160

gtgtggcgcc acatgggcaa gcaggtgcgc gactggcgca aggacgtgcg cagcggcgag 2220

cgccccaaga tccgcggcta ccagaaggac gtggtgggcg gcaacagcat cgagcagatc 2280

gagtacctgg agcgccagta caagttcctg aagagctgga gcttcttcgg caaggtgagc 2340

ggccaggtga tccgcgccga gaagggcagc cgcttcgcca tcaccctgcg cgagcacatc 2400

gaccacgcca aggaggaccg cctgaagaag ctggccgacc gcatcatcat ggaggccctg 2460

ggctacgtgt acgccctgga cgacgagcgc ggcaagggca agtgggtggc caagtacccc 2520

ccctgccagc tgatcctgct ggaggagctg agcgagtacc agttcaacaa cgaccgcccc 2580

cccagcgaga acaaccagct gatgcagtgg agccaccgcg gcgtgttcca ggagctgctg 2640

aaccaggccc aggtgcacga cctgctggtg ggcaccatgt acgccgcctt cagcagccgc 2700

ttcgacgccc gcaccggcgc ccccggcatc cgctgccgcc gcgtgcccgc ccgctgcgcc 2760

cgcgagcaga accccgagcc cttcccctgg tggctgaaca agttcgtggc cgagcacaag 2820

ctggacggct gccccctgcg cgccgacgac ctgatcccca ccggcgaggg cgagttcttc 2880

gtgagcccct tcagcgccga ggagggcgac ttccaccaga tccacgccga cctgaacgcc 2940

gcccagaacc tgcagcgccg cctgtggagc gacttcgaca tcagccagat ccgcctgcgc 3000

tgcgactggg gcgaggtgga cggcgagccc gtgctgatcc cccgcaccac cggcaagcgc 3060

accgccgaca gctacggcaa caaggtgttc tacaccaaga ccggcgtgac ctactacgag 3120

cgcgagcgcg gcaagaagcg ccgcaaggtg ttcgcccagg aggagctgag cgaggaggag 3180

gccgagctgc tggtggaggc cgacgaggcc cgcgagaaga gcgtggtgct gatgcgcgac 3240

cccagcggca tcatcaaccg cggcgactgg acccgccaga aggagttctg gagcatggtg 3300

aaccagcgca tcgagggcta cctggtgaag cagatccgca gccgcgtgcg cctgcaggag 3360

agcgcctgcg agaacaccgg cgacatc 3387

<210> 12
<211> 3441
<212> DNA
<213> Alicyclobacillus kakegawensis

<400> 12
atggccgtga agagcatcaa ggtgaagctg cgcctgagcg agtgccccga catcctggcc 60

ggcatgtggc agctgcaccg cgccaccaac gccggcgtgc gctactacac cgagtgggtg 120

agcctgatgc gccaggagat cctgtacagc cgcggccccg acggcggcca gcagtgctac 180

atgaccgccg aggactgcca gcgcgagctg ctgcgccgcc tgcgcaaccg ccagctgcac 240

aacggccgcc aggaccagcc cggcaccgac gccgacctgc tggccatcag ccgccgcctg 300

tacgagatcc tggtgctgca gagcatcggc aagcgcggcg acgcccagca gatcgccagc 360

agcttcctga gccccctggt ggaccccaac agcaagggcg gccgcggcga ggccaagagc 420

ggccgcaagc ccgcctggca gaagatgcgc gaccagggcg acccccgctg ggtggccgcc 480

cgcgagaagt acgagcagcg caaggccgtg gaccccagca aggagatcct gaacagcctg 540

gacgccctgg gcctgcgccc cctgttcgcc gtgttcaccg agacctaccg cagcggcgtg 600

gactggaagc ccctgggcaa gagccagggc gtgcgcacct gggaccgcga catgttccag 660

caggccctgg agcgcctgat gagctgggag agctggaacc gccgcgtggg cgaggagtac 720

gcccgcctgt tccagcagaa gatgaagttc gagcaggagc acttcgccga gcagagccac 780

ctggtgaagc tggcccgcgc cctggaggcc gacatgcgcg ccgccagcca gggcttcgag 840

gccaagcgcg gcaccgccca ccagatcacc cgccgcgccc tgcgcggcgc cgaccgcgtg 900

ttcgagatct ggaagagcat ccccgaggag gccctgttca gccagtacga cgaggtgatc 960

cgccaggtgc aggccgagaa gcgccgcgac ttcggcagcc acgacctgtt cgccaagctg 1020

gccgagccca agtaccagcc cctgtggcgc gccgacgaga ccttcctgac ccgctacgcc 1080

ctgtacaacg gcgtgctgcg cgacctggag aaggcccgcc agttcgccac cttcaccctg 1140

cccgacgcct gcgtgaaccc catctggacc cgcttcgaga gcagccaggg cagcaacctg 1200

cacaagtacg agttcctgtt cgaccacctg ggccccggcc gccacgccgt gcgcttccag 1260

cgcctgctgg tggtggagag cgagggcgcc aaggagcgcg acagcgtggt ggtgcccgtg 1320

gcccccagcg gccagctgga caagctggtg ctgcgcgagg aggagaagag cagcgtggcc 1380

ctgcacctgc acgacaccgc ccgccccgac ggcttcatgg ccgagtgggc cggcgccaag 1440

ctgcagtacg agcgcagcac cctggcccgc aaggcccgcc gcgacaagca gggcatgcgc 1500

agctggcgcc gccagcccag catgctgatg agcgccgccc agatgctgga ggacgccaag 1560

caggccggcg acgtgtacct gaacatcagc gtgcgcgtga agagccccag cgaggtgcgc 1620

ggccagcgcc gcccccccta cgccgccctg ttccgcatcg acgacaagca gcgccgcgtg 1680

accgtgaact acaacaagct gagcgcctac ctggaggagc accccgacaa gcagatcccc 1740

ggcgcccccg gcctgctgag cggcctgcgc gtgatgagcg tggacctggg cctgcgcacc 1800

agcgccagca tcagcgtgtt ccgcgtggcc aagaaggagg aggtggaggc cctgggcgac 1860

ggccgccccc cccactacta ccccatccac ggcaccgacg acctggtggc cgtgcacgag 1920

cgcagccacc tgatccagat gcccggcgag accgagacca agcagctgcg caagctgcgc 1980

gaggagcgcc aggccgtgct gcgccccctg ttcgcccagc tggccctgct gcgcctgctg 2040

gtgcgctgcg gcgccgccga cgagcgcatc cgcacccgca gctggcagcg cctgaccaag 2100

cagggccgcg agttcaccaa gcgcctgacc cccagctggc gcgaggccct ggagctggag 2160

ctgacccgcc tggaggccta ctgcggccgc gtgcccgacg acgagtggag ccgcatcgtg 2220

gaccgcaccg tgatcgccct gtggcgccgc atgggcaagc aggtgcgcga ctggcgcaag 2280

caggtgaaga gcggcgccaa ggtgaaggtg aagggctacc agctggacgt ggtgggcggc 2340

aacagcctgg cccagatcga ctacctggag cagcagtaca agttcctgcg ccgctggagc 2400

ttcttcgccc gcgccagcgg cctggtggtg cgcgccgacc gcgagagcca cttcgccgtg 2460

gccctgcgcc agcacatcga gaacgccaag cgcgaccgcc tgaagaagct ggccgaccgc 2520

atcctgatgg aggccctggg ctacgtgtac gaggccagcg gcccccgcga gggccagtgg 2580

accgcccagc accccccctg ccagctgatc atcctggagg agctgagcgc ctaccgcttc 2640

agcgacgacc gcccccccag cgagaacagc aagctgatgg cctggggcca ccgcggcatc 2700

ctggaggagc tggtgaacca ggcccaggtg cacgacgtgc tggtgggcac cgtgtacgcc 2760

gccttcagca gccgcttcga cgcccgcacc ggcgcccccg gcgtgcgctg ccgccgcgtg 2820

cccgcccgct tcgtgggcgc caccgtggac gacagcctgc ccctgtggct gaccgagttc 2880

ctggacaagc accgcctgga caagaacctg ctgcgccccg acgacgtgat ccccaccggc 2940

gagggcgagt tcctggtgag cccctgcggc gaggaggccg cccgcgtgcg ccaggtgcac 3000

gccgacatca acgccgccca gaacctgcag cgccgcctgt ggcagaactt cgacatcacc 3060

gagctgcgcc tgcgctgcga cgtgaagatg ggcggcgagg gcaccgtgct ggtgccccgc 3120

gtgaacaacg cccgcgccaa gcagctgttc ggcaagaagg tgctggtgag ccaggacggc 3180

gtgaccttct tcgagcgcag ccagaccggc ggcaagcccc acagcgagaa gcagaccgac 3240

ctgaccgaca aggagctgga gctgatcgcc gaggccgacg aggcccgcgc caagagcgtg 3300

gtgctgttcc gcgaccccag cggccacatc ggcaagggcc actggatccg ccagcgcgag 3360

ttctggagcc tggtgaagca gcgcatcgag agccacaccg ccgagcgcat ccgcgtgcgc 3420

ggcgtgggca gcagcctgga c 3441

<210> 13
<211> 3438
<212> DNA
<213> Alicyclobacillus macrosporangiidus

<400> 13
atgaacgtgg ccgtgaagag catcaaggtg aagctgatgc tgggccacct gcccgagatc 60

cgcgagggcc tgtggcacct gcacgaggcc gtgaacctgg gcgtgcgcta ctacaccgag 120

tggctggccc tgctgcgcca gggcaacctg taccgccgcg gcaaggacgg cgcccaggag 180

tgctacatga ccgccgagca gtgccgccag gagctgctgg tgcgcctgcg cgaccgccag 240

aagcgcaacg gccacaccgg cgaccccggc accgacgagg agctgctggg cgtggcccgc 300

cgcctgtacg agctgctggt gccccagagc gtgggcaaga agggccaggc ccagatgctg 360

gccagcggct tcctgagccc cctggccgac cccaagagcg agggcggcaa gggcaccagc 420

aagagcggcc gcaagcccgc ctggatgggc atgaaggagg ccggcgacag ccgctgggtg 480

gaggccaagg cccgctacga ggccaacaag gccaaggacc ccaccaagca ggtgatcgcc 540

agcctggaga tgtacggcct gcgccccctg ttcgacgtgt tcaccgagac ctacaagacc 600

atccgctgga tgcccctggg caagcaccag ggcgtgcgcg cctgggaccg cgacatgttc 660

cagcagagcc tggagcgcct gatgagctgg gagagctgga acgagcgcgt gggcgccgag 720

ttcgcccgcc tggtggaccg ccgcgaccgc ttccgcgaga agcacttcac cggccaggag 780

cacctggtgg ccctggccca gcgcctggag caggagatga aggaggccag ccccggcttc 840

gagagcaaga gcagccaggc ccaccgcatc accaagcgcg ccctgcgcgg cgccgacggc 900

atcatcgacg actggctgaa gctgagcgag ggcgagcccg tggaccgctt cgacgagatc 960

ctgcgcaagc gccaggccca gaacccccgc cgcttcggca gccacgacct gttcctgaag 1020

ctggccgagc ccgtgttcca gcccctgtgg cgcgaggacc ccagcttcct gagccgctgg 1080

gccagctaca acgaggtgct gaacaagctg gaggacgcca agcagttcgc caccttcacc 1140

ctgcccagcc cctgcagcaa ccccgtgtgg gcccgcttcg agaacgccga gggcaccaac 1200

atcttcaagt acgacttcct gttcgaccac ttcggcaagg gccgccacgg cgtgcgcttc 1260

cagcgcatga tcgtgatgcg cgacggcgtg cccaccgagg tggagggcat cgtggtgccc 1320

atcgccccca gccgccagct ggacgccctg gcccccaacg acgccgccag ccccatcgac 1380

gtgttcgtgg gcgaccccgc cgcccccggc gccttccgcg gccagttcgg cggcgccaag 1440

atccagtacc gccgcagcgc cctggtgcgc aagggccgcc gcgaggagaa ggcctacctg 1500

tgcggcttcc gcctgcccag ccagcgccgc accggcaccc ccgccgacga cgccggcgag 1560

gtgttcctga acctgagcct gcgcgtggag agccagagcg agcaggccgg ccgccgcaac 1620

cccccctacg ccgccgtgtt ccacatcagc gaccagaccc gccgcgtgat cgtgcgctac 1680

ggcgagatcg agcgctacct ggccgagcac cccgacaccg gcatccccgg cagccgcggc 1740

ctgaccagcg gcctgcgcgt gatgagcgtg gacctgggcc tgcgcaccag cgccgccatc 1800

agcgtgttcc gcgtggccca ccgcgacgag ctgacccccg acgcccacgg ccgccagccc 1860

ttcttcttcc ccatccacgg catggaccac ctggtggccc tgcacgagcg cagccacctg 1920

atccgcctgc ccggcgagac cgagagcaag aaggtgcgca gcatccgcga gcagcgcctg 1980

gaccgcctga accgcctgcg cagccagatg gccagcctgc gcctgctggt gcgcaccggc 2040

gtgctggacg agcagaagcg cgaccgcaac tgggagcgcc tgcagagcag catggagcgc 2100

ggcggcgagc gcatgcccag cgactggtgg gacctgttcc aggcccaggt gcgctacctg 2160

gcccagcacc gcgacgccag cggcgaggcc tggggccgca tggtgcaggc cgccgtgcgc 2220

accctgtggc gccagctggc caagcaggtg cgcgactggc gcaaggaggt gcgccgcaac 2280

gccgacaagg tgaagatccg cggcatcgcc cgcgacgtgc ccggcggcca cagcctggcc 2340

cagctggact acctggagcg ccagtaccgc ttcctgcgca gctggagcgc cttcagcgtg 2400

caggccggcc aggtggtgcg cgccgagcgc gacagccgct tcgccgtggc cctgcgcgag 2460

cacatcgaca acggcaagaa ggaccgcctg aagaagctgg ccgaccgcat cctgatggag 2520

gccctgggct acgtgtacgt gaccgacggc cgccgcgccg gccagtggca ggccgtgtac 2580

cccccctgcc agctggtgct gctggaggag ctgagcgagt accgcttcag caacgaccgc 2640

ccccccagcg agaacagcca gctgatggtg tggagccacc gcggcgtgct ggaggagctg 2700

atccaccagg cccaggtgca cgacgtgctg gtgggcacca tccccgccgc cttcagcagc 2760

cgcttcgacg cccgcaccgg cgcccccggc atccgctgcc gccgcgtgcc cagcatcccc 2820

ctgaaggacg cccccagcat ccccatctgg ctgagccact acctgaagca gaccgagcgc 2880

gacgccgccg ccctgcgccc cggcgagctg atccccaccg gcgacggcga gttcctggtg 2940

acccccgccg gccgcggcgc cagcggcgtg cgcgtggtgc acgccgacat caacgccgcc 3000

cacaacctgc agcgccgcct gtgggagaac ttcgacctga gcgacatccg cgtgcgctgc 3060

gaccgccgcg agggcaagga cggcaccgtg gtgctgatcc cccgcctgac caaccagcgc 3120

gtgaaggagc gctacagcgg cgtgatcttc accagcgagg acggcgtgag cttcaccgtg 3180

ggcgacgcca agacccgccg ccgcagcagc gccagccagg gcgagggcga cgacctgagc 3240

gacgaggagc aggagctgct ggccgaggcc gacgacgccc gcgagcgcag cgtggtgctg 3300

ttccgcgacc ccagcggctt cgtgaacggc ggccgctgga ccgcccagcg cgccttctgg 3360

ggcatggtgc acaaccgcat cgagaccctg ctggccgagc gcttcagcgt gagcggcgcc 3420

gccgagaagg tgcgcggc 3438

<210> 14
<211> 3324
<212> DNA
<213> Bacillus hisashii

<400> 14
atggccaccc gcagcttcat cctgaagatc gagcccaacg aggaggtgaa gaagggcctg 60

tggaagaccc acgaggtgct gaaccacggc atcgcctact acatgaacat cctgaagctg 120

atccgccagg aggccatcta cgagcaccac gagcaggacc ccaagaaccc caagaaggtg 180

agcaaggccg agatccaggc cgagctgtgg gacttcgtgc tgaagatgca gaagtgcaac 240

agcttcaccc acgaggtgga caaggacgag gtgttcaaca tcctgcgcga gctgtacgag 300

gagctggtgc ccagcagcgt ggagaagaag ggcgaggcca accagctgag caacaagttc 360

ctgtaccccc tggtggaccc caacagccag agcggcaagg gcaccgccag cagcggccgc 420

aagccccgct ggtacaacct gaagatcgcc ggcgacccca gctgggagga ggagaagaag 480

aagtgggagg aggacaagaa gaaggacccc ctggccaaga tcctgggcaa gctggccgag 540

tacggcctga tccccctgtt catcccctac accgacagca acgagcccat cgtgaaggag 600

atcaagtgga tggagaagag ccgcaaccag agcgtgcgcc gcctggacaa ggacatgttc 660

atccaggccc tggagcgctt cctgagctgg gagagctgga acctgaaggt gaaggaggag 720

tacgagaagg tggagaagga gtacaagacc ctggaggagc gcatcaagga ggacatccag 780

gccctgaagg ccctggagca gtacgagaag gagcgccagg agcagctgct gcgcgacacc 840

ctgaacacca acgagtaccg cctgagcaag cgcggcctgc gcggctggcg cgagatcatc 900

cagaagtggc tgaagatgga cgagaacgag cccagcgaga agtacctgga ggtgttcaag 960

gactaccagc gcaagcaccc ccgcgaggcc ggcgactaca gcgtgtacga gttcctgagc 1020

aagaaggaga accacttcat ctggcgcaac caccccgagt acccctacct gtacgccacc 1080

ttctgcgaga tcgacaagaa gaagaaggac gccaagcagc aggccacctt caccctggcc 1140

gaccccatca accaccccct gtgggtgcgc ttcgaggagc gcagcggcag caacctgaac 1200

aagtaccgca tcctgaccga gcagctgcac accgagaagc tgaagaagaa gctgaccgtg 1260

cagctggacc gcctgatcta ccccaccgag agcggcggct gggaggagaa gggcaaggtg 1320

gacatcgtgc tgctgcccag ccgccagttc tacaaccaga tcttcctgga catcgaggag 1380

aagggcaagc acgccttcac ctacaaggac gagagcatca agttccccct gaagggcacc 1440

ctgggcggcg cccgcgtgca gttcgaccgc gaccacctgc gccgctaccc ccacaaggtg 1500

gagagcggca acgtgggccg catctacttc aacatgaccg tgaacatcga gcccaccgag 1560

agccccgtga gcaagagcct gaagatccac cgcgacgact tccccaaggt ggtgaacttc 1620

aagcccaagg agctgaccga gtggatcaag gacagcaagg gcaagaagct gaagagcggc 1680

atcgagagcc tggagatcgg cctgcgcgtg atgagcatcg acctgggcca gcgccaggcc 1740

gccgccgcca gcatcttcga ggtggtggac cagaagcccg acatcgaggg caagctgttc 1800

ttccccatca agggcaccga gctgtacgcc gtgcaccgcg ccagcttcaa catcaagctg 1860

cccggcgaga ccctggtgaa gagccgcgag gtgctgcgca aggcccgcga ggacaacctg 1920

aagctgatga accagaagct gaacttcctg cgcaacgtgc tgcacttcca gcagttcgag 1980

gacatcaccg agcgcgagaa gcgcgtgacc aagtggatca gccgccagga gaacagcgac 2040

gtgcccctgg tgtaccagga cgagctgatc cagatccgcg agctgatgta caagccctac 2100

aaggactggg tggccttcct gaagcagctg cacaagcgcc tggaggtgga gatcggcaag 2160

gaggtgaagc actggcgcaa gagcctgagc gacggccgca agggcctgta cggcatcagc 2220

ctgaagaaca tcgacgagat cgaccgcacc cgcaagttcc tgctgcgctg gagcctgcgc 2280

cccaccgagc ccggcgaggt gcgccgcctg gagcccggcc agcgcttcgc catcgaccag 2340

ctgaaccacc tgaacgccct gaaggaggac cgcctgaaga agatggccaa caccatcatc 2400

atgcacgccc tgggctactg ctacgacgtg cgcaagaaga agtggcaggc caagaacccc 2460

gcctgccaga tcatcctgtt cgaggacctg agcaactaca acccctacga ggagcgcagc 2520

cgcttcgaga acagcaagct gatgaagtgg agccgccgcg agatcccccg ccaggtggcc 2580

ctgcagggcg agatctacgg cctgcaggtg ggcgaggtgg gcgcccagtt cagcagccgc 2640

ttccacgcca agaccggcag ccccggcatc cgctgcagcg tggtgaccaa ggagaagctg 2700

caggacaacc gcttcttcaa gaacctgcag cgcgagggcc gcctgaccct ggacaagatc 2760

gccgtgctga aggagggcga cctgtacccc gacaagggcg gcgagaagtt catcagcctg 2820

agcaaggacc gcaagtgcgt gaccacccac gccgacatca acgccgccca gaacctgcag 2880

aagcgcttct ggacccgcac ccacggcttc tacaaggtgt actgcaaggc ctaccaggtg 2940

gacggccaga ccgtgtacat ccccgagagc aaggaccaga agcagaagat catcgaggag 3000

ttcggcgagg gctacttcat cctgaaggac ggcgtgtacg agtgggtgaa cgccggcaag 3060

ctgaagatca agaagggcag cagcaagcag agcagcagcg agctggtgga cagcgacatc 3120

ctgaaggaca gcttcgacct ggccagcgag ctgaagggcg agaagctgat gctgtaccgc 3180

gaccccagcg gcaacgtgtt ccccagcgac aagtggatgg ccgccggcgt gttcttcggc 3240

aagctggagc gcatcctgat cagcaagctg accaaccagt acagcatcag caccatcgag 3300

gacgacagca gcaagcagag catg 3324

<210> 15
<211> 3324
<212> DNA
<213> Bacillus

<400> 15
   atggccatcc gcagcatcaa gctgaagctg aagacccaca ccggccccga ggcccagaac 60
   ctgcgcaagg gcatctggcg cacccaccgc ctgctgaacg agggcgtggc ctactacatg 120
   aagatgctgc tgctgttccg ccaggagagc accggcgagc gccccaagga ggagctgcag 180
   gaggagctga tctgccacat ccgcgagcag cagcagcgca accaggccga caagaacacc 240
   caggccctgc ccctggacaa ggccctggag gccctgcgcc agctgtacga gctgctggtg 300
   cccagcagcg tgggccagag cggcgacgcc cagatcatca gccgcaagtt cctgagcccc 360
   ctggtggacc ccaacagcga gggcggcaag ggcaccagca aggccggcgc caagcccacc 420
   tggcagaaga agaaggaggc caacgacccc acctgggagc aggactacga gaagtggaag 480
   aagcgccgcg aggaggaccc caccgccagc gtgatcacca ccctggagga gtacggcatc 540
   cgccccatct tccccctgta caccaacacc gtgaccgaca tcgcctggct gcccctgcag 600
   agcaaccagt tcgtgcgcac ctgggaccgc gacatgctgc agcaggccat cgagcgcctg 660
   ctgagctggg agagctggaa caagcgcgtg caggaggagt acgccaagct gaaggagaag 720
   atggcccagc tgaacgagca gctggagggc ggccaggagt ggatcagcct gctggagcag 780
   tacgaggaga accgcgagcg cgagctgcgc gagaacatga ccgccgccaa cgacaagtac 840
   cgcatcacca agcgccagat gaagggctgg aacgagctgt acgagctgtg gagcaccttc 900
   cccgccagcg ccagccacga gcagtacaag gaggccctga agcgcgtgca gcagcgcctg 960
   cgcggccgct tcggcgacgc ccacttcttc cagtacctga tggaggagaa gaaccgcctg 1020
   atctggaagg gcaaccccca gcgcatccac tacttcgtgg cccgcaacga gctgaccaag 1080
   cgcctggagg aggccaagca gagcgccacc atgaccctgc ccaacgcccg caagcacccc 1140
   ctgtgggtgc gcttcgacgc ccgcggcggc aacctgcagg actactacct gaccgccgag 1200
   gccgacaagc cccgcagccg ccgcttcgtg accttcagcc agctgatctg gcccagcgag 1260
   agcggctgga tggagaagaa ggacgtggag gtggagctgg ccctgagccg ccagttctac 1320
   cagcaggtga agctgctgaa gaacgacaag ggcaagcaga agatcgagtt caaggacaag 1380
   ggcagcggca gcaccttcaa cggccacctg ggcggcgcca agctgcagct ggagcgcggc 1440
   gacctggaga aggaggagaa gaacttcgag gacggcgaga tcggcagcgt gtacctgaac 1500
   gtggtgatcg acttcgagcc cctgcaggag gtgaagaacg gccgcgtgca ggccccctac 1560
   ggccaggtgc tgcagctgat ccgccgcccc aacgagttcc ccaaggtgac cacctacaag 1620
   agcgagcagc tggtggagtg gatcaaggcc agcccccagc acagcgccgg cgtggagagc 1680
   ctggccagcg gcttccgcgt gatgagcatc gacctgggcc tgcgcgccgc cgccgccacc 1740
   agcatcttca gcgtggagga gagcagcgac aagaacgccg ccgacttcag ctactggatc 1800
   gagggcaccc ccctggtggc cgtgcaccag cgcagctaca tgctgcgcct gcccggcgag 1860
   caggtggaga agcaggtgat ggagaagcgc gacgagcgct tccagctgca ccagcgcgtg 1920
   aagttccaga tccgcgtgct ggcccagatc atgcgcatgg ccaacaagca gtacggcgac 1980
   cgctgggacg agctggacag cctgaagcag gccgtggagc agaagaagag ccccctggac 2040
   cagaccgacc gcaccttctg ggagggcatc gtgtgcgacc tgaccaaggt gctgccccgc 2100
   aacgaggccg actgggagca ggccgtggtg cagatccacc gcaaggccga ggagtacgtg 2160
   ggcaaggccg tgcaggcctg gcgcaagcgc ttcgccgccg acgagcgcaa gggcatcgcc 2220
   ggcctgagca tgtggaacat cgaggagctg gagggcctgc gcaagctgct gatcagctgg 2280
   agccgccgca cccgcaaccc ccaggaggtg aaccgcttcg agcgcggcca caccagccac 2340
   cagcgcctgc tgacccacat ccagaacgtg aaggaggacc gcctgaagca gctgagccac 2400
   gccatcgtga tgaccgccct gggctacgtg tacgacgagc gcaagcagga gtggtgcgcc 2460
   gagtaccccg cctgccaggt gatcctgttc gagaacctga gccagtaccg cagcaacctg 2520
   gaccgcagca ccaaggagaa cagcaccctg atgaagtggg cccaccgcag catccccaag 2580
   tacgtgcaca tgcaggccga gccctacggc atccagatcg gcgacgtgcg cgccgagtac 2640
   agcagccgct tctacgccaa gaccggcacc cccggcatcc gctgcaagaa ggtgcgcggc 2700
   caggacctgc agggccgccg cttcgagaac ctgcagaagc gcctggtgaa cgagcagttc 2760
   ctgaccgagg agcaggtgaa gcagctgcgc cccggcgaca tcgtgcccga cgacagcggc 2820
   gagctgttca tgaccctgac cgacggcagc ggcagcaagg aggtggtgtt cctgcaggcc 2880
   gacatcaacg ccgcccacaa cctgcagaag cgcttctggc agcgctacaa cgagctgttc 2940
   aaggtgagct gccgcgtgat cgtgcgcgac gaggaggagt acctggtgcc caagaccaag 3000
   agcgtgcagg ccaagctggg caagggcctg ttcgtgaaga agagcgacac cgcctggaag 3060
   gacgtgtacg tgtgggacag ccaggccaag ctgaagggca agaccacctt caccgaggag 3120
   agcgagagcc ccgagcagct ggaggacttc caggagatca tcgaggaggc cgaggaggcc 3180
   aagggcacct accgcaccct gttccgcgac cccagcggcg tgttcttccc cgagagcgtg 3240
   tggtaccccc agaaggactt ctggggcgag gtgaagcgca agctgtacgg caagctgcgc 3300
   gagcgcttcc tgaccaaggc ccgc 3324

<210> 16
<211> 3336
<212> DNA
<213> Bacillus

<400> 16
   atggccatcc gcagcatcaa gctgaagatg aagaccaaca gcggcaccga cagcatctac 60
   ctgcgcaagg ccctgtggcg cacccaccag ctgatcaacg agggcatcgc ctactacatg 120
   aacctgctga ccctgtaccg ccaggaggcc atcggcgaca agaccaagga ggcctaccag 180
   gccgagctga tcaacatcat ccgcaaccag cagcgcaaca acggcagcag cgaggagcac 240
   ggcagcgacc aggagatcct ggccctgctg cgccagctgt acgagctgat catccccagc 300
   agcatcggcg agagcggcga cgccaaccag ctgggcaaca agttcctgta ccccctggtg 360
   gaccccaaca gccagagcgg caagggcacc agcaacgccg gccgcaagcc ccgctggaag 420
   cgcctgaagg aggagggcaa ccccgactgg gagctggaga agaagaagga cgaggagcgc 480
   aaggccaagg accccaccgt gaagatcttc gacaacctga acaagtacgg cctgctgccc 540
   ctgttccccc tgttcaccaa catccagaag gacatcgagt ggctgcccct gggcaagcgc 600
   cagagcgtgc gcaagtggga caaggacatg ttcatccagg ccatcgagcg cctgctgagc 660
   tgggagagct ggaaccgccg cgtggccgac gagtacaagc agctgaagga gaagaccgag 720
   agctactaca aggagcacct gaccggcggc gaggagtgga tcgagaagat ccgcaagttc 780
   gagaaggagc gcaacatgga gctggagaag aacgccttcg cccccaacga cggctacttc 840
   atcaccagcc gccagatccg cggctgggac cgcgtgtacg agaagtggag caagctgccc 900
   gagagcgcca gccccgagga gctgtggaag gtggtggccg agcagcagaa caagatgagc 960
   gagggcttcg gcgaccccaa ggtgttcagc ttcctggcca accgcgagaa ccgcgacatc 1020
   tggcgcggcc acagcgagcg catctaccac atcgccgcct acaacggcct gcagaagaag 1080
   ctgagccgca ccaaggagca ggccaccttc accctgcccg acgccatcga gcaccccctg 1140
   tggatccgct acgagagccc cggcggcacc aacctgaacc tgttcaagct ggaggagaag 1200
   cagaagaaga actactacgt gaccctgagc aagatcatct ggcccagcga ggagaagtgg 1260
   atcgagaagg agaacatcga gatccccctg gcccccagca tccagttcaa ccgccagatc 1320
   aagctgaagc agcacgtgaa gggcaagcag gagatcagct tcagcgacta cagcagccgc 1380
   atcagcctgg acggcgtgct gggcggcagc cgcatccagt tcaaccgcaa gtacatcaag 1440
   aaccacaagg agctgctggg cgagggcgac atcggccccg tgttcttcaa cctggtggtg 1500
   gacgtggccc ccctgcagga gacccgcaac ggccgcctgc agagccccat cggcaaggcc 1560
   ctgaaggtga tcagcagcga cttcagcaag gtgatcgact acaagcccaa ggagctgatg 1620
   gactggatga acaccggcag cgccagcaac agcttcggcg tggccagcct gctggagggc 1680
   atgcgcgtga tgagcatcga catggccagc cgcaccagcg ccagcgtgag catcttcgag 1740
   gtggtgaagg agctgcccaa ggaccaggag cagaagctgt tctacagcat caacgacacc 1800
   gagctgttcg ccatccacaa gcgcagcttc ctgctgaacc tgcccggcga ggtggtgacc 1860
   aagaacaaca agcagcagcg ccaggagcgc cgcaagaagc gccagttcgt gcgcagccag 1920
   atccgcatgc tggccaacgt gctgcgcctg gagaccaaga agacccccga cgagcgcaag 1980
   aaggccatcc acaagctgat ggagatcgtg cagagctacg acagctggac cgccagccag 2040
   aaggaggtgt gggagaagga gctgaacctg ctgaccaaca tggccgcctt caacgacgag 2100
   atctggaagg agagcctggt ggagctgcac caccgcatcg agccctacgt gggccagatc 2160
   gtgagcaagt ggcgcaaggg cctgagcgag ggccgcaaga acctggccgg catcagcatg 2220
   tggaacatcg acgagctgga ggacacccgc cgcctgctga tcagctggag caagcgcagc 2280
   cgcacccccg gcgaggccaa ccgcatcgag accgacgagc ccttcggcag cagcctgctg 2340
   cagcacatcc agaacgtgaa ggacgaccgc ctgaagcaga tggccaacct gatcatcatg 2400
   accgccctgg gcttcaagta cgacaaggag gagaaggacc gctacaagcg ctggaaggag 2460
   acctaccccg cctgccagat catcctgttc gagaacctga accgctacct gttcaacctg 2520
   gaccgcagcc gccgcgagaa cagccgcctg atgaagtggg cccaccgcag catcccccgc 2580
   accgtgagca tgcagggcga gatgttcggc ctgcaggtgg gcgacgtgcg cagcgagtac 2640
   agcagccgct tccacgccaa gaccggcgcc cccggcatcc gctgccacgc cctgaccgag 2700
   gaggacctga aggccggcag caacaccctg aagcgcctga tcgaggacgg cttcatcaac 2760
   gagagcgagc tggcctacct gaagaagggc gacatcatcc ccagccaggg cggcgagctg 2820
   ttcgtgaccc tgagcaagcg ctacaagaag gacagcgaca acaacgagct gaccgtgatc 2880
   cacgccgaca tcaacgccgc ccagaacctg cagaagcgct tctggcagca gaacagcgag 2940
   gtgtaccgcg tgccctgcca gctggcccgc atgggcgagg acaagctgta catccccaag 3000
   agccagaccg agaccatcaa gaagtacttc ggcaaggcca gcttcgtgaa gaacaacacc 3060
   gagcaggagg tgtacaagtg ggagaagagc gagaagatga agatcaagac cgacaccacc 3120
   ttcgacctgc aggacctgga cggcttcgag gacatcagca agaccatcga gctggcccag 3180
   gagcagcaga agaagtacct gaccatgttc cgcgacccca gcggctactt cttcaacaac 3240
   gagacctggc gcccccagaa ggagtactgg agcatcgtga acaacatcat caagagctgc 3300
   ctgaagaaga agatcctgag caacaaggtg gagctg                                3336

<210>  17
<211>  3447
<212>  DNA
<213>  Desulfovibrio inopinatus

<400>  17
   atgcccaccc gcaccatcaa cctgaagctg gtgctgggca agaaccccga gaacgccacc      60
   ctgcgccgcg ccctgttcag cacccaccgc ctggtgaacc aggccaccaa gcgcatcgag     120
   gagttcctgc tgctgtgccg cggcgaggcc taccgcaccg tggacaacga gggcaaggag     180
   gccgagatcc cccgccacgc cgtgcaggag gaggccctgg ccttcgccaa ggccgcccag     240
   cgccacaacg gctgcatcag cacctacgag gaccaggaga tcctggacgt gctgcgccag     300
   ctgtacgagc gcctggtgcc cagcgtgaac gagaacaacg aggccggcga cgcccaggcc     360
   gccaacgcct gggtgagccc cctgatgagc gccgagagcg agggcggcct gagcgtgtac     420
   gacaaggtgc tggacccccc ccccgtgtgg atgaagctga aggaggagaa ggcccccggc     480
   tgggaggccg ccagccagat ctggatccag agcgacgagg gccagagcct gctgaacaag     540
   cccggcagcc ccccccgctg gatccgcaag ctgcgcagcg gccagccctg gcaggacgac     600
   ttcgtgagcg accagaagaa gaagcaggac gagctgacca agggcaacgc ccccctgatc     660
   aagcagctga aggagatggg cctgctgccc ctggtgaacc ccttcttccg ccacctgctg     720
   gaccccgagg gcaagggcgt gagcccctgg gaccgcctgg ccgtgcgcgc cgccgtggcc     780
   cacttcatca gctgggagag ctggaaccac cgcacccgcg ccgagtacaa cagcctgaag     840
   ctgcgccgcg acgagttcga ggccgccagc gacgagttca aggacgactt caccctgctg     900
   cgccagtacg aggccaagcg ccacagcacc ctgaagagca tcgccctggc cgacgacagc     960
   aacccctacc gcatcggcgt gcgcagcctg cgcgcctgga accgcgtgcg cgaggagtgg    1020
   atcgacaagg gcgccaccga ggagcagcgc gtgaccatcc tgagcaagct gcagacccag    1080
   ctgcgcggca agttcggcga ccccgacctg ttcaactggc tggcccagga ccgccacgtg    1140
   cacctgtgga gcccccgcga cagcgtgacc cccctggtgc gcatcaacgc cgtggacaag    1200
   gtgctgcgcc gccgcaagcc ctacgccctg atgaccttcg cccacccccg cttccacccc    1260
   cgctggatcc tgtacgaggc ccccggcggc agcaacctgc gccagtacgc cctggactgc    1320
   accgagaacg ccctgcacat caccctgccc ctgctggtgg acgacgccca cggcacctgg    1380
   atcgagaaga agatccgcgt gcccctggcc cccagcggcc agatccagga cctgaccctg    1440
   gagaagctgg agaagaagaa gaaccgcctg tactaccgca gcggcttcca gcagttcgcc    1500
   ggcctggccg gcggcgccga ggtgctgttc caccgcccct acatggagca cgacgagcgc    1560
   agcgaggaga gcctgctgga gcgccccggc gccgtgtggt tcaagctgac cctggacgtg    1620
   gccacccagg ccccccccaa ctggctggac ggcaagggcc gcgtgcgcac cccccccgag 1680
   gtgcaccact tcaagaccgc cctgagcaac aagagcaagc acacccgcac cctgcagccc 1740
   ggcctgcgcg tgctgagcgt ggacctgggc atgcgcacct tcgccagctg cagcgtgttc 1800
   gagctgatcg agggcaagcc cgagaccggc cgcgccttcc ccgtggccga cgagcgcagc 1860
   atggacagcc ccaacaagct gtgggccaag cacgagcgca gcttcaagct gaccctgccc 1920
   ggcgagaccc ccagccgcaa ggaggaggag gagcgcagca tcgcccgcgc cgagatctac 1980
   gccctgaagc gcgacatcca gcgcctgaag agcctgctgc gcctgggcga ggaggacaac 2040
   gacaaccgcc gcgacgccct gctggagcag ttcttcaagg gctggggcga ggaggacgtg 2100
   gtgcccggcc aggccttccc ccgcagcctg ttccagggcc tgggcgccgc ccccttccgc 2160
   agcacccccg agctgtggcg ccagcactgc cagacctact acgacaaggc cgaggcctgc 2220
   ctggccaagc acatcagcga ctggcgcaag cgcacccgcc cccgccccac cagccgcgag 2280
   atgtggtaca agacccgcag ctaccacggc ggcaagagca tctggatgct ggagtacctg 2340
   gacgccgtgc gcaagctgct gctgagctgg agcctgcgcg gccgcaccta cggcgccatc 2400
   aaccgccagg acaccgcccg cttcggcagc ctggccagcc gcctgctgca ccacatcaac 2460
   agcctgaagg aggaccgcat caagaccggc gccgacagca tcgtgcaggc cgcccgcggc 2520
   tacatccccc tgccccacgg caagggctgg gagcagcgct acgagccctg ccagctgatc 2580
   ctgttcgagg acctggcccg ctaccgcttc cgcgtggacc gcccccgccg cgagaacagc 2640
   cagctgatgc agtggaacca ccgcgccatc gtggccgaga ccaccatgca ggccgagctg 2700
   tacggccaga tcgtggagaa caccgccgcc ggcttcagca gccgcttcca cgccgccacc 2760
   ggcgcccccg gcgtgcgctg ccgcttcctg ctggagcgcg acttcgacaa cgacctgccc 2820
   aagccctacc tgctgcgcga gctgagctgg atgctgggca acaccaaggt ggagagcgag 2880
   gaggagaagc tgcgcctgct gagcgagaag atccgccccg gcagcctggt gccctgggac 2940
   ggcggcgagc agttcgccac cctgcacccc aagcgccaga ccctgtgcgt gatccacgcc 3000
   gacatgaacg ccgcccagaa cctgcagcgc cgcttcttcg gccgctgcgg cgaggccttc 3060
   cgcctggtgt gccagcccca cggcgacgac gtgctgcgcc tggccagcac ccccggcgcc 3120
   cgcctgctgg gcgccctgca gcagctggag aacggccagg gcgccttcga gctggtgcgc 3180
   gacatgggca gcaccagcca gatgaaccgc ttcgtgatga agagcctggg caagaagaag 3240
   atcaagcccc tgcaggacaa caacggcgac gacgagctgg aggacgtgct gagcgtgctg 3300
   cccgaggagg acgacaccgg ccgcatcacc gtgttccgcg acagcagcgg catcttcttc 3360
   ccctgcaacg tgtggatccc cgccaagcag ttctggcccg ccgtgcgcgc catgatctgg 3420
   aaggtgatgg ccagccacag cctgggc 3447

<210>  18
<211>  3270
<212>  DNA
<213>  Laceyella sediminis

<400>  18
atgagcatcc gcagcttcaa gctgaagatc aagaccaaga gcggcgtgaa cgccgaggag      60

ctgcgccgcg gcctgtggcg cacccaccag ctgatcaacg acggcatcgc ctactacatg      120

aactggctgg tgctgctgcg ccaggaggac ctgttcatcc gcaacgagga gaccaacgag      180

atcgagaagc gcagcaagga ggagatccag ggcgagctgc tggagcgcgt gcacaagcag      240

cagcagcgca accagtggag cggcgaggtg gacgaccaga ccctgctgca gaccctgcgc      300

cacctgtacg aggagatcgt gcccagcgtg atcggcaaga gcggcaacgc cagcctgaag      360

gcccgcttct tcctgggccc cctggtggac cccaacaaca agaccaccaa ggacgtgagc      420

aagagcggcc ccacccccaa gtggaagaag atgaaggacg ccggcgaccc caactgggtg      480

caggagtacg agaagtacat ggccgagcgc cagaccctgg tgcgcctgga ggagatgggc      540

ctgatccccc tgttccccat gtacaccgac gaggtgggcg acatccactg gctgccccag      600

gccagcggct acacccgcac ctgggaccgc gacatgttcc agcaggccat cgagcgcctg      660

ctgagctggg agagctggaa ccgccgcgtg cgcgagcgcc gcgcccagtt cgagaagaag      720

acccacgact tcgccagccg cttcagcgag agcgacgtgc agtggatgaa caagctgcgc      780

gagtacgagg cccagcagga gaagagcctg gaggagaacg ccttcgcccc caacgagccc      840

tacgccctga ccaagaaggc cctgcgcggc tgggagcgcg tgtaccacag ctggatgcgc      900

ctggacagcg ccgccagcga ggaggcctac tggcaggagg tggccacctg ccagaccgcc      960

atgcgcggcg agttcggcga ccccgccatc taccagttcc tggcccagaa ggagaaccac      1020

gacatctggc gcggctaccc cgagcgcgtg atcgacttcg ccgagctgaa ccacctgcag      1080

cgcgagctgc gccgcgccaa ggaggacgcc accttcaccc tgcccgacag cgtggaccac      1140

cccctgtggg tgcgctacga ggcccccggc ggcaccaaca tccacggcta cgacctggtg      1200

caggacacca agcgcaacct gaccctgatc ctggacaagt tcatcctgcc cgacgagaac      1260

ggcagctggc acgaggtgaa gaaggtgccc ttcagcctgg ccaagagcaa gcagttccac      1320

cgccaggtgt ggctgcagga ggagcagaag cagaagaagc gcgaggtggt gttctacgac      1380

tacagcacca acctgcccca cctgggcacc ctggccggcg ccaagctgca gtgggaccgc      1440

aacttcctga acaagcgcac ccagcagcag atcgaggaga ccggcgagat cggcaaggtg      1500

ttcttcaaca tcagcgtgga cgtgcgcccc gccgtggagg tgaagaacgg ccgcctgcag      1560

aacggcctgg gcaaggccct gaccgtgctg acccaccccg acggcaccaa gatcgtgacc      1620

ggctggaagg ccgagcagct ggagaagtgg gtgggcgaga gcggccgcgt gagcagcctg      1680

ggcctggaca gcctgagcga gggcctgcgc gtgatgagca tcgacctggg ccagcgcacc 1740

agcgccaccg tgagcgtgtt cgagatcacc aaggaggccc ccgacaaccc ctacaagttc 1800

ttctaccagc tggagggcac cgagctgttc gccgtgcacc agcgcagctt cctgctggcc 1860

ctgcccggcg agaacccccc ccagaagatc aagcagatgc gcgagatccg ctggaaggag 1920

cgcaaccgca tcaagcagca ggtggaccag ctgagcgcca tcctgcgcct gcacaagaag 1980

gtgaacgagg acgagcgcat ccaggccatc gacaagctgc tgcagaaggt ggccagctgg 2040

cagctgaacg aggagatcgc caccgcctgg aaccaggccc tgagccagct gtacagcaag 2100

gccaaggaga acgacctgca gtggaaccag gccatcaaga acgcccacca ccagctggag 2160

cccgtggtgg gcaagcagat cagcctgtgg cgcaaggacc tgagcaccgg ccgccagggc 2220

atcgccggcc tgagcctgtg gagcatcgag gagctggagg ccaccaagaa gctgctgacc 2280

cgctggagca agcgcagccg cgagcccggc gtggtgaagc gcatcgagcg cttcgagacc 2340

ttcgccaagc agatccagca ccacatcaac caggtgaagg agaaccgcct gaagcagctg 2400

gccaacctga tcgtgatgac cgccctgggc tacaagtacg accaggagca gaagaagtgg 2460

atcgaggtgt accccgcctg ccaggtggtg ctgttcgaga acctgcgcag ctaccgcttc 2520

agctacgagc gcagccgccg cgagaacaag aagctgatgg agtggagcca ccgcagcatc 2580

cccaagctgg tgcagatgca gggcgagctg ttcggcctgc aggtggccga cgtgtacgcc 2640

gcctacagca gccgctacca cggccgcacc ggcgcccccg gcatccgctg ccacgccctg 2700

accgaggccg acctgcgcaa cgagaccaac atcatccacg agctgatcga ggccggcttc 2760

atcaaggagg agcaccgccc ctacctgcag cagggcgacc tggtgccctg gagcggcggc 2820

gagctgttcg ccaccctgca gaagccctac gacaaccccc gcatcctgac cctgcacgcc 2880

gacatcaacg ccgcccagaa catccagaag cgcttctggc accccagcat gtggttccgc 2940

gtgaactgcg agagcgtgat ggagggcgag atcgtgacct acgtgcccaa gaacaagacc 3000

gtgcacaaga agcagggcaa gaccttccgc ttcgtgaagg tggagggcag cgacgtgtac 3060

gagtgggcca agtggagcaa gaaccgcaac aagaacacct tcagcagcat caccgagcgc 3120

aagcccccca gcagcatgat cctgttccgc gaccccagcg gcaccttctt caaggagcag 3180

gagtgggtgg agcagaagac cttctggggc aaggtgcaga gcatgatcca ggcctacatg 3240

aagaagacca tcgtgcagcg catggaggag 3270

<210> 19
<211> 3357
<212> DNA
<213> Spirochaetes

<400> 19
atgagcttca ccatcagcta ccccttcaag ctgatcatca agaacaagga cgaggccaag 60

gccctgctgg acacccacca gtacatgaac gagggcgtga agtactacct ggagaagctg 120
ctgatgttcc gccaggagaa gatcttcatc ggcgaggacg agaccggcaa gcgcatctac 180
atcgaggaga ccgagtacaa gaagcagatc gaggagttct acctgatcaa gaagaccgag 240
ctgggccgca acctgaccct gaccctggac gagttcaaga ccctgatgcg cgagctgtac 300
atctgcctgg tgagcagcag catggagaac aagaagggct tccccaacgc ccagcaggcc 360
agcctgaaca tcttcagccc cctgttcgac gccgagagca agggctacat cctgaaggag 420
gagaacaaca acatcagcct gatccacaag gactacggca agatcctgct gaagcgcctg 480
cgcgacaaca acctgatccc catcttcacc aagttcaccg acatcaagaa gatcaccgcc 540
aagctgagcc ccaccgccct ggaccgcatg atcttcgccc aggccatcga gaagctgctg 600
agctacgaga gctggtgcaa gctgatgatc aaggagcgct tcgacaagga ggtgaagatc 660
aaggagctgg agaacaagtg cgagaacaag caggagcgcg acaagatctt cgagatcctg 720
gagaagtacg aggaggagcg ccagaagacc ttcgagcagg acagcggctt cgccaagaag 780
ggcaagttct acatcaccgg ccgcatgctg aagggcttcg acgagatcaa ggagaagtgg 840
ctgaaggaga aggaccgcag cgagcagaac ctgatcaaca tcctgaacaa gtaccagacc 900
gacaacagca agctggtggg cgaccgcaac ctgttcgagt tcatcatcaa gctggagaac 960
cagtgcctgt ggaacggcga catcgactac ctgaagatca agcgcgacat caacaagaac 1020
cagatctggc tggaccgccc cgagatgccc cgcttcacca tgcccgactt caagaagcac 1080
cccctgtggt accgctacga ggaccccagc aacagcaact tccgcaacta caagatcgag 1140
gtggtgaagg acgagaacta catcaccatc cccctgatca ccgagcgcaa caacgagtac 1200
ttcgaggaga actacacctt caacctggcc aagctgaaga agctgagcga gaacatcacc 1260
ttcatcccca agagcaagaa caaggagttc gagttcatcg acagcaacga cgaggaggag 1320
gacaagaagg accagaagaa gagcaagcag tacatcaagt actgcgacac cgccaagaac 1380
accagctacg gcaagagcgg cggcatccgc ctgtacttca accgcaacga gctggagaac 1440
tacaaggacg gcaagaagat ggacagctac accgtgttca ccctgagcat ccgcgactac 1500
aagagcctgt tcgccaagga gaagctgcag ccccagatct tcaacaccgt ggacaacaag 1560
atcaccagcc tgaagatcca gaagaagttc ggcaacgagg agcagaccaa cttcctgagc 1620
tacttcaccc agaaccagat caccaagaag gactggatgg acgagaagac cttccagaac 1680
gtgaaggagc tgaacgaggg catccgcgtg ctgagcgtgg acctgggcca gcgcttcttc 1740
gccgccgtga gctgcttcga gatcatgagc gagatcgaca acaacaagct gttcttcaac 1800
ctgaacgacc agaaccacaa gatcatccgc atcaacgaca agaactacta cgccaagcac 1860
atctacagca agaccatcaa gctgagcggc gaggacgacg acctgtacaa ggagcgcaag 1920
atcaacaaga actacaagct gagctaccag gagcgcaaga acaagatcgg catcttcacc 1980

cgccagatca acaagctgaa ccagctgctg aagatcatcc gcaacgacga gatcgacaag 2040

gagaagttca aggagctgat cgagaccacc aagcgctacg tgaagaacac ctacaacgac 2100

ggcatcatcg actggaacaa cgtggacaac aagatcctga gctacgagaa caaggaggac 2160

gtgatcaacc tgcacaagga gctggacaag aagctggaga tcgacttcaa ggagttcatc 2220

cgcgagtgcc gcaagcccat cttccgcagc ggcggcctga gcatgcagcg catcgacttc 2280

ctggagaagc tgaacaagct gaagcgcaag tgggtggccc gcacccagaa gagcgccgag 2340

agcatcgtgc tgacccccaa gttcggctac aagctgaagg agcacatcaa cgagctgaag 2400

gacaaccgcg tgaagcaggg cgtgaactac atcctgatga ccgccctggg ctacatcaag 2460

gacaacgaga tcaagaacga cagcaagaag aagcagaagg aggactgggt gaagaagaac 2520

cgcgcctgcc agatcatcct gatggagaag ctgaccgagt acaccttcgc cgaggaccgc 2580

ccccgcgagg agaacagcaa gctgcgcatg tggagccacc gccagatctt caacttcctg 2640

cagcagaagg ccagcctgtg gggcatcctg gtgggcgacg tgttcgcccc ctacaccagc 2700

aagtgcctga gcgacaacaa cgcccccggc atccgctgcc accaggtgac caagaaggac 2760

ctgatcgaca acagctggtt cctgaagatc gtggtgaagg acgacgcctt ctgcgacctg 2820

atcgagatca acaaggagaa cgtgaagaac aagagcatca agatcaacga catcctgccc 2880

ctgcgcggcg gcgagctgtt cgccagcatc aaggacggca agctgcacat cgtgcaggcc 2940

gacatcaacg ccagccgcaa catcgccaag cgcttcctga gccagatcaa ccccttccgc 3000

gtggtgctga agaaggacaa ggacgagacc ttccacctga agaacgagcc caactacctg 3060

aagaactact acagcatcct gaacttcgtg cccaccaacg aggagctgac cttcttcaag 3120

gtggaggaga acaaggacat caagcccacc aagcgcatca agatggacaa gcacgagaag 3180

gagagcaccg acgagggcga cgactacagc aagaaccaga tcgccctgtt ccgcgacgac 3240

agcggcatct tcttcgacaa gagcctgtgg gtggacggca agatcttctg gagcgtggtg 3300

aagaacaaga tgaccaagct gctgcgcgag cgcaacaaca agaagaacgg cagcaag 3357

<210> 20
<211> 3426
<212> DNA
<213> Tuberibacillus calidus

<400> 20
atgaacatcc acctgaagga gctgatccgc atggccacca agagcttcat cctgaagatg 60

aagaccaaga acaaccccca gctgcgcctg agcctgtgga agacccacga gctgttcaac 120

ttcggcgtgg cctactacat ggacctgctg agcctgttcc gccagaagga cctgtacatg 180

cacaacgacg aggaccccga ccaccccgtg gtgctgaaga aggaggagat ccaggagcgc 240

ctgtggatga aggtgcgcga gacccagcag aagaacggct tccacggcga ggtgagcaag 300

gacgaggtgc tggagaccct gcgcgccctg tacgaggagc tggtgcccag cgccgtgggc 360

aagagcggcg aggccaacca gatcagcaac aagtacctgt accccctgac cgaccccgcc 420

agccagagcg gcaagggcac cgccaacagc ggccgcaagc cccgctggaa gaagctgaag 480

gaggccggcg accccagctg gaaggacgcc tacgagaagt gggagaagga gcgccaggag 540

gaccccaagc tgaagatcct ggccgccctg cagagcttcg gcctgatccc cctgttccgc 600

cccttcaccg agaacgacca caaggccgtg atcagcgtga agtggatgcc caagagcaag 660

aaccagagcg tgcgcaagtt cgacaaggac atgttcaacc aggccatcga gcgcttcctg 720

agctgggaga gctggaacga gaaggtggcc gaggactacg agaagaccgt gagcatctac 780

gagagcctgc agaaggagct gaagggcatc agcaccaagg ccttcgagat catggagcgc 840

gtggagaagg cctacgaggc ccacctgcgc gagatcacct tcagcaacag cacctaccgc 900

atcggcaacc gcgccatccg cggctggacc gagatcgtga agaagtggat gaagctggac 960

cccagcgccc cccagggcaa ctacctggac gtggtgaagg actaccagcg ccgccacccc 1020

cgcgagagcg gcgacttcaa gctgttcgag ctgctgagcc gccccgagaa ccaggccgcc 1080

tggcgcgagt accccgagtt cctgcccctg tacgtgaagt accgccacgc cgagcagcgc 1140

atgaagaccg ccaagaagca ggccaccttc accctgtgcg accccatccg ccaccccctg 1200

tgggtgcgct acgaggagcg cagcggcacc aacctgaaca agtaccgcct gatcatgaac 1260

gagaaggaga aggtggtgca gttcgaccgc ctgatctgcc tgaacgccga cggccactac 1320

gaggagcagg aggacgtgac cgtgcccctg gcccccagcc agcagttcga cgaccagatc 1380

aagttcagca gcgaggacac cggcaagggc aagcacaact tcagctacta ccacaagggc 1440

atcaactacg agctgaaggg caccctgggc ggcgcccgca tccagttcga ccgcgagcac 1500

ctgctgcgcc gccagggcgt gaaggccggc aacgtgggcc gcatcttcct gaacgtgacc 1560

ctgaacatcg agcccatgca gcccttcagc cgcagcggca acctgcagac cagcgtgggc 1620

aaggccctga aggtgtacgt ggacggctac cccaaggtgg tgaacttcaa gcccaaggag 1680

ctgaccgagc acatcaagga gagcgagaag aacaccctga ccctgggcgt ggagagcctg 1740

cccaccggcc tgcgcgtgat gagcgtggac ctgggccagc gccaggccgc cgccatcagc 1800

atcttcgagg tggtgagcga gaagcccgac gacaacaagc tgttctaccc cgtgaaggac 1860

accgacctgt tcgccgtgca ccgcaccagc ttcaacatca agctgcccgg cgagaagcgc 1920

accgagcgcc gcatgctgga gcagcagaag cgcgaccagg ccatccgcga cctgagccgc 1980

aagctgaagt tcctgaagaa cgtgctgaac atgcagaagc tggagaagac cgacgagcgc 2040

gagaagcgcg tgaaccgctg gatcaaggac cgcgagcgcg aggaggagaa ccccgtgtac 2100

gtgcaggagt tcgagatgat cagcaaggtg ctgtacagcc cccacagcgt gtgggtggac 2160

cagctgaaga gcatccaccg caagctggag gagcagctgg gcaaggagat cagcaagtgg 2220

cgccagagca tcagccaggg ccgccagggc gtgtacggca tcagcctgaa gaacatcgag 2280

gacatcgaga agacccgccg cctgctgttc cgctggagca tgcgccccga gaaccccggc 2340

gaggtgaagc agctgcagcc cggcgagcgc ttcgccatcg accagcagaa ccacctgaac 2400

cacctgaagg acgaccgcat caagaagctg gccaaccaga tcgtgatgac cgccctgggc 2460

taccgctacg acggcaagcg caagaagtgg atcgccaagc accccgcctg ccagctggtg 2520

ctgttcgagg acctgagccg ctacgccttc tacgacgagc gcagccgcct ggagaaccgc 2580

aacctgatgc gctggagccg ccgcgagatc cccaagcagg tggcccagat cggcggcctg 2640

tacggcctgc tggtgggcga ggtgggcgcc cagtacagca gccgcttcca cgccaagagc 2700

ggcgcccccg gcatccgctg ccgcgtggtg aaggagcacg agctgtacat caccgagggc 2760

ggccagaagg tgcgcaacca gaagttcctg gacagcctgg tggagaacaa catcatcgag 2820

cccgacgacg cccgccgcct ggagcccggc gacctgatcc gcgaccaggg cggcgacaag 2880

ttcgccaccc tggacgagcg cggcgagctg gtgatcaccc acgccgacat caacgccgcc 2940

cagaacctgc agaagcgctt ctggacccgc acccacggcc tgtaccgcat ccgctgcgag 3000

agccgcgaga tcaaggacgc cgtggtgctg gtgcccagcg acaaggacca gaaggagaag 3060

atggagaacc tgttcggcat cggctacctg cagcccttca agcaggagaa cgacgtgtac 3120

aagtgggtga agggcgagaa gatcaagggc aagaagacca gcagccagag cgacgacaag 3180

gagctggtga gcgagatcct gcaggaggcg agcgtgatgg ccgacgagct gaagggcaac 3240

cgcaagaccc tgttccgcga ccccagcggc tacgtgttcc ccaaggaccg ctggtacacc 3300

ggcggccgct acttcggcac cctggagcac ctgctgaagc gcaagctggc cgagcgccgc 3360

ctgttcgacg gcggcagcag ccgccgcggc ctgttcaacg gcaccgacag caacaccaac 3420

gtggag 3426

<210> 21
<211> 2870
<212> DNA
<213> Artificial Sequence

<220>
<223> pCAG-2AeGFP ²¿•ÖÐòÁÐ (CAG-NLS-XmaI-NheI-NLS-T2A-eGFP-SV40)

<400> 21
gacattgatt attgactagt tattaatagt aatcaattac ggggtcatta gttcatagcc 60

catatatgga gttccgcgtt acataactta cggtaaatgg cccgcctggc tgaccgccca 120

acgacccccg cccattgacg tcaataatga cgtatgttcc catagtaacg ccaataggga 180

ctttccattg acgtcaatgg gtggactatt tacggtaaac tgcccacttg gcagtacatc 240

aagtgtatca tatgccaagt acgcccccta ttgacgtcaa tgacggtaaa tggcccgcct 300

ggcattatgc ccagtacatg accttatggg actttcctac ttggcagtac atctacgtat 360

tagtcatcgc tattaccatg ggtcgaggtg agccccacgt tctgcttcac tctccccatc 420

tcccccccct ccccaccccc aattttgtat ttatttattt tttaattatt ttgtgcagcg 480

atgggggcgg gggggggggg ggcgcgcgcc aggcggggcg gggcggggcg aggggcgggg 540

cggggcgagg cggagaggtg cggcggcagc caatcagagc ggcgcgctcc gaaagtttcc 600

ttttatggcg aggcggcggc ggcggcggcc ctataaaaag cgaagcgcgc ggcgggcggg 660

agtcgctgcg ttgccttcgc cccgtgcccc gctccgcgcc gcctcgcgcc gcccgccccg 720

gctctgactg accgcgttac tcccacaggt gagcgggcgg gacggccctt ctcctccggg 780

ctgtaattag cgcttggttt aatgacggct cgtttctttt ctgtggctgc gtgaaagcct 840

taaagggctc cgggagggcc ctttgtgcgg ggggagcggc tcggggggtg cgtgcgtgtg 900

tgtgtgcgtg gggagcgccg cgtgcggccc gcgctgcccg gcggctgtga gcgctgcggg 960

cgcggcgcgg ggctttgtgc gctccgcgtg tgcgcgaggg gagcgcggcc ggggggcggt 1020

gccccgcggt gcgggggggc tgcgagggga acaaaggctg cgtgcggggt gtgtgcgtgg 1080

gggggtgagc agggggtgtg ggcgcggcgg tcggctgtaa cccccccctg cacccccctc 1140

cccgagttgc tgagcacggc ccggcttcgg gtgcggggct ccgtacgggg cgtggcgcgg 1200

ggctcgccgt gccgggcggg gggtggcggc aggtgggggt gccgggcggg gcggggccgc 1260

ctcgggccgg ggagggctcg ggggaggggc gcggcggccc ccggagcgcc ggcggctgtc 1320

gaggcgcggc gagccgcagc cattgccttt tatggtaatc gtgcgagagg gcgcagggac 1380

ttcctttgtc ccaaatctgt gcggagccga aatctgggag gcgccgccgc acccccctct 1440

agcgggcgcg gggcgaagcg gtgcggcgcc ggcaggaagg aaatgggcgg ggagggcctt 1500

cgtgcgtcgc cgcgccgccg tccccttctc catctccagc ctcggggctg tccgcagggg 1560

gacggctgcc ttcggggggg acggggcagg gcggggttcg gcttctggcg tgtgaccggc 1620

ggctctagcg cctctgctaa ccatgttcat gccttcttct ttttcctaca gctcctgggc 1680

aacgtgctgg ttattgtgct gtctcatcat tttggcaaag ctagtgaatt ctaatacgac 1740

tcactatagg ccgccaccat gcccaagaag aagaggaagg ttcccggggc tagcccaaag 1800

aagaagagga aagtctctag ataccttatg atgttccaga ttatgccgga tacccatacg 1860

atgtccctga ctatgcaggc tcctacccct tatgacgtcc cagactacgc cggatccagg 1920

tccggcggcg gagagggcag aggaagtctt ctaacatgcg gtgacgtgga ggagaatccc 1980

ggcccaatgg tgagcaaggg cgaggagctg ttcaccgggg tggtgcccat cctggtcgag 2040

ctggacggcg acgtaaacgg ccacaagttc agcgtgtccg gcgagggcga gggcgatgcc 2100

acctacggca agctgaccct gaagttcatc tgcaccaccg gcaagctgcc cgtgccctgg 2160

cccaccctcg tgaccaccct gacctacggc gtgcagtgct tcagccgcta ccccgaccac 2220

atgaagcagc acgacttctt caagtccgcc atgcccgaag gctacgtcca ggagcgcacc 2280

atcttcttca aggacgacgg caactacaag acccgcgccg aggtgaagtt cgagggcgac 2340

accctggtga accgcatcga gctgaagggc atcgacttca aggaggacgg caacatcctg 2400

gggcacaagc tggagtacaa ctacaacagc cacaacgtct atatcatggc cgacaagcag 2460

aagaacggca tcaaggtgaa cttcaagatc cgccacaaca tcgaggacgg cagcgtgcag 2520

ctcgccgacc actaccagca gaacaccccc atcggcgacg gccccgtgct gctgcccgac 2580

aaccactacc tgagcaccca gtccgccctg agcaaagacc ccaacgagaa gcgcgatcac 2640

atggtcctgc tggagttcgt gaccgccgcc gggatcactc tcggcatgga cgagctgtac 2700

aagtaactgc agcgcgggga tctcatgctg gagttcttcg cccaccccaa cttgtttatt 2760

gcagcttata atggttacaa ataaagcaat agcatcacaa atttcacaaa taaagcattt 2820

ttttcactgc attctagttg tggtttgtcc aaactcatca atgtatctta 2870

<210> 22
<211> 410
<212> DNA
<213> Artificial Sequence

<220>
<223> pUC19-U6 ²¿•ÖÐòÁÐ(U6-BasI-HindIII)

<220>
<221> misc_feature
<222> (283)..(289)
<223> n is a, c, g, or t

<220>
<221> misc_feature
<222> (297)..(375)
<223> n is a, c, g, or t

<400> 22
tgtaaaacga cggccagtga attcgagggc ctatttccca tgattccttc atatttgcat 60

atacgataca aggctgttag agagataatt ggaattaatt tgactgtaaa cacaaagata 120

ttagtacaaa atacgtgacg tagaaagtaa taatttcttg ggtagtttgc agttttaaaa 180

ttatgtttta aaatggacta tcatatgctt accgtaactt gaaagtattt cgatttcttg 240

gctttatata tcttgtggaa aggacgaaac accggagaga ccnnnnnnng gtctcannnn 300

nnnnnnnnnn nnnnnnnnnn nnnnnnnnnn nnnnnnnnnn nnnnnnnnnn nnnnnnnnnn 360

nnnnnnnnnn nnnnnaagct tggcgtaatc atggtcatag ctgtttcctg 410

<210> 23
<211> 476
<212> DNA

<213>  Artificial Sequence

<220>
<223>  pUC19-U6-AasgRNA ²¿•ÖÐòÁÐ(U6-AasgRNA_ scaffold-BasI-BasI-terminator)

<400>  23
tgtaaaacga cggccagtga attcgagggc ctatttccca tgattccttc atatttgcat      60

atacgataca aggctgttag agagataatt ggaattaatt tgactgtaaa cacaaagata     120

ttagtacaaa atacgtgacg tagaaagtaa taatttcttg ggtagtttgc agttttaaaa     180

ttatgtttta aaatggacta tcatatgctt accgtaactt gaaagtattt cgatttcttg     240

gctttatata tcttgtggaa aggacgaaac accgggtcta aaggacagaa tttttcaacg     300

ggtgtgccaa tggccacttt ccaggtggca aagcccgttg aacttctcaa aaagaacgct     360

cgctcagtgt tctgacgtcg gatcactgag cgagcgatct gagaagtggc acagagaccg     420

agagagggtc tcattttttt taagcttggc gtaatcatgg tcatagctgt ttcctg         476


<210>  24
<211>  476
<212>  DNA
<213>  Artificial Sequence

<220>
<223>  pUC19-U6-AksgRNA²¿•ÖÐòÁÐ(U6-AksgRNA1_ scaffold-BasI-BasI-ÖÕÖ¹×Ó)

<400>  24
tgtaaaacga cggccagtga attcgagggc ctatttccca tgattccttc atatttgcat      60

atacgataca aggctgttag agagataatt ggaattaatt tgactgtaaa cacaaagata     120

ttagtacaaa atacgtgacg tagaaagtaa taatttcttg ggtagtttgc agttttaaaa     180

ttatgtttta aaatggacta tcatatgctt accgtaactt gaaagtattt cgatttcttg     240

gctttatata tcttgtggaa aggacgaaac accggtcgtc tataggacgg cgaggacaac     300

gggaagtgcc aatgtgctct ttccaagagc aaacaccccg ttggcttcaa gatgaccgct     360

cgctcagcga tctgacaacg gatcgctgag cgagcggtct gagaagtggc acagagaccg     420

agagagggtc tcattttttt taagcttggc gtaatcatgg tcatagctgt ttcctg         476


<210>  25
<211>  484
<212>  DNA
<213>  Artificial Sequence

<220>
<223>  pUC19-U6-AmsgRNA²¿•ÖÐòÁÐ(U6-AmsgRNA1_ scaffold-BasI-BasI-ÖÕÖ¹×Ó)

<400>  25
tgtaaaacga cggccagtga attcgagggc ctatttccca tgattccttc atatttgcat      60

atacgataca aggctgttag agagataatt ggaattaatt tgactgtaaa cacaaagata     120

ttagtacaaa atacgtgacg tagaaagtaa taatttcttg ggtagtttgc agttttaaaa     180

ttatgtttta aaatggacta tcatatgctt accgtaactt gaaagtattt cgatttcttg 240

gctttatata tcttgtggaa aggacgaaac accggggaat tgccgatcta taggacggca 300

gattcaacgg gatgtgccaa tgcactcttt ccaggagtga acaccccgtt ggcttcaaca 360

tgatcgcccg ctcaacggtc cgatgtcgga tcgttgagcg ggcgatctga gaagtggcac 420

agagaccgag agagggtctc atttttttta agcttggcgt aatcatggtc atagctgttt 480

cctg 484

<210> 26
<211> 439
<212> DNA
<213> Artificial Sequence

<220>
<223> pUC19-U6-BhsgRNA ²¿•ÖÐòÁÐ(U6-BhsgRNA1_ scaffold-BasI-BasI-ÖÕÖ¹×Ó)

<400> 26
tgtaaaacga cggccagtga attcgagggc ctatttccca tgattccttc atatttgcat 60

atacgataca aggctgttag agagataatt ggaattaatt tgactgtaaa cacaaagata 120

ttagtacaaa atacgtgacg tagaaagtaa taatttcttg ggtagtttgc agttttaaaa 180

ttatgtttta aaatggacta tcatatgctt accgtaactt gaaagtattt cgatttcttg 240

gctttatata tcttgtggaa aggacgaaac accgggaggt tctgtctttt ggtcaggaca 300

accgtctagc tataagtgct gcagggtgtg agaaactcct attgctggac gatgtctctt 360

acgaggcatt agcacagaga ccgagagagg gtctcatttt ttttaagctt ggcgtaatca 420

tggtcatagc tgtttcctg 439

<210> 27
<211> 471
<212> DNA
<213> Artificial Sequence

<220>
<223> pUC19-U6-BssgRNA ²¿•ÖÐòÁÐ(U6-BssgRNA1_ scaffold-BasI-BasI-ÖÕÖ¹×Ó)

<400> 27
tgtaaaacga cggccagtga attcgagggc ctatttccca tgattccttc atatttgcat 60

atacgataca aggctgttag agagataatt ggaattaatt tgactgtaaa cacaaagata 120

ttagtacaaa atacgtgacg tagaaagtaa taatttcttg ggtagtttgc agttttaaaa 180

ttatgtttta aaatggacta tcatatgctt accgtaactt gaaagtattt cgatttcttg 240

gctttatata tcttgtggaa aggacgaaac accggccata agtcgactta catatccgtg 300

cgtgtgcatt atgggcccat ccacaggtct attcccacgg ataatcacga ctttccacta 360

agctttcgaa tgttcgaaag cttagtggaa agcttcgtgg ttagcacaga gaccgagaga 420

gggtctcatt ttttttaagc ttggcgtaat catggtcata gctgtttcct g 471

<210> 28
<211> 469
<212> DNA
<213> Artificial Sequence

<220>
<223> pUC19-U6-Bs3sgRNA²¿•ÖÐòÁÐ(U6-BssgRNA1_ scaffold-BasI-BasI-ÖÕÖ¹×Ó)

<400> 28
tgtaaaacga cggccagtga attcgagggc ctatttccca tgattccttc atatttgcat 60

atacgataca aggctgttag agagataatt ggaattaatt tgactgtaaa cacaaagata 120

ttagtacaaa atacgtgacg tagaaagtaa taatttcttg ggtagtttgc agttttaaaa 180

ttatgtttta aaatggacta tcatatgctt accgtaactt gaaagtattt cgatttcttg 240

gctttatata tcttgtggaa aggacgaaac accggggtga cctatagggt caatgaatct 300

gtgcgtgtgc cataagtaat taaaaattac ccaccacagg attatcttat ttctgctaag 360

tgtttagttg cctgaatact tagcagaaat aatgatgatt ggcacagaga ccgagagagg 420

gtctcatttt ttttaagctt ggcgtaatca tggtcatagc tgtttcctg 469

<210> 29
<211> 457
<212> DNA
<213> Artificial Sequence

<220>
<223> pUC19-U6-LssgRNA ²¿•ÖÐòÁÐ(U6-BssgRNA1_ scaffold-BasI-BasI-ÖÕÖ¹×Ó)

<400> 29
tgtaaaacga cggccagtga attcgagggc ctatttccca tgattccttc atatttgcat 60

atacgataca aggctgttag agagataatt ggaattaatt tgactgtaaa cacaaagata 120

ttagtacaaa atacgtgacg tagaaagtaa taatttcttg ggtagtttgc agttttaaaa 180

ttatgtttta aaatggacta tcatatgctt accgtaactt gaaagtattt cgatttcttg 240

gctttatata tcttgtggaa aggacgaaac accggggcaa agaatactgt gcgtgtgcta 300

aggatggaaa aaatccattc aaccacagga ttacattatt tatctaatca cttaaatctt 360

taagtgatta gatgaattaa atgtgattag cacagagacc gagagagggt ctcatttttt 420

ttaagcttgg cgtaatcatg gtcatagctg tttcctg 457

<210> 30
<211> 465
<212> DNA
<213> Artificial Sequence

<220>
<223> pUC19-U6-SbsgRNA ²¿•ÖÐòÁÐ(U6-BssgRNA1_ scaffold-BasI-BasI-ÖÕÖ¹×Ó)

<400>  30
tgtaaaacga cggccagtga attcgagggc ctatttccca tgattccttc atatttgcat      60

atacgataca aggctgttag agagataatt ggaattaatt tgactgtaaa cacaaagata     120

ttagtacaaa atacgtgacg tagaaagtaa taatttcttg ggtagtttgc agttttaaaa     180

ttatgtttta aaatggacta tcatatgctt accgtaactt gaaagtattt cgatttcttg     240

gctttatata tcttgtggaa aggacgaaac accgggtctt agggtatatc ccaaatttgt     300

cttagtatgt gcattgctta cagcgacaac taaggtttgt ttatcttttt tttacattgt     360

aagatgtttt acattataaa aagaagataa tcttattgca cagagaccga gagagggtct     420

catttttttt aagcttggcg taatcatggt catagctgtt tcctg                     465


<210>  31
<211>  137
<212>  DNA
<213>  Artificial Sequence

<220>
<223>  AasgRNA_ scaffold

<400>  31
gtctaaagga cagaattttt caacgggtgt gccaatggcc actttccagg tggcaaagcc      60

cgttgaactt ctcaaaaaga acgctcgctc agtgttctga cgtcggatca ctgagcgagc     120

gatctgagaa gtggcac                                                    137


<210>  32
<211>  137
<212>  DNA
<213>  Artificial Sequence

<220>
<223>  AksgRNA1_ scaffold

<400>  32
tcgtctatag gacggcgagg acaacgggaa gtgccaatgt gctctttcca agagcaaaca      60

ccccgttggc ttcaagatga ccgctcgctc agcgatctga caacggatcg ctgagcgagc     120

ggtctgagaa gtggcac                                                    137


<210>  33
<211>  145
<212>  DNA
<213>  Artificial Sequence

<220>
<223>  AmsgRNA1_ scaffold

<400>  33
ggaattgccg atctatagga cggcagattc aacgggatgt gccaatgcac tctttccagg      60

agtgaacacc ccgttggctt caacatgatc gcccgctcaa cggtccgatg tcggatcgtt     120

gagcgggcga tctgagaagt ggcac                                          145

<210>  34
<211>  100
<212>  DNA
<213>  Artificial Sequence

<220>
<223>  BhsgRNA_ scaffold

<400>  34
gaggttctgt cttttggtca ggacaaccgt ctagctataa gtgctgcagg gtgtgagaaa      60

ctcctattgc tggacgatgt ctcttttatt tctttttctt tggatgtcca agaaaaaaga      120

aatgatacga ggcattagca c                                              141

<210>  35
<211>  132
<212>  DNA
<213>  Artificial Sequence

<220>
<223>  BssgRNA_ scaffold

<400>  35
ccataagtcg acttacatat ccgtgcgtgt gcattatggg cccatccaca ggtctattcc      60

cacggataat cacgactttc cactaagctt tcgaatgttc gaaagcttag tggaaagctt      120

cgtggttagc ac                                                        132

<210>  36
<211>  130
<212>  DNA
<213>  Artificial Sequence

<220>
<223>  Bs3sgRNA_ scaffold

<400>  36
ggtgacctat agggtcaatg aatctgtgcg tgtgccataa gtaattaaaa attacccacc      60

acaggattat cttatttctg ctaagtgttt agttgcctga atacttagca gaaataatga      120

tgattggcac                                                           130

<210>  37
<211>  118
<212>  DNA
<213>  Artificial Sequence

<220>
<223>  LssgRNA_ scaffold

<400>  37
ggcaaagaat actgtgcgtg tgctaaggat ggaaaaaatc cattcaacca caggattaca      60

ttatttatct aatcacttaa atctttaagt gattagatga attaaatgtg attagcac      118

<210>  38
<211>  126
<212>  DNA
<213>  Artificial Sequence

<220>
<223>  SbsgRNA_ scaffold

<400>  38
gtcttagggt atatcccaaa tttgtcttag tatgtgcatt gcttacagcg acaactaagg      60

tttgtttatc ttttttttac attgtaagat gttttacatt ataaaaagaa gataatctta      120

ttgcac                                                                 126


<210>  39
<211>  86
<212>  DNA
<213>  Artificial Sequence

<220>
<223>  artsgRA1 scaffold

<400>  39
ggtctaaagg acagaatttt tcaacgggtg tgccaatggc cactttccag gtggcaaagc      60

ccgttgaact tcaagcgaag tggcac                                           86


<210>  40
<211>  84
<212>  DNA
<213>  Artificial Sequence

<220>
<223>  artsgRA2 scaffold

<400>  40
ggtctaaagg acagaagaca acgggaagtg ccaatgtgct ctttccaaga gcaaacaccc      60

cgttgacttc aagcgaagtg gcac                                             84


<210>  41
<211>  79
<212>  DNA
<213>  Artificial Sequence

<220>
<223>   artsgRA3 scaffold

<400>  41
ggtctaaagg acagaaaatc tgtgcgtgtg ccataagtaa ttaaaaatta cccaccacag      60

acttcaagcg aagtggcac                                                   79


<210>  42
<211>  91
<212>  DNA

<213>  Artificial Sequence

<220>
<223>  artsgRA4 scaffold

<400>  42
ggtcgtctat aggacggcga gtttttcaac gggtgtgcca atggccactt tccaggtggc      60

aaagcccgtt gaacttcaag cgaagtggca c                                     91


<210>  43
<211>  89
<212>  DNA
<213>  Artificial Sequence

<220>
<223>  artsgRA5 scaffold

<400>  43
ggtcgtctat aggacggcga ggacaacggg aagtgccaat gtgctctttc caagagcaaa      60

caccccgttg acttcaagcg aagtggcac                                        89


<210>  44
<211>  84
<212>  DNA
<213>  Artificial Sequence

<220>
<223>  artsgRA6 scaffold

<400>  44
ggtcgtctat aggacggcga gaatctgtgc gtgtgccata agtaattaaa aattacccac      60

cacagacttc aagcgaagtg gcac                                             84


<210>  45
<211>  90
<212>  DNA
<213>  Artificial Sequence

<220>
<223>  artsgRA7 scaffold

<400>  45
ggtgacctat agggtcaatg tttttcaacg ggtgtgccaa tggccacttt ccaggtggca      60

aagcccgttg aacttcaagc gaagtggcac                                       90


<210>  46
<211>  88
<212>  DNA
<213>  Artificial Sequence

<220>
<223>  artsgRA8 scaffold

<400>  46
ggtgacctat agggtcaatg gacaacggga agtgccaatg tgctctttcc aagagcaaac      60

accccgttga cttcaagcga agtggcac                                          88

<210>  47
<211>  83
<212>  DNA
<213>  Artificial Sequence

<220>
<223>  artsgRA9 scaffold

<400>  47
ggtgacctat agggtcaatg aatctgtgcg tgtgccataa gtaattaaaa attacccacc      60

acagacttca agcgaagtgg cac                                              83

<210>  48
<211>  85
<212>  DNA
<213>  Artificial Sequence

<220>
<223>  artsgRA10 scaffold

<400>  48
ggtctaaagg acagaatttt tcaacgggtg tgccaatggc cactttccag gtggcaaagc      60

ccgttgagct tcaaagaagt ggcac                                            85

<210>  49
<211>  83
<212>  DNA
<213>  Artificial Sequence

<220>
<223>  artsgRA11 scaffold

<400>  49
ggtctaaagg acagaagaca acgggaagtg ccaatgtgct ctttccaaga gcaaacaccc      60

cgttggcttc aaagaagtgg cac                                              83

<210>  50
<211>  78
<212>  DNA
<213>  Artificial Sequence

<220>
<223>  artsgRA12 scaffold

<400>  50
ggtctaaagg acagaaaatc tgtgcgtgtg ccataagtaa ttaaaaatta cccaccacag      60

gcttcaaaga agtggcac                                                    78

<210>  51
<211>  90
<212>  DNA

<213>  Artificial Sequence

<220>
<223>  artsgRA13 scaffold

<400>  51
ggtcgtctat aggacggcga gtttttcaac gggtgtgcca atggccactt tccaggtggc      60

aaagcccgtt gagcttcaaa gaagtggcac                                        90


<210>  52
<211>  88
<212>  DNA
<213>  Artificial Sequence

<220>
<223>  artsgRA14 scaffold

<400>  52
ggtcgtctat aggacggcga ggacaacggg aagtgccaat gtgctctttc caagagcaaa      60

caccccgttg gcttcaaaga agtggcac                                          88


<210>  53
<211>  83
<212>  DNA
<213>  Artificial Sequence

<220>
<223>  artsgRA15 scaffold

<400>  53
ggtcgtctat aggacggcga gaatctgtgc gtgtgccata agtaattaaa aattacccac      60

cacaggcttc aaagaagtgg cac                                               83


<210>  54
<211>  89
<212>  DNA
<213>  Artificial Sequence

<220>
<223>  artsgRA16 scaffold

<400>  54
ggtgacctat agggtcaatg tttttcaacg ggtgtgccaa tggccacttt ccaggtggca      60

aagcccgttg agcttcaaag aagtggcac                                         89


<210>  55
<211>  87
<212>  DNA
<213>  Artificial Sequence

<220>
<223>  artsgRA17 scaffold

<400>  55
ggtgacctat agggtcaatg gacaacggga agtgccaatg tgctctttcc aagagcaaac      60

accccgttgg cttcaaagaa gtggcac                                          87

<210>  56
<211>  82
<212>  DNA
<213>  Artificial Sequence

<220>
<223>  artsgRA18 scaffold

<400>  56
ggtgacctat agggtcaatg aatctgtgcg tgtgccataa gtaattaaaa attacccacc      60

acaggcttca aagaagtggc ac                                               82

<210>  57
<211>  89
<212>  DNA
<213>  Artificial Sequence

<220>
<223>  artsgRA19 scaffold

<400>  57
ggtctaaagg acagaatttt tcaacgggtg tgccaatggc cactttccag gtggcaaagc      60

ccgttgagat tatctatgat gattggcac                                        89

<210>  58
<211>  87
<212>  DNA
<213>  Artificial Sequence

<220>
<223>  artsgRA20 scaffold

<400>  58
ggtctaaagg acagaagaca acgggaagtg ccaatgtgct ctttccaaga gcaaacaccc      60

cgttggatta tctatgatga ttggcac                                          87

<210>  59
<211>  82
<212>  DNA
<213>  Artificial Sequence

<220>
<223>  artsgRA21 scaffold

<400>  59
ggtctaaagg acagaaaatc tgtgcgtgtg ccataagtaa ttaaaaatta cccaccacag      60

gattatctat gatgattggc ac                                               82

<210>  60
<211>  94
<212>  DNA

<213>  Artificial Sequence

<220>
<223>  artsgRA22 scaffold

<400>  60
ggtcgtctat aggacggcga gtttttcaac gggtgtgcca atggccactt tccaggtggc      60

aaagcccgtt gagattatct atgatgattg gcac                                  94


<210>  61
<211>  92
<212>  DNA
<213>  Artificial Sequence

<220>
<223>  artsgRA23 scaffold

<400>  61
ggtcgtctat aggacggcga ggacaacggg aagtgccaat gtgctctttc caagagcaaa      60

caccccgttg gattatctat gatgattggc ac                                    92


<210>  62
<211>  87
<212>  DNA
<213>  Artificial Sequence

<220>
<223>  artsgRA24 scaffold

<400>  62
ggtcgtctat aggacggcga gaatctgtgc gtgtgccata agtaattaaa aattacccac      60

cacaggatta tctatgatga ttggcac                                          87


<210>  63
<211>  93
<212>  DNA
<213>  Artificial Sequence

<220>
<223>  artsgRA25 scaffold

<400>  63
ggtgacctat agggtcaatg tttttcaacg ggtgtgccaa tggccacttt ccaggtggca      60

aagcccgttg agattatcta tgatgattgg cac                                   93


<210>  64
<211>  91
<212>  DNA
<213>  Artificial Sequence

<220>
<223>  artsgRA26 scaffold

<400>  64
ggtgacctat agggtcaatg gacaacggga agtgccaatg tgctctttcc aagagcaaac      60

accccgttgg attatctatg atgattggca c                                    91

<210>  65
<211>  86
<212>  DNA
<213>  Artificial Sequence

<220>
<223>  artsgRA27 scaffold

<400>  65
ggtgacctat agggtcaatg aatctgtgcg tgtgccataa gtaattaaaa attacccacc      60

acaggattat ctatgatgat tggcac                                           86

<210>  66
<211>  82
<212>  DNA
<213>  Artificial Sequence

<220>
<223>  artsgRA28 scaffold

<400>  66
ggtctaaagg acagaacaac gggatgtgcc aatgcactct ttccaggagt gaacaccccg      60

ttgacttcaa gcgaagtggc ac                                               82

<210>  67
<211>  87
<212>  DNA
<213>  Artificial Sequence

<220>
<223>  artsgRA29 scaffold

<400>  67
ggtcgtctat aggacggcga gcaacgggat gtgccaatgc actctttcca ggagtgaaca      60

ccccgttgac ttcaagcgaa gtggcac                                          87

<210>  68
<211>  99
<212>  DNA
<213>  Artificial Sequence

<220>
<223>  artsgRA30 scaffold

<400>  68
ggaattgccg atctatagga cggcagattt ttttcaacgg gtgtgccaat ggccactttc      60

caggtggcaa agcccgttga acttcaagcg aagtggcac                             99

<210>  69
<211>  97
<212>  DNA

<213>  Artificial Sequence

<220>
<223>  artsgRA31 scaffold

<400>  69
ggaattgccg atctatagga cggcagattg acaacgggaa gtgccaatgt gctctttcca      60

agagcaaaca ccccgttgac ttcaagcgaa gtggcac                               97


<210>  70
<211>  95
<212>  DNA
<213>  Artificial Sequence

<220>
<223>  artsgRA32 scaffold

<400>  70
ggaattgccg atctatagga cggcagattc aacgggatgt gccaatgcac tctttccagg      60

agtgaacacc ccgttgactt caagcgaagt ggcac                                 95


<210>  71
<211>  81
<212>  DNA
<213>  Artificial Sequence

<220>
<223>  artsgRA33 scaffold

<400>  71
ggtctaaagg acagaacaac gggatgtgcc aatgcactct ttccaggagt gaacaccccg      60

ttggcttcaa agaagtggca c                                                81


<210>  72
<211>  86
<212>  DNA
<213>  Artificial Sequence

<220>
<223>  artsgRA34 scaffold

<400>  72
ggtcgtctat aggacggcga gcaacgggat gtgccaatgc actctttcca ggagtgaaca      60

ccccgttggc ttcaaagaag tggcac                                           86


<210>  73
<211>  98
<212>  DNA
<213>  Artificial Sequence

<220>
<223>  artsgRA35 scaffold

<400>  73
ggaattgccg atctatagga cggcagattt ttttcaacgg gtgtgccaat ggccactttc      60

caggtggcaa agcccgttga gcttcaaaga agtggcac 98

<210> 74
<211> 96
<212> DNA
<213> Artificial Sequence

<220>
<223> artsgRA36 scaffold

<400> 74
ggaattgccg atctatagga cggcagattg acaacgggaa gtgccaatgt gctctttcca 60

agagcaaaca ccccgttggc ttcaaagaag tggcac 96

<210> 75
<211> 94
<212> DNA
<213> Artificial Sequence

<220>
<223> artsgRA37 scaffold

<400> 75
ggaattgccg atctatagga cggcagattc aacgggatgt gccaatgcac tctttccagg 60

agtgaacacc ccgttggctt caaagaagtg gcac 94

<210> 76
<211> 20
<212> DNA
<213> Artificial Sequence

<220>
<223> CCR5 site 10

<400> 76
tccttctcct gaacaccttc 20

<210> 77
<211> 20
<212> DNA
<213> Artificial Sequence

<220>
<223> CCR5 site 28

<400> 77
tttggcctga ataattgcag 20

<210> 78
<211> 20
<212> DNA
<213> Artificial Sequence

<220>
<223> DNMT1 site 16

<400>  78
cccttcagct aaaataaagg                                                   20


<210>  79
<211>  20
<212>  DNA
<213>  Artificial Sequence

<220>
<223>  RNF2 site 8

<400>  79
tagtcatggt gttcttcaac                                                   20


<210>  80
<211>  20
<212>  DNA
<213>  Artificial Sequence

<220>
<223>  Site 5

<400>  80
gctctcaaga cccacaatcc                                                   20

## Description

### Technical field

The invention relates to the field of genetic engineering. In particular, the present invention relates to genome editing systems based on C2c1 nuclease and methods. The present invention also relates to artificial guide RNA which can be combined with C2c1 nuclease and used for genome editing

### Background technique

Precise genome editing is a promising area for its bright prospects in gene therapy, especially with the appearance of CRISPR-Cas (Clustered Regularly Interspaced Short Palindromic Repeats-CRISPR-associated proteins) systems. Up to now, three types of CRISPR-Cas systems, type II Cas9(Cong, L. et al. Science 339, 819-823 (2013); Mali, P. et al. Science 339, 823-826 (2013)), type V-A Cpf13 (Zetsche, B. et al. Cell 163, 759-771 (2015)) and type V-B C2c1, have been successfully harnessed to facilitate mammalian genome engineering. For type II and V CRISPR-Cas systems, the guide RNA and Cas effector protein are the two core components for target DNA recognition and cleavage (Wright, A.V, Nunez, J.K. & Doudna, J.A. Cell 164, 29-44 (2016); Shmakov, S. et al. Nat Rev Microbiol 15, 169-182 (2017)). Previous studies indicated that the RNA and protein components were interchangeable in closely related Cas9 systems (Fonfara, I. et al. Nucleic Acids Res 42, 2577-2590 (2014)) as well as Cpf1 systems (Zetsche, B. et al. Cell 163, 759-771 (2015)), and could be preliminarily optimized (Nishimasu, H. et al. Cell 156, 935-949 (2014); Zalatan, J.G. et al. Cell 160, 339-350 (2015)). Though a number of emerging CRISPR-Cas systems and boosting investigations promote the wide applications of CRISPR-Cas systems (Wright, A.V, Nunez, J.K. & Doudna, J.A. Cell 164, 29-44 (2016); Shmakov, S. et al. Nat Rev Microbiol 15, 169-182 (2017)), there is still little known about how to re-design or even de novo synthesize an enzymatic genome-engineering system.

The Type V-B CRISPR-C2c1 system is an emerging promising technology for genome engineering. However, C2c1, which can be used for mammalian genome editing, is rare, greatly limiting its application. There remains a need in the art for new C2c1 nuclease-based genome editing systems that can be used for mammalian genome editing.

### Briefly Description of the Invention

In one aspect, the present invention provides a genome editing system for site-directed modification of a target sequence in the genome of a cell, comprising at least one of the following i) to v):
i) a C2c1 protein or variant thereof, and a guide RNA;
ii) an expression construct comprising a nucleotide sequence encoding a C2c1 protein or variant thereof, and a guide RNA;
iii) a C2c1 protein or variant thereof, and an expression construct comprising a nucleotide sequence encoding a guide RNA;
iv) an expression construct comprising a nucleotide sequence encoding a C2c1 protein or variant thereof, and an expression construct comprising a nucleotide sequence encoding a guide RNA;
v) an expression construct comprising a nucleotide sequence encoding a C2c1 protein or variant thereof and a nucleotide sequence encoding a guide RNA;
wherein the guide RNA is capable of forming complex with the C2c1 protein or variant thereof and targeting the C2c1 protein ortholog or variant thereof to the target sequence in the genome of the cell.

In some embodiments, the C2c1 protein is AaC2c1 protein derived from *Alicyclobacillus acidiphilus,* AkC2c1 protein derived from *Alicyclobacillus kakegawensis,* AmC2c1 protein derived from Alicyclobacillus macrosporangiidus, BhC2c1 protein derived from *Bacillus hisashii,* BsC2c1 protein derived from the genus *Bacillus,* Bs3C2c1 protein derived from the genus *Bacillus,* DiC2c1 protein derived from *Desulfovibrio inopinatus,* LsC2c1 protein derived from *Laceyella sediminis,* SbC2c1 protein derived from *Spirochaetes bacterium,* TcC2c1 protein derived from *Tuberibacillus calidus.* For example, the C2c1 protein is the AaC2c1 protein derived from *Alicyclobacillus acidiphilus* NBRC 100859, the AkC2c1 protein derived from *Alicyclobacillus kakegawensis* NBRC 103104, the AmC2c1 protein derived from *Alicyclobacillus macrosporangiidus* strain DSM 17980, the BhC2c1 protein derived from *Bacillus hisashii* strain C4, the BsC2c1 protein derived from genus *Bacillus* NSP2.1, the Bs3C2c1 protein derived from genus *Bacillus* V3-13 contig _40, the DiC2c1 protein derived from *Desulfovibrio inopinatus* DSM 10711, the LsC2c1 protein derived from *Laceyella sediminis* strain RHA1, the SbC2c1 protein derived from *Spirochaetes bacterium* GWB1_27_13, the TcC2c1 protein derived from *Tuberibacillus calidus* DSM 17572.

In the second aspect, the present invention provides a method of site-directed modifying a target sequence in the genome of a cell, comprising introducing the genome editing system of the invention into the cell.

In the third aspect, the invention provides a method of treating a disease in a subject in need thereof, comprising delivering to the subject an effective amount of a genome editing system of the invention to modify a gene related with the disease in the subject.

In the fourth aspect, the invention provides the use of a genome editing system of the invention for the preparation of a pharmaceutical composition for treating a disease in a subject in need thereof, wherein the genome editing system is for modifying a gene related with the disease in the subject.

In a fifth aspect, the invention provides a kit for use in the method of the invention, the kit comprising the genome editing system of the invention, and instructions for use.

In the sixth aspect, the invention provides a pharmaceutical composition for treating a disease in a subject in need thereof, comprising a genome editing system of the invention and a pharmaceutically acceptable carrier, wherein the genome editing system is for modifying a gene related with the disease in the subject.

### Description of the drawings

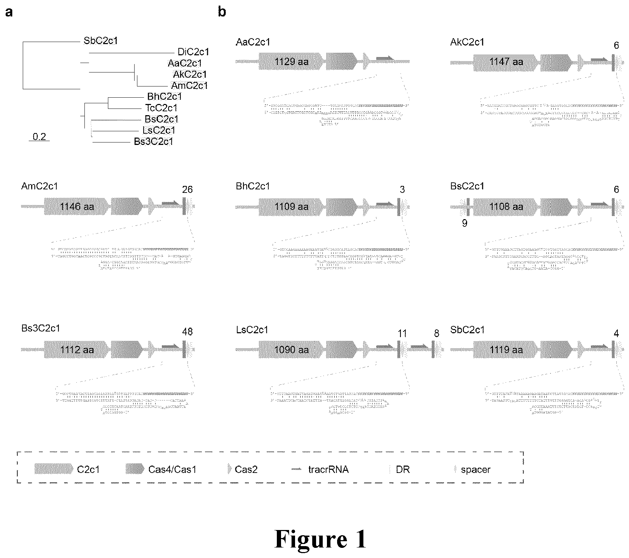

Figure 1 Phylogenetic tree of non-redundant C2c1 orthologs and their loci chosen for genome-editing testing.
   (a) Neighbor Joining phylogenetic tree showing the evolutionary relationships of C2c1 orthologs tested in this study. (b) Maps of bacterial genomic loci corresponding to the 8 C2c1 proteins highlighted in (a). In silico co-folding of the crRNA DR and putative tracrRNA shows stable secondary structure. DR, direct repeat. The number of each bacterial genomic spacers is indicated above or below their CRISPR array.
Figure 2 Protein alignment of C2c1 orthologs. Multiple sequence alignment of the amino acid sequences the 10 C2c1 orthologs tested in this study. Residues that are conserved are highlighted with a red background and conserved mutations are highlighted with an outline and red font.
Figure 3 C2c1 orthologs mediated genome targeting in human 293T cells.
   (a) T7EI assay results indicating the genome targeting activity of the eight C2c1 proteins combined with their cognate sgRNAs in the human genome. Red triangles indicate the cleaved bands. (b) T7EI assay results indicating the simultaneous multiplex genome targeting mediated by Bs3C2c1 combined with its cognate sgRNAs (Bs3sgRNAs) in human 293T cells. (c) Sanger sequencing showing representative indels induced by Bs3C2c1 combined with Bs3sgRNAs. PAM and protospacer sequences are colored in red and blue, respectively. Indels and insertions are symbolled with purple dashes and green lowercases, respectively.

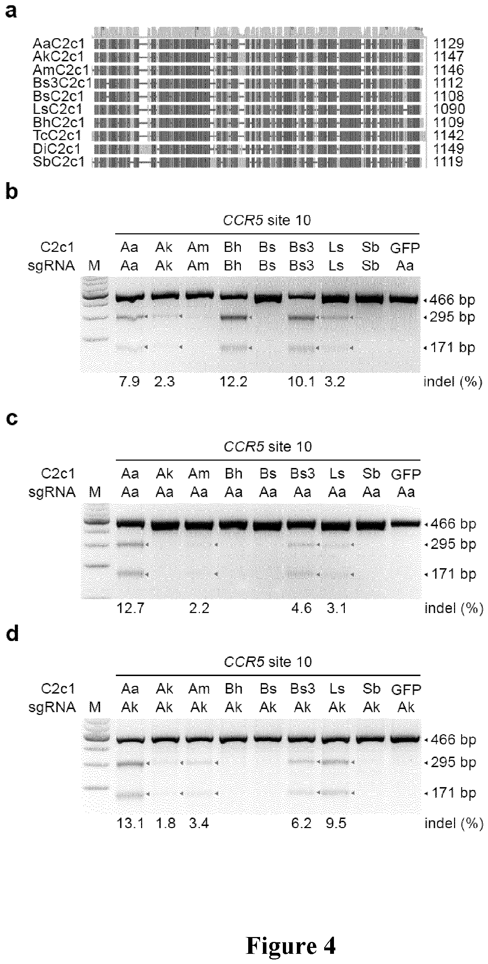

Figure 4 Orthogonal C2c1 proteins for RNA-guided genome editing. (a) Graphical overview of the 10 C2c1 orthologs tested in this study. Sizes (amino acids) are indicated. (b) T7EI assay results indicating the genome targeting activity of the eight C2c1 orthologs directed by their cognate sgRNAs in human 293T cells. Red triangles indicate the cleaved bands. (c-d) T7EI assay results indicating the genome targeting activity of the eight C2c1 orthologs directed by AasgRNA (c) and AksgRNA (d) in human 293T cells. Red triangles indicate the cleaved bands.

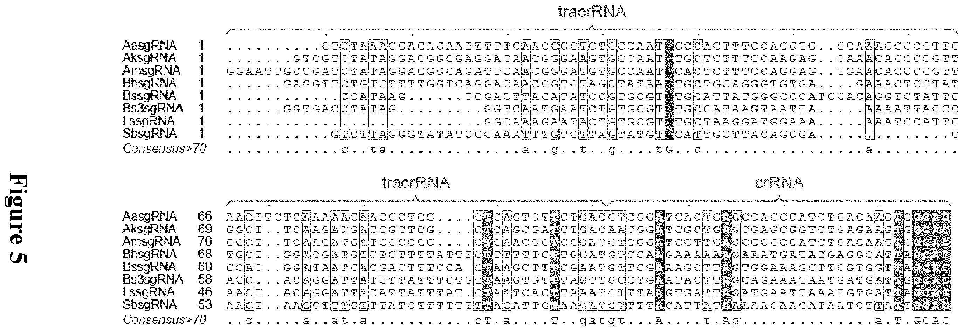

Figure 5 DNA alignment of sgRNAs of C2c1. Multiple sequence alignment of the DNA sequences of the 8 sgRNAs derived from the 10 C2c1 loci tested in this study.

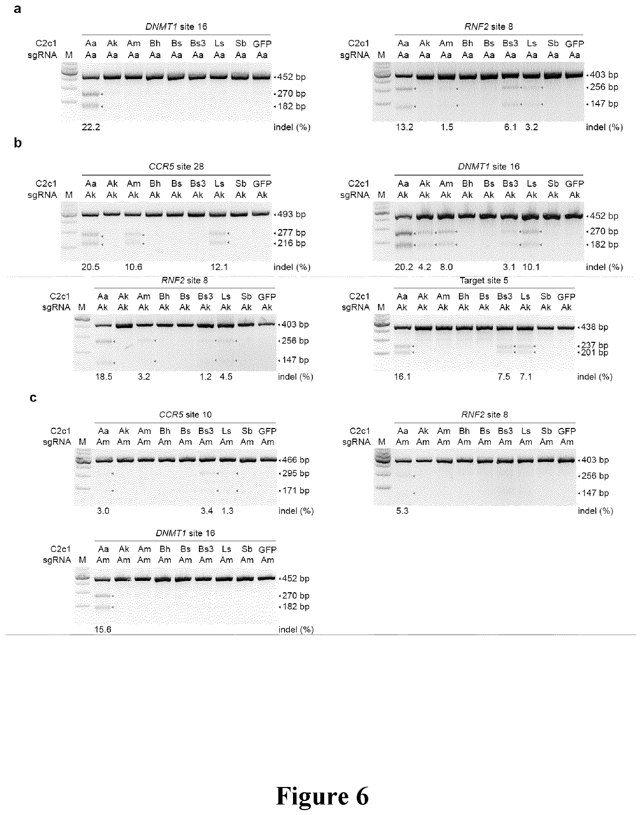

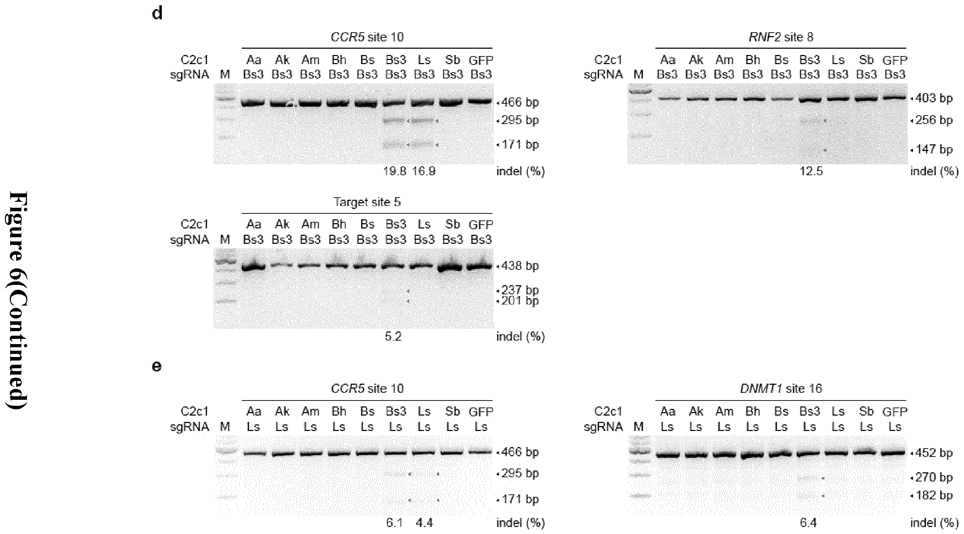

Figure 6 Interchangeability between C2c1 orthologs and their sgRNAs.
   T7EI assay results indicating the genome targeting activity of the eight C2c1 orthologs directed by AasgRNA (a), AksgRNA (b), AmsgRNA (c), Bs3sgRNA (d) and LssgRNA (e) in human 293T cells. Red triangles indicate the cleaved bands.

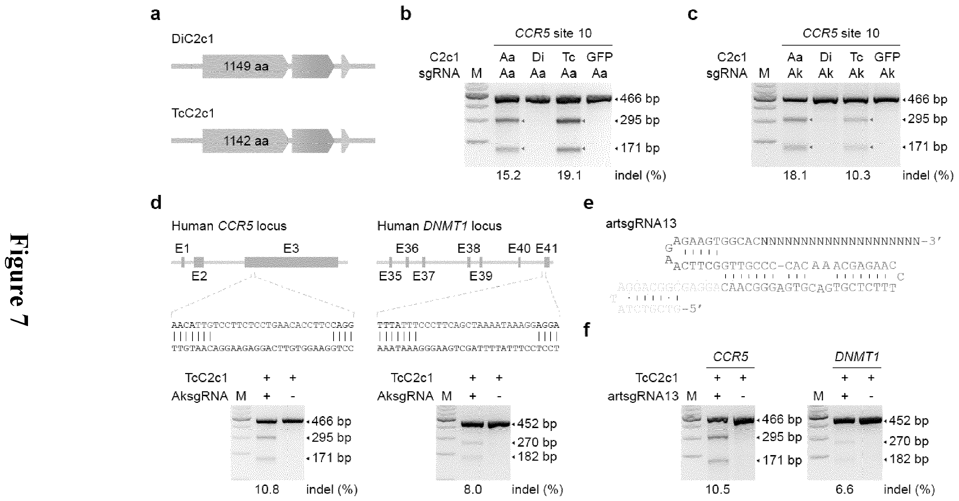

Figure 7 Artificial sgRNAs mediated multiplex genome targeting. (a) Maps pf bacterial genomic loci corresponding to DiC2c1 and TcC2c1. The two C2c1 loci have no CRISPR array. (b-c) T7EI assay results indicating the genome targeting activity of AaC2c1, DiC2c1 and TcC2c1 directed by AasgRNA (b) and AksgRNA (c) in the human 293T cells. Red triangles indicate the cleaved bands. (d) T7EI assay results indicating the simultaneous multiplex genome targeting mediated by TcC2c1 combined with AksgRNAs in human 293T cells. (e) Schematic illustrating the secondary structures of artificial sgRNA scaffold 13 (artsgRNA13). (f) T7EI assay results indicating the simultaneous multiplex genome targeting mediated by TcC2c1 combined with artsgRNA13s in human 293T cells.

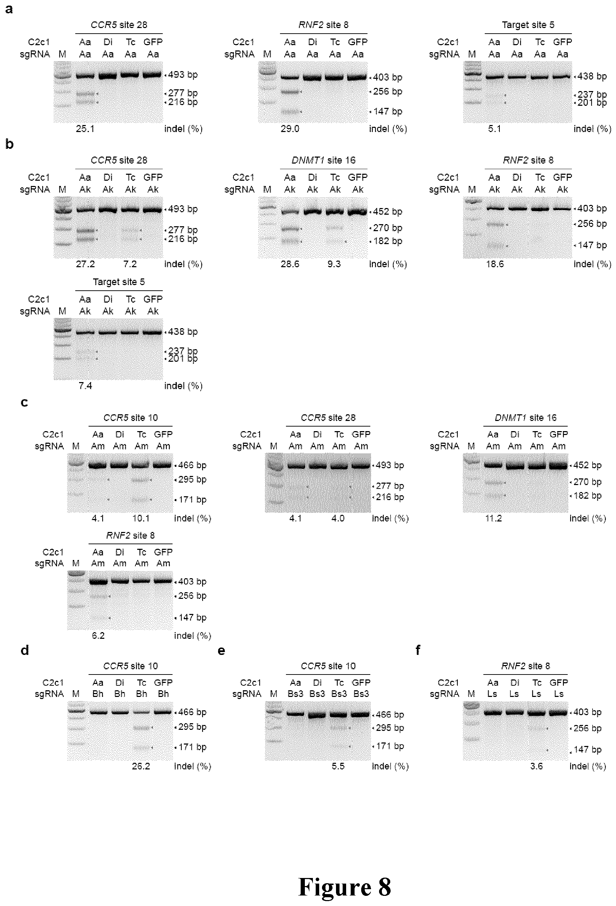

Figure 8 Orthogonal sgRNAs directed C2c1 for genome editing.
   T7EI assay results indicating the genome targeting activity of AaC2c1, DiC2c1 and TcC2c1 directed by AasgRNA (a), AksgRNA (b), AmsgRNA (c), Bs3sgRNA (d) and LssgRNA (e) in the human 293T cells. Red triangles indicate the cleaved bands.

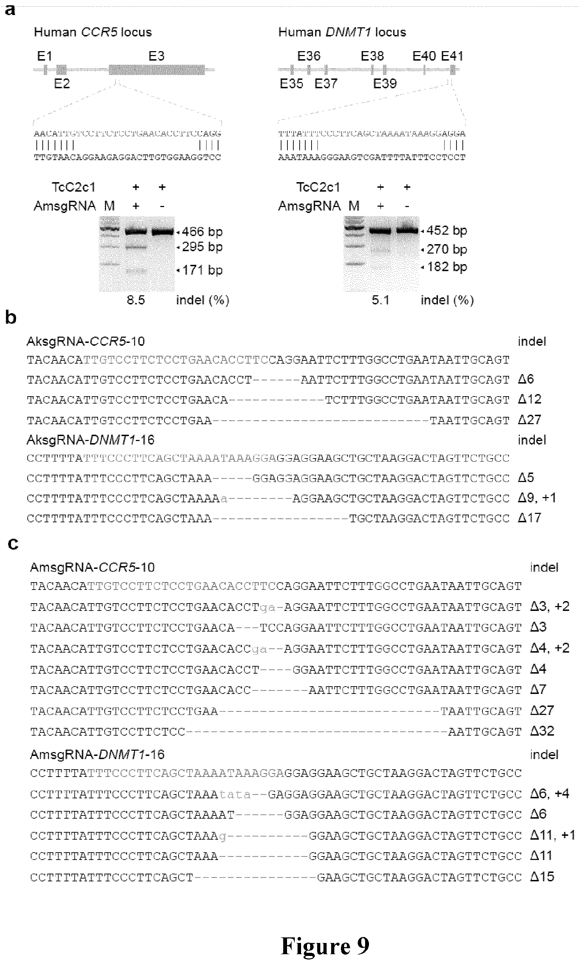

Figure 9 TcC2c1-mediated multiplex genome editing.
   (a) T7EI assay results indicating the simultaneous multiplex genome targeting mediated by TcC2c1 combined with AmsgRNAs in human 293T cells. (b-c) Sanger sequencing shows representative indels induced by TcC2c1 combined with AksgRNAs (b) and AmsgRNAs (c). PAM and protospacer sequences are colored in red and blue, respectively. Indels and insertions are symbolled with purple dashes and green lowercases, respectively.

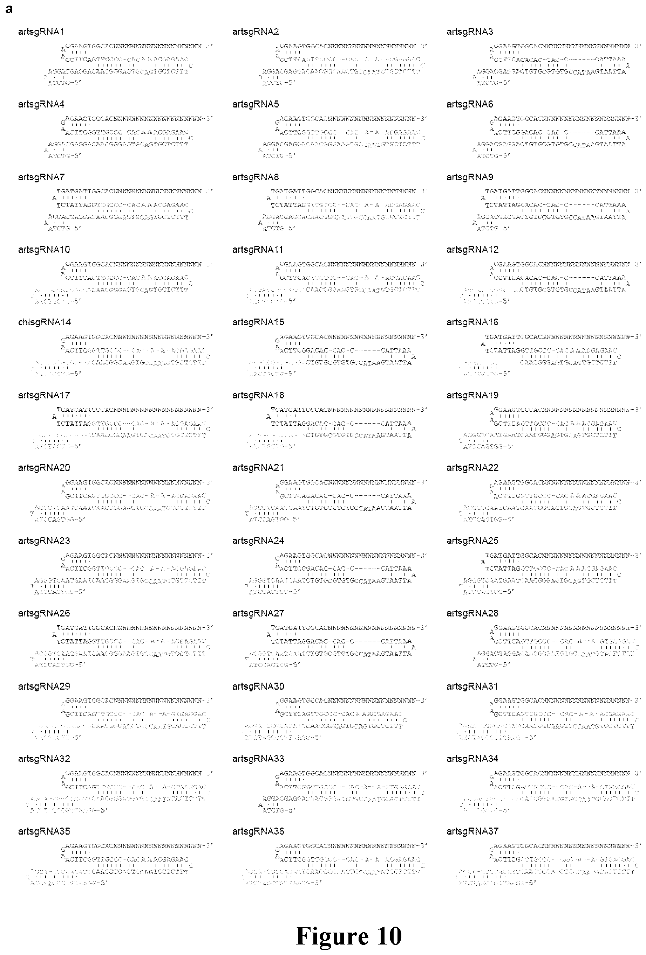

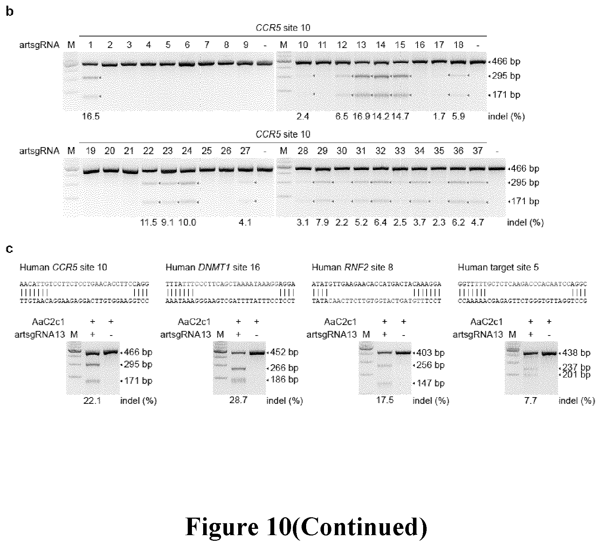

Figure 10 Artificial sgRNAs directed TcC2c1 for genome editing.
   (a) Schematic illustrating the secondary structures of the 36 artificial sgRNA (artsgRNA) scaffolds (scaffold: 1 - 12 and 14 - 37). (b) T7EI assay results indicating the genome targeting activity of TcC2c1 directed by artsgRNAs in the human 293T cells. Red triangles indicate the cleaved bands. (c) T7EI assay results indicating the simultaneous multiplex genome targeting mediated by AaC2c1 combined with artsgRNA13s in human 293T cells.

### Detailed Description of the Invention

### 1. Definition

In the present invention, the scientific and technical terms used herein have the meaning as commonly understood by a person skilled in the art unless otherwise specified. Also, the protein and nucleic acid chemistry, molecular biology, cell and tissue culture, microbiology, immunology related terms, and laboratory procedures used herein are terms and routine steps that are widely used in the corresponding field. For example, standard recombinant DNA and molecular cloning techniques used in the present invention are well known to those skilled in the art and are more fully described in the following document: Sambrook, J., Fritsch, E.F. and Maniatis, T., Molecular Cloning: A Laboratory Manual; Cold Spring Harbor Laboratory Press: Cold Spring Harbor, 1989 (hereinafter referred to as "Sambrook"). In the meantime, in order to better understand the present invention, definitions and explanations of related terms are provided below.

In one aspect, the present invention provides a genome editing system for site-directed modification of a target sequence in the genome of a cell, comprising at least one of the following i) to v):
i) a C2c1 protein or variant thereof, and a guide RNA;
ii) an expression construct comprising a nucleotide sequence encoding a C2c1 protein or variant thereof, and a guide RNA;
iii) a C2c1 protein or variant thereof, and an expression construct comprising a nucleotide sequence encoding a guide RNA;
iv) an expression construct comprising a nucleotide sequence encoding a C2c1 protein or variant thereof, and an expression construct comprising a nucleotide sequence encoding a guide RNA;
v) an expression construct comprising a nucleotide sequence encoding a C2c1 protein or variant thereof and a nucleotide sequence encoding a guide RNA;
wherein the guide RNA is capable of forming complex with the C2c1 protein or variant thereof and targeting the C2c1 protein or variant thereof to the target sequence in the genome of the cell. In some embodiments, the targeting results in substitution, deletion and/or addition of one or more nucleotides in the target sequence.

"Genome" as used herein encompasses not only chromosomal DNA present in the nucleus, but also organellar DNA present in the subcellular components (e.g., mitochondria, plastids) of the cell.

"C2c1 nuclease", "C2c1 protein" and "C2c1" are used interchangeably herein and refer to an RNA-directed nuclease comprising a C2c1 protein or a fragment thereof. C2c1 has a guide RNA-mediated DNA binding activity and DNA cleavage activity, and can target and cleave DNA target sequences to form DNA double-strand breaks (DSBs) under the guidance of a guide RNA. DSB can activate the intracellular intrinsic repair mechanism, non-homologous end joining (NHEJ) and homologous recombination (HR), to repair DNA damage in cells. During the repair process, the specific DNA sequence is subjected to site-directed editing.

In some embodiments, the C2c1 protein is an AaC2c1 protein derived from *Alicyclobacillus acidiphilus,* an AkC2c1 protein derived from *Alicyclobacillus kakegawensis,* an AmC2c1 protein derived from *Alicyclobacillus macrosporangiidus,* a BhC2c1 protein derived from *Bacillus hisashii,* a BsC2c1 protein derived from the genus *Bacillus,* a Bs3C2c1 protein derived from the genus Bacillus, a DiC2c1 protein derived from *Desulfovibrio inopinatus,* a LsC2c1 protein derived from *Laceyella sediminis,* a SbC2c1 protein derived from *Spirochaetes bacterium,* a TcC2c1 protein derived from *Tuberibacillus calidus.*

For example, the C2c1 protein is the AaC2c1 protein derived from *Alicyclobacillus acidiphilus* NBRC 100859, the AkC2c1 protein derived from *Alicyclobacillus kakegawensis* NBRC 103104, the AmC2c1 protein derived from *Alicyclobacillus macrosporangiidus* strain DSM 17980, the BhC2c1 protein derived from *Bacillus hisashii* strain C4, and the BsC2c1 protein derived from genus *Bacillus* NSP2.1, the Bs3C2c1 protein derived from *Bacillus* genus V3-13 contig _40, the DiC2c1 protein derived from *Desulfovibrio inopinatus* DSM 10711, the LsC2c1 protein derived from *Laceyella sediminis* strain RHA1, the SbC2c1 protein derived from *Spirochaetes bacterium* GWB1_27_13, the TcC2c1 protein derived from *Tuberibacillus calidus* DSM 17572.

In some embodiments of the invention, the C2c1 protein is a C2c1 protein whose natural locus does not have a CRISPR array. In some embodiments, the C2c1 protein whose natural locus does not have a CRISPR array is a DiC2c1 or TcC2c1 protein.

In some embodiments, the C2c1 protein comprises the amino acid sequence set forth in any one of SEQ ID NOs:1-10. For example, the AaC2c1, AkC2c1, AmC2c1, BhC2c1, BsC2c1, Bs3C2c1, DiC2c1, LsC2c1, SbC2c1, and TcC2c1 proteins comprise the amino acid sequences set forth in SEQ ID NOs: 1-10, respectively.

In some embodiments, the variant of the C2c1 protein comprises an amino acid sequence having at least 80%, at least 85%, at least 90%, at least 95%, at least 96%, at least 97%, at least 98%, at least 99% sequence identity with a wild-type C2c1 protein (e.g., wild-type AaC2c1, AkC2c1, AmC2c1, BhC2c1, BsC2c1, Bs3C2c1, DiC2c1, LsC2c1, SbC2c1, or TcC2c1 protein), respectively, and has genomic editing and/or targeting activity of a wild-type C2c1 protein (e.g., wild-type AaC2c1, AkC2c1, AmC2c1, BhC2c1, BsC2c1, Bs3C2c1, DiC2c1, LsC2c1, SbC2c1, TcC2c1 protein), respectively.

In some embodiments, the variant of the C2c1 protein comprises an amino acid sequence in which one or more amino acid residues are substituted, deleted or added relative to a wild-type C2c1 protein (e.g., wild-type AaC2c1, AkC2c1, AmC2c1, BhC2c1, BsC2c1, Bs3C2c1, DiC2c1, LsC2c1, SbC2c1, TcC2c1 protein), respectively, and has genome editing and/or targeting activity of wild-type C2c1 proteins (e.g., wild-type AaC2c1, AkC2c1, AmC2c1, BhC2c1, BsC2c1, Bs3C2c1, DiC2c1, LsC2c1, SbC2c1, TcC2c1 protein). For example, the variant of the C2c1 protein comprises an amino acid sequence in which 1, 2, 3, 4, 5, 6, 7, 8, 9, 10 amino acid residue(s) are substituted, deleted or added relative to a wild-type C2c1 protein (e.g., wild-type AaC2c1, AkC2c1, AmC2c1, BhC2c1, BsC2c1, Bs3C2c1, DiC2c1, LsC2c1, SbC2c1, TcC2c1 proteins), respectively. In some embodiments, the amino acid substitution is a conservative substitution.

"Polypeptide," "peptide," and "protein" are used interchangeably in the present invention to refer to a polymer of amino acid residues. The terms apply to an amino acid polymer in which one or more amino acid residues is artificial chemical analogue of corresponding naturally occurring amino acid(s), as well as to a naturally occurring amino acid polymer. The terms "polypeptide," "peptide," "amino acid sequence," and "protein" may also include modified forms including, but not limited to, glycosylation, lipid ligation, sulfation, γ carboxylation of glutamic acid residues, and ADP-ribosylation.

Sequence "identity" has recognized meaning in the art, and the percentage of sequence identity between two nucleic acids or polypeptide molecules or regions can be calculated using the disclosed techniques. Sequence identity can be measured along the entire length of a polynucleotide or polypeptide or along a region of the molecule. (See, for example, Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991)_{∘} Although there are many methods for measuring the identity between two polynucleotides or polypeptides, the term "identity" is well known to the skilled person (Carrillo, H. & Lipman, D., SIAM J Applied Math 48: 1073 (1988)).

Suitable conservative amino acid substitution in peptides or proteins are known to those skilled in the art and can generally be carried out without altering the biological activity of the resulting molecule. In general, one skilled in the art recognizes that a single amino acid substitution in a non-essential region of a polypeptide does not substantially alter biological activity (See, for example, Watson et al., Molecular Biology of the Gene, 4th Edition, 1987, The Benjamin/Cummings Pub. co., p.224).

In some embodiments, the variant of the C2c1 protein encompasses a nuclease-dead C2c1 protein (dC2c1). The nuclease-dead C2c1 protein refers to a C2c1 protein that retains the RNA-mediated DNA-binding activity but does not have DNA cleavage activity. In some embodiments, the nuclease-dead C2c1 protein encompasses a C2c1 nickase that cleaves only one strand of double-stranded target DNA.

In some embodiments, the variant of the C2c1 protein is a fusion protein of dC2c1 and a deaminase. For example, dC2c1 and deaminase in the fusion protein can be linked by a linker such as a peptide linker.

As used herein, "deaminase" refers to an enzyme that catalyzes a deamination reaction. In some embodiments of the invention, the deaminase refers to a cytosine deaminase capable of accepting single-stranded DNA as a substrate and capable of catalyzing the deamination of cytidine or deoxycytidine to uracil or deoxyuracil, respectively. In some embodiments of the invention, the deaminase refers to adenine deaminase capable of accepting single-stranded DNA as a substrate and capable of catalyzing adenosine or deoxyadenosine (A) into inosine (I). Base editing in the target DNA sequence, such as C to T conversion or A to G conversion, can be achieved by using a fusion protein of a C2c1 variant and a deaminase.

In some embodiments of the present invention, the C2c1 protein or variant thereof of the present invention further comprises a nuclear localization sequence (NLS). In general, one or more NLSs in the C2c1 protein or variant thereof should be of sufficient strength to drive the C2c1 protein or variant thereof in the nucleus of a plant cell to achieve an amount accumulation of base editing function. In general, the intensity of nuclear localization activity is determined by the number, location, one or more specific NLSs used of the NLS in the C2c1 protein or variant thereof, or a combination of these factors.

In some embodiments of the present invention, the NLS of the C2c1 protein or variant thereof of the present invention may be located at the N-terminus and/or C-terminus. In some embodiments, the C2c1 protein or variant thereof comprises about 1, 2, 3, 4, 5, 6, 7, 8, 9, 10 or more NLSs. In some embodiments, the C2c1 protein or variant thereof comprises about 1, 2, 3, 4, 5, 6, 7, 8, 9, 10 or more NLS at or near the N-terminus. In some embodiments, the C2c1 protein or variant thereof comprises about 1, 2, 3, 4, 5, 6, 7, 8, 9, 10 or more NLSs at or near the C-terminus. In some embodiments, the C2c1 protein or variant thereof comprises a combination of these, such as comprises one or more NLSs at the N-terminus and one or more NLSs at the C-terminus. When there is more than one NLS, each can be selected to be independent of other NLSs. In some preferred embodiments of the present invention, the C2c1 protein or variant thereof comprises two NLSs, for example, the two NLSs are located at the N-terminus and the C-terminus, respectively.

In general, NLS consists of one or more short sequences of positively charged lysine or arginine exposed on the surface of the protein, but other types of NLS are also known. Non-limiting examples of NLS include: KKRKV, PKKKRKV, or SGGSPKKKRKV.

Furthermore, depending on the location of the DNA to be edited, the C2c1 protein or variant thereof of the present invention may also include other localization sequences, such as cytoplasmic localization sequences, chloroplast localization sequences, mitochondrial localization sequences, and the like.

In some embodiments of the invention, the target sequence is 18-35 nucleotides in length, preferably 20 nucleotides. In some embodiments of the invention, the 5'-end flanking of the target sequence is a protospacer adjacent motif (PAM) sequence selected from 5'TTTN-3', 5'ATTN-3', 5'GTTN-3', 5'CTTN-3', 5'TTC-3', 5'TTG-3', 5'TTA-3', 5'TTT-3', 5'TAN-3', 5'TGN-3', 5'TCN-3' and 5'ATC-3', wherein N is selected from A, G, C and T.

In the present invention, the target sequence to be modified may be located at any location in the genome, for example, in a functional gene such as a protein-encoding gene, or may be, for example, located in a gene expression regulatory region such as a promoter region or an enhancer region, thereby the gene functional modification or gene expression modification can be achieved. The substitution, deletion and/or addition in the target sequence of the genome can be detected by T7EI, PCR/RE or sequencing methods.

"guide RNA" and "gRNA" can be used interchangeably herein, typically composed of crRNA and tracrRNA molecules that are partially complementary to each other to form a complex, wherein the crRNA comprises a sequence that is sufficiently identical to the target sequence to hybridize to the complement of the target sequence and direct the CRISPR complex (C2c1+crRNA+tracrRNA) to sequence specifically bind to the target sequence. However, a single guide RNA (sgRNA) containing both crRNA and tracrRNA characteristics can be designed and used.

In some embodiments of the invention, the guide RNA is sgRNA. In some particular embodiments, the sgRNA is encoded by a nucleotide sequence selected from the group consisting of: 
5'-GTCTAAAGGACAGAATTTTTCAACGGGTGTGCCAATGGCCACTTTCCAGGT
GGCAAAGCCCGTTGAACTTCTCAAAAAGAACGCTCGCTCAGTGTTCTGACGTCGG
ATCACTGAGCGAGCGATCTGAGAAGTGGCAC-Nx-3'(AasgRNA) ;
5'-tcgtctataGGACGGCGAGGACAACGGGAAGTGCCAATGTGCTCTTTCCAAGAG
CAAACACCCCGTTGGCTTCAAGATGACCGCTCGCTCAGCGATCTGACAACGGATC
GCTGAGCGAGCGGTCTGAGAAGTGGCAC-Nx-3'(AksgRNA1);
5'-ggaattgccgatctaTAGGACGGCAGATTCAACGGGATGTGCCAATGCACTCTTTCC
AGGAGTGAACACCCCGTTGGCTTCAACATGATCGCCCGCTCAACGGTCCGATGTC
GGATCGTTGAGCGGGCGATCTGAGAAGTGGCAC-Nx-3' (AmsgRNA1) ;
5'-GAGGTTCTGTCTTTTGGTCAGGACAACCGTCTAGCTATAAGTGCTGCAGGG
TGTGAGAAACTCCTATTGCTGGACGATGTCTCTTTTATTTCTTTTTCTTGGATGTCC
AAGAAAAAAGAAATGATACGAGGCATTAGCAC-Nx-3'(BhsgRNA) ;
5'-CCATAAGTCGACTTACATATCCGTGCGTGTGCATTATGGGCCCATCCACAGG
TCTATTCCCACGGATAATCACGACTTTCCACTAAGCTTTCGAATGTTCGAAAGCTTA
 
GTGGAAAGCTTCGTGGTTAGCAC-Nx-3' (BssgRNA) ;
5'-GGTGACCTATAGGGTCAATGAATCTGTGCGTGTGCCATAAGTAATTAAAAAT
TACCCACCACAGGATTATCTTATTTCTGCTAAGTGTTTAGTTGCCTGAATACTTAGC
AGAAATAATGATGATTGGCAC-Nx-3' (Bs3sgRNA) ;
5'-GGCAAAGAATACTGTGCGTGTGCTAAGGATGGAAAAAATCCATTCAACCAC
AGGATTACATTATTTATCTAATCACTTAAATCTTTAAGTGATTAGATGAATTAAATGT
GATTAGCAC-Nx-3' (LssgRNA) ; or
5'-GTCTTAGGGTATATCCCAAATTTGTCTTAGTATGTGCATTGCTTACAGCGACA
ACTAAGGTTTGTTTATCTTTTTTTACATTGTAAGATGTTTTACATTATAAAAAGAAG
ATAATCTTATTGCAC-Nx-3' (SbsgRNA) ;
 wherein Nx represents nucleotide sequence that consists of X consecutive nucleotides(spacer sequence), N is independently selected from A, G, C and T; X is an integer of 18 ≤ X ≤ 35, preferably, X=20. In some embodiments, the sequence Nx (spacer sequence) is capable of specifically hybridizing to the complement of the target sequence. The sequence other than Nx in the sgRNA is a scaffold sequence of sgRNA. In some embodiments, the sgRNA comprises a scaffold sequence encoded by the nucleotide sequence of any one of SEQ ID NOs: 31-38.

The present inventors have surprisingly found that the C2c1 protein and the guide RNA in different C2c1 systems can be used interchangeably, thereby enabling the artificial design of universal guide RNAs.

Thus, in some embodiments, the invention provides an artificial sgRNA, which selected from the nucleotide sequence encoding: 
5’-GGTCTAAAGGACAGAATTTTTCAACGGGTGTGCCAATGGCCACTTTCCAGG
TGGCAAAGCCCGTTGAACTTCAAGCGAAGTGGCAC-Nx-3’(artsgRNA1);
5’-GGTCTAAAGGACAGAAGACAACGGGAAGTGCCAATGTGCTCTTTCCAAGA
GCAAACACCCCGTTGACTTCAAGCGAAGTGGCAC-Nx-3’(artsgRNA2);
5’-GGTCTAAAGGACAGAAAATCTGTGCGTGTGCCATAAGTAATTAAAAATTAC
CCACCACAGACTTCAAGCGAAGTGGCAC-Nx-3’(artsgRNA3);
5’-GGTCGTCTATAGGACGGCGAGTTTTTCAACGGGTGTGCCAATGGCCACTTT
CCAGGTGGCAAAGCCCGTTGAACTTCAAGCGAAGTGGCAC-Nx-3’(artsgRNA4);
5’-GGTCGTCTATAGGACGGCGAGGACAACGGGAAGTGCCAATGTGCTCTTTCC
AAGAGCAAACACCCCGTTGACTTCAAGCGAAGTGGCAC-Nx-3’(artsgRNA5);
5’-GGTCGTCTATAGGACGGCGAGAATCTGTGCGTGTGCCATAAGTAATTAAAA
ATTACCCACCACAGACTTCAAGCGAAGTGGCAC-Nx-3’(artsgRNA6);
5’-GGTGACCTATAGGGTCAATGTTTTTCAACGGGTGTGCCAATGGCCACTTTCC
AGGTGGCAAAGCCCGTTGAACTTCAAGCGAAGTGGCAC-Nx-3’(artsgRNA7);
 
5’-GGTGACCTATAGGGTCAATGGACAACGGAAGTGCCAATGTGCTCTTTCCA
AGAGCAAACACCCCGTTGACTTCAAGCGAAGTGGCAC-Nx-3’(artsgRNA8);
5’-GGTGACCTATAGGGTCAATGAATCTGTGCGTGTGCCATAAGTAATTAAAAAT
TACCCACCACAGACTTCAAGCGAAGTGGCAC-Nx-3’(artsgRNA9);
5’-GGTCTAAAGGACAGAATTTTCAACGGGTGTGCCAATGGCCACTTTCCAGG
TGGCAAAGCCCGTTGAGCTTCAAAGAAGTGGCAC-Nx-3’(artsgRNA10);
5’-GGTCTAAAGGACAGAAGACAACGGAAGTGCCAATGTGCTCTTTCCAAGA
GCAAACACCCCGTTGGCTTCAAAGAAGTGGCAC-Nx-3’(artsgRNA11);
5’-GGTCTAAAGGACAGAAAATCTGTGCGTGTGCCATAAGTAATTAAAAATTAC
CCACCACAGGCTTCAAAGAAGTGGCAC-Nx-3’(artsgRNA12);
5’-GGTCGTCTATAGGACGGCGAGTTTTCAACGGGTGTGCCAATGGCCACTTT
CCAGGTGGCAAAGCCCGTTGAGCTTCAAAGAAGTGGCAC-Nx-3’(artsgRNA13);
5’-GGTCGTCTATAGGACGGCGAGGACAACGGAAGTGCCAATGTGCTCTTTCC
AAGAGCAAACACCCCGTTGGCTTCAAAGAAGTGGCAC-Nx-3’(artsgRNA14);
5’-GGTCGTCTATAGGACGGCGAGAATCTGTGCGTGTGCCATAAGTAATTAAAA
ATTACCCACCACAGGCTTCAAAGAAGTGGCAC-Nx-3’(artsgRNA15);
5’-GGTGACCTATAGGGTCAATGTTTTCAACGGGTGTGCCAATGGCCACTTTCC
AGGTGGCAAAGCCCGTTGAGCTTCAAAGAAGTGGCAC-Nx-3’(artsgRNA16);
5’-GGTGACCTATAGGGTCAATGGACAACGGAAGTGCCAATGTGCTCTTTCCA
AGAGCAAACACCCCGTTGGCTTCAAAGAAGTGGCAC-Nx-3’(artsgRNA17);
5’-GGTGACCTATAGGGTCAATGAATCTGTGCGTGTGCCATAAGTAATTAAAAAT
TACCCACCACAGGCTTCAAAGAAGTGGCAC-Nx-3’(artsgRNA18);
5’-GGTCTAAAGGACAGAATTTTCAACGGGTGTGCCAATGGCCACTTTCCAGG
TGGCAAAGCCCGTTGAGATTATCTATGATGATTGGCAC-Nx-3’(artsgRNA19);
5’-GGTCTAAAGGACAGAAGACAACGGAAGTGCCAATGTGCTCTTTCCAAGA
GCAAACACCCCGTTGGATTATCTATGATGATTGGCAC-Nx-3’(artsgRNA20) ;
5’-GGTCTAAAGGACAGAAAATCTGTGCGTGTGCCATAAGTAATTAAAAATTAC
CCACCACAGGATTATCTATGATGATTGGCAC-Nx-3’(artsgRNA21) ;
5’-GGTCGTCTATAGGACGGCGAGTTTTCAACGGGTGTGCCAATGGCCACTTT
CCAGGTGGCAAAGCCCGTTGAGATTATCTATGATGATTGGCAC-Nx-3’(artsgRNA22) ;
5’-GGTCGTCTATAGGACGGCGAGGACAACGGAAGTGCCAATGTGCTCTTTCC
AAGAGCAAACACCCCGTTGGATTATCTATGATGATTGGCAC-Nx-3’(artsgRNA23) ;
5’-GGTCGTCTATAGGACGGCGAGAATCTGTGCGTGTGCCATAAGTAATTAAAA
ATTACCCACCACAGGATTATCTATGATGATTGGCAC-Nx-3’(artsgRNA24) ;
5’-GGTGACCTATAGGGTCAATGTTTTCAACGGGTGTGCCAATGGCCACTTTCC
AGGTGGCAAAGCCCGTTGAGATTATCTATGATGATTGGCAC-Nx-3’(artsgRNA25) ;
 
5'-GGTGACCTATAGGGTCAATGGACAACGGGAAGTGCCAATGTGCTCTTTCCA
AGAGCAAACACCCCGTTGGATTATCTATGATGATTGGCAC-Nₓ-3'(artsgRNA26) ;
5'-GGTGACCTATAGGGTCAATGAATCTGTGCGTGTGCCATAAGTAATTAAAAAT
TACCCACCACAGGATTATCTATGATGATTGGCAC-Nₓ-3'(artsgRNA27) ;
5'-GGTCTAAAGGACAGAACAACGGGATGTGCCAATGCACTCTTTCCAGGAGT
GAACACCCCGTTGACTTCAAGCGAAGTGGCAC-Nₓ-3'(artsgRNA28) ;
5'-GGTCGTCTATAGGACGGCGAGCAACGGGATGTGCCAATGCACTCTTTCCAG
GAGTGAACACCCCGTTGACTTCAAGCGAAGTGGCAC-Nₓ-3'(artsgRNA29) ;
5'-GGAATTGCCGATCTATAGGACGGCAGATTTTTTCAACGGGTGTGCCAATG
GCCACTTTCCAGGTGGCAAAGCCCGTTGAACTTCAAGCGAAGTGGCAC-Nₓ-3'(artsg
RNA30) ;
5'-GGAATTGCCGATCTATAGGACGGCAGATTGACAACGGGAAGTGCCAATGTG
CTCTTTCCAAGAGCAAACACCCCGTTGACTTCAAGCGAAGTGGCAC-Nₓ-3'(artsgRN
A31) ;
5'-GGAATTGCCGATCTATAGGACGGCAGATTCAACGGGATGTGCCAATGCACT
CTTTCCAGGAGTGAACACCCCGTTGACTTCAAGCGAAGTGGCAC-Nₓ-3'(artsgRNA3
2) ;
5'-GGTCTAAAGGACAGAACAACGGGATGTGCCAATGCACTCTTTCCAGGAGT
GAACACCCCGTTGGCTTCAAAGAAGTGGCAC-Nₓ-3'(artsgRNA33) ;
5'-GGTCGTCTATAGGACGGCGAGCAACGGGATGTGCCAATGCACTCTTTCCAG
GAGTGAACACCCCGTTGGCTTCAAAGAAGTGGCAC-Nₓ-3'(artsgRNA34) ;
5'-GGAATTGCCGATCTATAGGACGGCAGATTTTTTCAACGGGTGTGCCAATG
GCCACTTTCCAGGTGGCAAAGCCCGTTGAGCTTCAAAGAAGTGGCAC-Nₓ-3'(artsgR
NA35) ;
5'-GGAATTGCCGATCTATAGGACGGCAGATTGACAACGGGAAGTGCCAATGTG
CTCTTTCCAAGAGCAAACACCCCGTTGGCTTCAAAGAAGTGGCAC-Nₓ-3'(artsgRN3
6A); or
5'-GGAATTGCCGATCTATAGGACGGCAGATTCAACGGGATGTGCCAATGCACT
CTTTCCAGGAGTGAACACCCCGTTGGCTTCAAAGAAGTGGCAC-Nₓ-3'(artsgRNA37)
,
 wherein Nx represents nucleotide sequence that consists of X consecutive nucleotides(spacer sequence), N is independently selected from A, G, C and T; X is an integer of 18 ≤ X ≤ 35, preferably, X=20. In some embodiments, the sequence Nx (spacer sequence) is capable of specifically hybridizing to the complement of the target sequence. The sequence other than Nx in the sgRNA is a scaffold sequence of sgRNA.

In some embodiments, the artificial sgRNA comprises a scaffold sequence encoded by the nucleotide sequence of any one of SEQ ID NOs:39-75.

In some embodiments, the guide RNA in the genome editing system of the invention is an artificial sgRNA of the invention.

To achieve efficient expression in target cells, in some embodiments of the invention, the nucleotide sequence encoding a C2c1 protein or variant thereof is codon optimized for the organism from which the cell to be genome edited is derived.

Codon optimization refers to the replacement of at least one codon (e.g., about or more than about 1, 2, 3, 4, 5, 10, 15, 20, 25, 50 or more codons) of a native sequence by a codon that is used more frequently or most frequently in the gene of the host cell, modifying the nucleic acid sequence while maintaining the native amino acid sequence to enhance expression in the host cell of interest. Different species show specific preferences for certain codons of a particular amino acid. Codon preference (difference in codon usage between organisms) is often associated with the efficiency of translation of messenger RNA (mRNA), which is believed to depend on the nature of the translated codon and the availability of specific transfer RNA (tRNA) molecules. The advantages of selected tRNAs within cells generally reflect the most frequently used codons for peptide synthesis. Therefore, genes can be customized to be best gene expressed in a given organism based on codon optimization. The codon usage table can be easily obtained, for example, in the Codon Usage Database available at www.kazusa.orjp/codon/, and these tables can be adjusted in different ways. See, Nakamura Y. et. al. "Codon usage tabulated from the international DNA sequence databases: status for the year 2000 Nucl.Acids Res, 28: 292 (2000).

The organism from which the cell can be genome-edited by the system of the present invention preferably is eukaryote, including but not limited to mammals such as human, mouse, rat, monkey, dog, pig, sheep, cattle, cat; poultry such as chicken, duck, goose; plants including monocots and dicots, such as rice, corn, wheat, sorghum, barley, soybean, peanut and Arabidopsis thaliana and so on.

In some embodiments of the invention, the nucleotide sequence encoding a C2c1 protein or variant thereof is codon optimized for human. In some embodiments, the codon-optimized nucleotide sequence encoding AaC2c1, AkC2c1, AmC2c1, BhC2c1, BsC2c1, Bs3C2c1, DiC2c1, LsC2c1, SbC2c1, TcC2c1 protein is selected from SEQ ID NOs: 11-20, respectively.

In some embodiments of the present invention, the nucleotide sequence encoding the C2c1 protein or variant thereof and/or the nucleotide sequence encoding the guide RNA is operably linked to an expression regulatory element such as a promoter.

As used in the present invention, "expression construct" refers to a vector such as a recombinant vector that is suitable for expression of a nucleotide sequence of interest in an organism. "Expression" refers to the production of a functional product. For example, expression of a nucleotide sequence may refer to the transcription of a nucleotide sequence (e.g., transcription to produce mRNA or functional RNA) and/or the translation of RNA into a precursor or mature protein. The "expression construct" of the present invention may be a linear nucleic acid fragment, a circular plasmid, a viral vector or, in some embodiments, an RNA that is capable of translation (such as mRNA).

The "expression construct" of the present invention may comprise regulatory sequences and nucleotide sequences of interest from different origins, or regulatory sequences and nucleotide sequences of interest from the same source but arranged in a manner different from that normally occurring in nature.

"Regulatory sequence" and "regulatory element" are used interchangeably to refer to a nucleotide sequence that is located upstream (5' non-coding sequence), middle or downstream (3' non-coding sequence) of a coding sequence and affects the transcription, RNA processing or stability or translation of the relevant coding sequence. Regulatory sequences may include, but are not limited to, promoters, translation leaders, introns and polyadenylation recognition sequences.

"Promoter" refers to a nucleic acid fragment capable of controlling the transcription of another nucleic acid fragment. In some embodiments of the present invention, the promoter is a promoter capable of controlling the transcription of a gene in a cell, whether or not it is derived from the cell. The promoter may be a constitutive promoter or tissue-specific promoter or developmentally-regulated promoter or inducible promoter.

"Constitutive promoter" refers to a promoter that may in general cause the gene to be expressed in most cases in most cell types. "Tissue-specific promoter" and "tissue-preferred promoter" are used interchangeably and mean that they are expressed primarily but not necessarily exclusively in one tissue or organ, but also in a specific cell or cell type. "Developmentally-regulated promoter" refers to a promoter whose activity is dictated by developmental events. "Inducible promoter" selectively expresses operably-linked DNA sequences in response to an endogenous or exogenous stimulus (environment, hormones, chemical signals, etc.).

As used herein, the term "operably linked" refers to the linkage of a regulatory element (e.g., but not limited to, a promoter sequence, a transcription termination sequence, etc.) to a nucleic acid sequence (e.g., a coding sequence or an open reading frame) such that transcription of the nucleotide sequence is controlled and regulated by the transcriptional regulatory element. Techniques for operably linking regulatory element regions to nucleic acid molecules are known in the art.

Examples of promoters that can be used in the present invention include, but are not limited to, the polymerase (pol) I, pol II or pol III promoters. Examples of the pol I promoter include the gallus RNA pol I promoter. Examples of the pol II promoters include, but are not limited to, the immediate-early cytomegalovirus (CMV) promoter, the Rous sarcoma virus long terminal repeat (RSV-LTR) promoter, and the immediate-early simian virus 40 (SV40) promoter. Examples of pol III promoters include the U6 and H1 promoters. An inducible promoter such as a metallothionein promoter can be used. Other examples of promoters include the T7 phage promoter, the T3 phage promoter, the β-galactosidase promoter, and the Sp6 phage promoter, and the like. Promoters that can be used in plants include, but are not limited to, cauliflower mosaic virus 35S promoter, maize Ubi-1 promoter, wheat U6 promoter, rice U3 promoter, maize U3 promoter, rice actin promoter.

The cell that can be edited by the method of the present invention preferably is an eukaryotic cell, including but not limited to a mammalian cell such as a cell of human, mouse, rat, monkey, dog, pig, sheep, cattle, cat; a cell of poultry such as chicken, duck, goose; a cell of plants including monocots and dicots, such as rice, corn, wheat, sorghum, barley, soybean, peanut and Arabidopsis thaliana and so on. In some embodiments of the invention, the cell is a eukaryotic cell, preferably a mammalian cell, more preferably a human cell.

In another aspect, the present invention provides a method of modifying the target sequence in cell genome, comprising introducing the genome editing system of the invention into the cell, whereby the guide RNA targets the C2c1 protein or variant thereof to a target sequence in the genome of the cell. In some embodiments, the targeting results in one or more nucleotides being substituted, deleted and/or added in the target sequence.

"Introduction" of a nucleic acid molecule (e.g., plasmid, linear nucleic acid fragment, RNA, etc.) or protein of the invention into a cell means that the nucleic acid or protein is used to transform a cell such that the nucleic acid or protein is capable of functioning in the cell. As used in the present invention, "transformation" includes both stable and transient transformations. "Stable transformation" refers to the introduction of exogenous nucleotide sequence into the genome, resulting in the stable inheritance of foreign sequence. Once stably transformed, the exogenous nucleic acid sequence is stably integrated into the genome of the organism and any of its successive generations. "Transient transformation" refers to the introduction of a nucleic acid molecule or protein into a cell, executing its function without the stable inheritance of an exogenous sequence. In transient transformation, the exogenous nucleic acid sequence is not integrated into the genome.

Methods that can be used to introduce the genome editing system of the present invention into a cell include, but are not limited to, calcium phosphate transfection, protoplast fusion, electroporation, lipofection, microinjection, viral infection (e.g., baculovirus, vaccinia virus, adenovirus, adeno-associated virus, lentivirus and other viruses), gene gun method, PEG-mediated protoplast transformation, Agrobacterium-mediated transformation.

In some embodiments, the method of the invention is performed in vitro. For example, the cell is an isolated cell. In some embodiments, the cell is a CAR-T cell. In some embodiments, the cell is an induced embryonic stem cell.

In other embodiments, the method of the invention may also be performed in vivo. For example, the cell is a cell within an organism, and the system of the present invention can be introduced into the cell in vivo by, for example, a virus-mediated method. For example, the cell can be a tumor cell within a patient.

In another aspect, the present invention provides a method of producing a genetically modified cell, comprising introducing the genome editing system of the present invention into a cell, whereby the guide RNA targets the C2c1 protein or variant thereof to a target sequence in the genome of the cell. In some embodiments, the targeting results in one or more nucleotides being substituted, deleted and/or added in the target sequence.

In another aspect, the invention also provides a genetically modified organism comprising a genetically modified cell produced by the methods of the invention or a progeny cell thereof.

As used herein, "organism" includes any organism that is suitable for genome editing, eukaryotes are preferred. Examples of the organism include, but are not limited to, mammals such as human, mouse, rat, monkey, dog, pig, sheep, cattle, cat; poultry such as chicken, duck, goose; plants including monocots and dicots such as rice, corn, wheat, sorghum, barley, soybean, peanut, Arabidopsis and the like. In some embodiments of the invention, the organism is eukaryote, preferably a mammal, and more preferably a human.

A "genetically modified organism" or "genetically modified cell" includes the organism or the cell which comprises within its genome an exogenous polynucleotide or a modified gene or modified expression regulatory sequence. For example, the exogenous polynucleotide is stably integrated within the genome of the organism or the cell such that the polynucleotide is passed on to successive generations. The exogenous polynucleotide may be integrated into the genome alone or as part of a recombinant DNA construct. The modified gene or modified expression regulatory sequence means that, in the genome of the organism or the cell, said sequence comprises one or more nucleotide substitution, deletion, or addition. "Exogenous" in reference to a sequence means a sequence from a foreign species, or refers to a sequence in which significant changes in composition and/or locus occur from its native form through deliberate human intervention if from the same species.

In another aspect, the invention provides a gene expression regulatory system based on a nuclease-dead C2c1 protein of the invention. This system, although not changing the sequence of the target gene, is also defined as a genome editing system within the scope herein.

In some embodiments, the gene expression regulation system is a gene suppressing or silencing system, comprising one of the following:
i) a nuclease-dead C2c1 protein or a fusion protein of the nuclease-dead C2c1 protein and a transcriptional repressor protein, and a guide RNA;
ii) an expression construct comprising a nucleotide sequence encoding a nuclease-dead C2c1 protein or a fusion protein of the nuclease-dead C2c1 protein and a transcriptional repressor protein, and a guide RNA;
iii) a nuclease-dead C2c1 protein or a fusion protein of the nuclease-dead C2c1 protein and a transcriptional repressor protein, and an expression construct comprising a nucleotide sequence encoding a guide RNA;
iv) an expression construct comprising a nucleotide sequence encoding a nuclease-dead C2c1 protein or a fusion protein of the nuclease-dead C2c1 protein and a transcriptional repressor protein, and an expression construct comprising a nucleotide sequence encoding a guide RNA; or
v) an expression construct comprising a nucleotide sequence encoding a nuclease-dead C2c1 protein or a fusion protein of the nuclease-dead C2c1 protein and a transcriptional repressor protein and a nucleotide sequence encoding a guide RNA.

The definition of the nuclease-dead C2c1 protein or the guide RNA is as described above. Selection of the transcriptional repressor protein is within the skill of those ordinary people in the art.

As used herein, gene suppression or silencing refers to the down-regulation or elimination of gene expression, preferably at the transcriptional level.

However, the gene expression regulatory system of the present invention can also use a fusion protein of a nuclease-dead C2c1 protein and a transcriptional activator protein. In this case, the gene expression regulatory system is a gene expression activation system. For example, the gene expression activation system of the present invention may comprise one of the following:
i) a fusion protein of a nuclease-dead C2c1 protein and a transcriptional activator protein, and a guide RNA;
ii) an expression construct comprising a nucleotide sequence encoding a fusion protein of a nuclease-dead C2c1 protein and a transcriptional activator protein, and a guide RNA;
iii) a fusion protein of a nuclease-dead C2c1 protein and a transcriptional activator protein, and an expression construct comprising a nucleotide sequence encoding a guide RNA;
iv) an expression construct comprising a nucleotide sequence encoding a fusion protein of a nuclease-dead C2c1 protein and a transcriptional activator protein, and an expression construct comprising a nucleotide sequence encoding a guide RNA; or
v) an expression construct comprising a nucleotide sequence encoding a fusion protein of a nuclease-dead C2c1 protein and a transcriptional activator protein and a nucleotide sequence encoding a guide RNA.

The definition of the nuclease-dead C2c1 protein or the guide RNA is as described above. Selection of the transcriptional activator protein is within the skill of those ordinary people in the art.

As used herein, gene activation refers to up-regulation of gene expression levels, preferably at the transcriptional level.

In another aspect, the invention also encompasses the use of the genome editing system of the invention in the treatment of diseases.

By modifying a disease-related gene by the genome editing system of the present invention, it is possible to achieve up-regulation, down-regulation, inactivation, activation, or mutation correction of the disease-related gene, thereby achieving prevention and/or treatment of the disease. For example, in the present invention, the target sequence may be located in the protein coding region of the disease-related gene, or may be, for example, located in a gene expression regulatory region such as a promoter region or an enhancer region, thereby enabling functional modification of the disease-related gene or modification of the expression of the disease-related gene.

A "disease-related" gene refers to any gene that produces a transcriptional or translational product at an abnormal level or in an abnormal form in a cell derived from a disease-affected tissue as compared to a tissue or cell of non-disease control. When altered expression is related with the appearance and/or progression of a disease, it may be a gene that is expressed at an abnormally high level or it may be a gene that is expressed at an abnormally low level. A disease-related gene also refers to a gene having one or more mutations or a genetic variation that is directly responsible for or has genetic linkage with one or more genes responsible for the etiology of the disease. The transcribed or translated product may be known or unknown and may be at normal or abnormal levels.

Accordingly, in another aspect, the invention also provides a method of treating a disease in a subject in need thereof, comprising delivering to the subject an effective amount of a genome editing system of the invention to modify a gene related to the disease.

In another aspect, the invention also provides the use of a genome editing system of the invention for the preparation of a pharmaceutical composition for treating a disease in a subject in need thereof, wherein the genome editing system is for modifying a gene related to the disease.

In another aspect, the invention also provides a pharmaceutical composition for treating a disease in a subject in need thereof, comprising a genome editing system of the invention and a pharmaceutically acceptable carrier, wherein the genome editing system is for modifying a gene related to the disease.

In some embodiments, the subject is a mammal, such as a human.

Examples of such diseases include, but are not limited to, tumors, inflammation, Parkinson's disease, cardiovascular disease, Alzheimer's disease, autism, drug addiction, age-related macular degeneration, schizophrenia, hereditary diseases, and the like.

In another aspect, the invention also includes a kit for use in the methods of the invention, the kit comprising the genome editing system of the invention, and an instruction. The kits generally include a label indicating the intended use and/or method of use of the contents in the kit. The term label includes any written or recorded material provided on or with the kit or otherwise provided with the kit.

### Example

In order to facilitate the understanding of the present invention, the present invention will be described more fully hereinafter with reference to the specific examples and the accompanying drawings. Preferred embodiments of the invention are shown in the drawings. However, the invention may be embodied in many different forms and is not limited to the embodiments described herein. Rather, these embodiments are provided so that the present disclosure will be more fully understood.

### Materials and Methods

### 1. DNA manipulations.

DNA manipulation including DNA preparation, digestion, ligation, amplification, synthesis, purification, agarose gel electrophoresis, etc. were conducted according to *Molecular Cloning: A Laboratory Manual* with some modifications. Briefly, targeting chimeric single guide RNA (sgRNA) scaffolds for cell transfection assay were constructed by ligation annealed oligonucleotides (oligos) (Table 1) into BsaI-digested pUC19-U6-sgRNA (SEQ ID NO:23-30) vectors.

**Table 1 Target sequences of human genomic targets.**

| Gene | Protospacer ID | Protospacer sequences (5' - 3') | 5'PAM | Strand |
|---|---|---|---|---|
| CCR5 | CCR5 site 10 | TCCTTCTCCTGAACACCTTC | TTG | + |
| | CCR5 site 28 | TTTGGCCTGAATAATTGCAG | TTC | + |
| DNMT1 | DNMT1 site 16 | CCCTTCAGCTAAAATAAAGG | TTT | + |
| RNF2 | RNF2 site 8 | TAGTCATGGTGTTCTTCAAC | TTG | - |
| - | Site 5 | GCTCTCAAGACCCACAATCC | TTT | + |

### 2. De novo gene synthesis and plasmid construction.

PSI-BLAST program (Altschul, S.F. et al. Nucleic Acids Res 25, 3389-3402 (1997)) was adopted to identify new CRISPR-C2c1 orthologs. Their coding sequences were humanized (Grote, A. et al., Nucleic Acids Res 33, W526-531 (2005)) and oligos for C2c1 gene and sgRNA synthesis were designed using GeneDesign program (Richardson, S.M. et al., Genome Res 16, 550-556 (2006)). Each C2c1 gene was synthesized according to article (Li, G. et al., Methods Mol Biol 1073, 9-17 (2013)). Purified products were assembled into expression vectors via homologous recombination in vitro using NEBuilder^{®} HiFi DNA Assembly Master Mix (NEB). The pCAG-2AeGFP (SEQ ID NO:21) and pUC19-U6 vectors (SEQ ID NO:22) were applied for mammalian expression of C2c1 proteins and sgRNAs, respectively.

### 3. Cell culture and transfection.

Human embryonic kidney 293T cells were cultured in Dulbecco s Modified Eagle' s Medium (DMEM, Gibco) supplemented with 10% fetal bovine serum (FBS, Gibco) and 1% Antibiotic-Antimycotic (Gibco) at 37 °C with 5% CO2 incubation. 293T cells were transfected using Lipofectamine LTX (Invitrogen) following the manufacturer's recommended protocol. For each well of a 48-well plate, a total of 400 ng plasmid (C2c1: sgRNA = 2: 1) were used. Then 48 h following transfection, cells were harvested directly for genomic DNA extraction.

### 4. T7 endonuclease I (T7EI) assay and Sanger sequencing.

Harvested cells were lysed directly with Buffer L (Bimake) supplemented with Protease K and incubated at 55°C for 3 h and inactivated at 95°C for 10 min. Genomic region surrounding the C2c1 target site for each gene was PCR-amplified (Supplementary Table 5). 200 ~ 400 ng PCR products were mixed with ddH2O to a final volume of 10 µL, and subjected to re-annealing process to enable heteroduplex formation according to previous methods. After re-annealing, products were treated with 1/10 volume of NEBufferTM 2.1 and 0.2 µ L T7EI (NEB) at 37 °C for 30 min, and analyzed on 3% agarose gels. Indels were quantitated based on relative band intensities (Cong, L. et al., Science 339, 819-823 (2013)). T7EI assay identified mutated products were subjected to be cloned into TA cloning vector and transformed into competent E. coli strain (Transgen Biotech). After overnight culture, colonies were randomly picked out and sequenced.

### Example 1. New C2clproteins idenfication

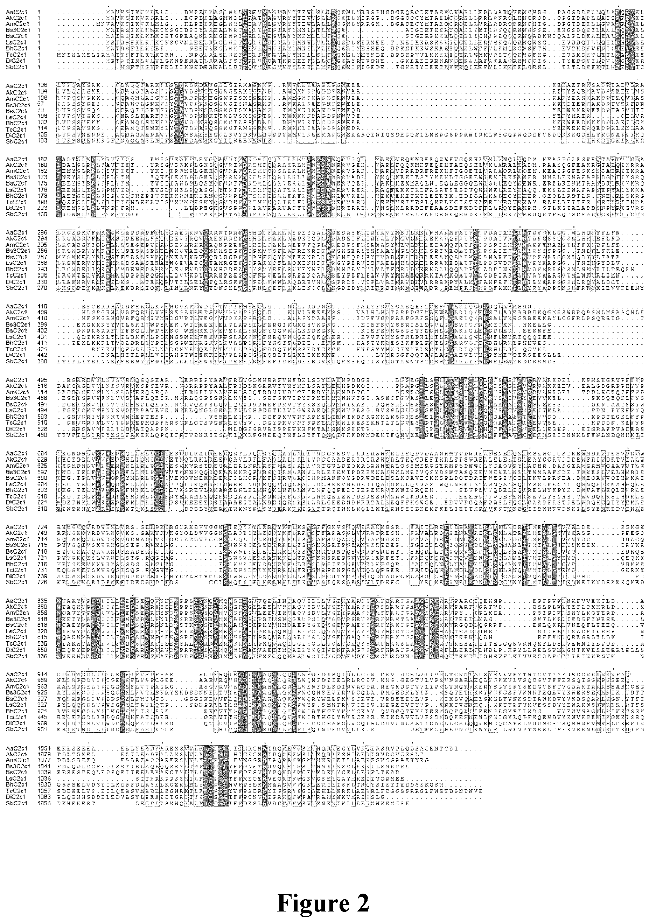

Six representative C2c1-family proteins from diverse bacteria as well as four previously reported C2c1 orthologs were selected, and de novo synthesized to conduct genome editing in human embryonic kidney 293T cells (Figs. 1, 2, and SEQ ID NO:1-10). Among these ten C2c1 orthologs, C2c1 from D. inopinatus (DiC2c1) and T. calidus (TcC2c1) have neither predicted precursor CRISPR RNA (pre-crRNA) nor the reverse-repeated trans-activating crRNA (tracrRNA) (Fig. 1b), suggesting that these two C2c1 proteins might be unsuitable for genome-editing applications.

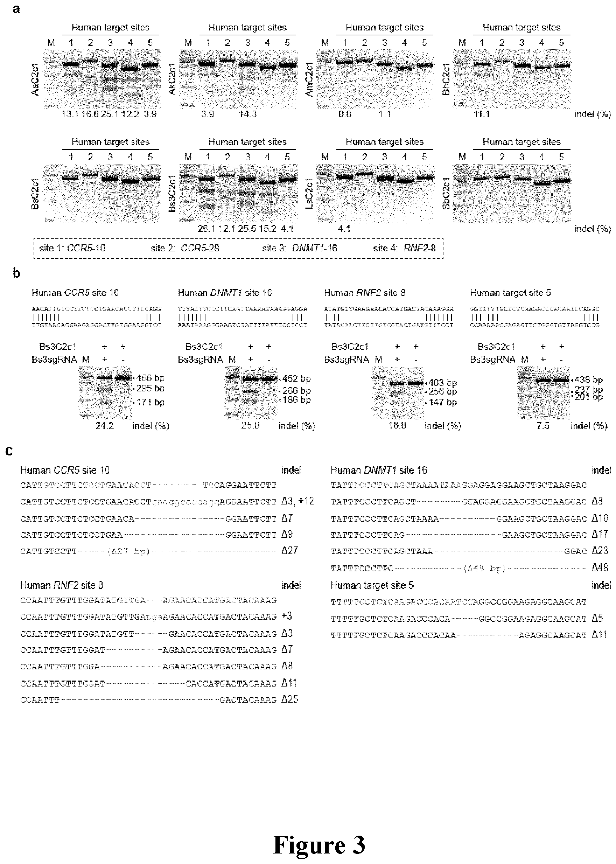

To conduct mammalian genome editing, we co-transfected 293T cells with individual C2c1 enzymes and their cognate chimeric single guide RNAs (sgRNAs) targeting human endogenous loci containing appropriate PAMs (Fig. 1). Results of T7 Endonuclease (T7EI) assay showed that apart from previously reported AaC2c1 and AkC2c1, four novel C2c1 orthologs (AmC2c1, BhC2c1, Bs3C2c1 and LsC2c1) could robustly edit the human genome, though their targeting efficiencies varied among different orthologs and at different targeting sites (Fig. 1b and Fig. 3a). We also achieved multiplex genome engineering by programming Bs3C2c1 to simultaneously edit four sites in the human genome by simply using multiple sgRNAs (Figs. 3b, c). These newly-identified C2c1 orthologs expand our options for C2c1-based genome engineering.

### Example 2. Exchangeability between different C2c1 and dual-RNA

To investigate the interchangeability between the dual-RNA and protein components in C2c1 systems, we first analyze the conservation of both C2c1 proteins and dual-RNAs. Besides the conserved amino acid sequences of C2c1 orthologs (Fig. 4a and Fig. 2), the DNA sequences of pre-crRNA:tracrRNA duplex and their secondary structures also exhibited high conservation (Figs. 1b and 5). Next, we conducted genome editing in 293T cells with the eight C2c1 orthologs complexed with respective sgRNAs from the eight C2c1 systems separately. As results of T7EI assay indicated, sgRNAs derived from AaC2c1, AkC2c1, AmC2c1, Bs3C2c1 and LsC2c1 loci could substitute the original sgRNAs for mammalian genome editing, though their activities varied between different C2c1 orthologs and sgRNAs (Figs. 4c, d and Fig. 6). These results demonstrated the exchangeability between C2c1 and dual-RNA from different C2c1 loci.

### Example 3. Using the C2c1 ortholog with no CRISPR array in natural loci for genome editing

To further demonstrate our hypothesis, we chose two C2c1 orthologs (DiC2c1 and TcC2c1) whose loci harbored no CRISPR array (Fig. 7a), making the sequences of their crRNA:tracrRNA duplex unpredictable, to conduct subsequent experiments. We co-transfected DiC2c1 and TcC2c1 as well as AaC2c1 combined with the sgRNAs derived from loci of other eight C2c1 orthologs targeting different genomic sites in 293T cells. T7EI assay results indicated that sgRNAs derived from AaC2c1, AkC2c1, AmC2c1, Bs3C2c1 and LsC2c1 could enable TcC2c1 to robustly edit the human genome (Figs. 7b, c and Fig. 8). Furthermore, TcC2c1 could facilitate multiplex genome editing simultaneously directed by either AasgRNAs or AksgRNAs (Fig. 7d and Fig. 9). These above results demonstrated that the interchangeability between C2c1 and dual-RNA from different systems could empower the C2c1 ortholog with no CRISPR array in natural loci to engineer the mammalian genomes.

### Example 4. Design artificial sgRNA for C2c1-mediated genome editing

The exchangeability between C2c1 and dual-RNA in different C2c1 systems further promoted us to design novel artificial sgRNA (artsgRNA) scaffolds to facilitate C2c1-mediated genome editing. Considering the conservation of DNA sequences and secondary structure among C2c1 orthologs (Figs. 1b and 3), we designed and de novo synthesized 37 sgRNA scaffolds targeting the human *CCR5* locus (Figs. 7e and 10a). The results of T7EI assay suggested that 22 artsgRNA scaffolds could work efficiently (Fig. 10b). To verify the generalization of our artsgRNAs, we leveraged multiplex genome editing using TcC2c1 or AaC2c1 directed by artsgRNA13 (Fig. 10a). T7EI assay results indicated that artsgRNA13 could simultaneously facilitate multiplex genome engineering with both TcC2c1 and AaC2c1 (Fig. 7f and Fig. 10c). Our results suggested that we could design and synthesize artsgRNAs to facilitate C2c1-mediated multiplex genome editing.

**Table 2 Sequences and information involved in the present invention**

| **SEQ ID NO** | **Information** |
|---|---|
| SEQ ID NO:1 | AaC2c1 amino acid sequence from Alicyclobacillus acidiphilus NBRC 100859 (GeneBank ID: NZ BCQI01000053.1) |
| SEQ ID NO:2 | AkC2c1 amino acid sequence from Alicyclobacillus kakegawensis NBRC 103104 (GeneBank ID: NZ BCRP01000027.1) |
| SEQ ID NO:3 | AmC2c1 amino acid sequence from Alicyclobacillus macrosporangiidus strain DSM 17980 (GeneBank ID: NZ FPBV01000001.1) |
| SEQ ID NO:4 | BhC2c1 amino acid sequence from Bacillus hisashii strain C4 (GeneBank ID: NZ NJGA01000060.1) |
| SEQ ID NO:5 | BsC2c1 amino acid sequence from Bacillus sp. NSP2.1 (GeneBank ID: NZ KI301973.1) |
| SEQ ID NO:6 | Bs3C2c1 amino acid sequence from Bacillus sp. V3-13 contig _40 (GeneBank ID: NZ PGUZ01000040.1) |
| SEQ ID NO:7 | DiC2c1 amino acid sequence from Desulfovibrio inopinatus DSM 10711 (GeneBank: NZ KE386879.1) |
| SEQ ID NO:8 | LsC2c1 amino acid sequence from Laceyella sediminis strain RHA1 (GeneBank ID: NZ PVTZ01000002.1) |
| SEQ ID NO:9 | SbC2c1 amino acid sequence from Spirochaetes bacterium GWB1_27_13 (GeneBank ID: MIAN01000063.1) |
| SEQ ID NO:10 | TcC2c1 amino acid sequence from Tuberibacillus calidus DSM 17572 (GeneBank ID: NZ KE387196.1) |
| SEQ ID NO:11 | Humanized AaC2c1 coding sequence from Alicyclobacillus acidiphilus |
| | NBRC 100859 (GeneBank ID: NZ BCQI01000053.1) |
| SEQ ID NO:12 | Humanized AkC2c1 coding sequence from Alicyclobacillus kakegawensis NBRC 103104 (GeneBank ID: NZ BCRP01000027.1) |
| SEQ ID NO:13 | Humanized AmC2c1 coding sequence from Alicyclobacillus macrosporangiidus strain DSM 17980 (GeneBank ID: NZ FPBV01000001.1) |
| SEQ ID NO:14 | Humanized BhC2c1 coding sequence from Bacillus hisashii strain C4 (GeneBank ID: NZ NJGA01000060.1) |
| SEQ ID NO:15 | Humanized BsC2c1 coding sequence from Bacillus sp. NSP2.1 (GeneBank ID: NZ KI301973.1) |
| SEQ ID NO:16 | Humanized Bs3C2c1 coding sequence from Bacillus sp. V3-13 contig _40 (GeneBank ID: NZ PGUZ01000040.1) |
| SEQ ID NO:17 | Humanized DiC2c1 coding sequence from Desulfovibrio inopinatus DSM 10711 (GeneBank: NZ KE386879.1) |
| SEQ ID NO:18 | Humanized LsC2c1 coding sequence from Laceyella sediminis strain RHA 1 (GeneBank ID: NZ PVTZ01000002.1) |
| SEQ ID NO:19 | Humanized SbC2c1 coding sequence from Spirochaetes bacterium GWB1 27 13 (GeneBank ID: MIAN01000063.1) |
| SEQ ID NO:20 | Humanized TcC2c1 coding sequence from Tuberibacillus calidus DSM 17572 (GeneBank ID: NZ KE387196.1) |
| SEQ ID NO:21 | pCAG-2AeGFP partial sequence (CAG- NLS- XmaI- Nhel- NLS- T2A-eGFP- SV40) |
| SEQ ID NO:22 | pUC19-U6 partial sequence (U6-BasI-HindIII) |
| SEQ ID NO:23 | pUC19-U6-AasgRNA partial sequence (U6-AasgRNA_ scaffold -BasI-BasI-terminator) |
| SEQ ID NO:24 | pUC19-U6-AksgRNA partial sequence (U6-AksgRNA1_scaffold-BasI-BasI-terminator) |
| SEQ ID NO:25 | pUC19-U6-AmsgRNA partial sequence (U6-AmsgRNAl_ scaffold-BasI-BasI- terminator) |
| SEQ ID NO:26 | pUC19-U6-BhsgRNA partial sequence (U6-BhsgRNA1_scaffold-BasI-BasI-terminator) |
| SEQ ID NO:27 | pUC19-U6-BssgRNA partial sequence (U6-BssgRNA1_scaffold-BasI-BasI-terminator) |
| SEQ ID NO:28 | pUC19-U6-Bs3sgRNA partial sequence (U6-BssgRNA1_scaffold- BasI-BasI- terminator) |
| SEQ ID NO:29 | pUC19-U6-LssgRNA partial sequence (U6-BssgRNA1_scaffold- BasI-BasI- terminator) |
| SEQ ID NO:30 | pUC19-U6-SbsgRNA partial sequence (U6-BssgRNA1_scaffold-BasI-BasI-terminator) |
| SEQ ID NO:31 | AasgRNA scaffold |
| SEQ ID NO:32 | AksgRNA1 scaffold |
| SEQ ID NO:33 | AmsgRNA1 scaffold |
| SEQ ID NO:34 | BhsgRNA scaffold |
| SEQ ID NO:35 | BssgRNA scaffold |
| SEQ ID NO:36 | Bs3sgRNA scaffold |
| SEQ ID NO:37 | LssgRNA scaffold |
| SEQ ID NO:38 | SbsgRNA scaffold |
| SEQ ID NO:39 | artsgRA1 scaffold |
| SEQ ID NO:40 | artsgRA2 scaffold |
| SEQ ID NO:41 | artsgRA3 scaffold |
| SEQ ID NO:42 | artsgRA4 scaffold |
| SEQ ID NO:43 | artsgRA5 scaffold |
| SEQ ID NO:44 | artsgRA6 scaffold |
| SEQ ID NO:45 | artsgRA7 scaffold |
| SEQ ID NO:46 | artsgRA8 scaffold |
| SEQ ID NO:47 | artsgRA9 scaffold |
| SEQ ID NO:48 | artsgRA10 scaffold |
| SEQ ID NO:49 | artsgRA11 scaffold |
| SEQ ID NO:50 | artsgRA12 scaffold |
| SEQ ID NO:51 | artsgRA13 scaffold |
| SEQ ID NO:52 | artsgRA14 scaffold |
| SEQ ID NO:53 | artsgRA15 scaffold |
| SEQ ID NO:54 | artsgRA16 scaffold |
| SEQ ID NO:55 | artsgRA17 scaffold |
| SEQ ID NO:56 | artsgRA18 scaffold |
| SEQ ID NO:57 | artsgRA19 scaffold |
| SEQ ID NO:58 | artsgRA20 scaffold |
| SEQ ID NO:59 | artsgRA21 scaffold |
| SEQ ID NO:60 | artsgRA22 scaffold |
| SEQ ID NO:61 | artsgRA23 scaffold |
| SEQ ID NO:62 | artsgRA24 scaffold |
| SEQ ID NO:63 | artsgRA25 scaffold |
| SEQ ID NO:64 | artsgRA26 scaffold |
| SEQ ID NO:65 | artsgRA27 scaffold |
| SEQ ID NO:66 | artsgRA28 scaffold |
| SEQ ID NO:67 | artsgRA29 scaffold |
| SEQ ID NO:68 | artsgRA30 scaffold |
| SEQ ID NO:69 | artsgRA31 scaffold |
| SEQ ID NO:70 | artsgRA32 scaffold |
| SEQ ID NO:71 | artsgRA33 scaffold |
| SEQ ID NO:72 | artsgRA34 scaffold |
| SEQ ID NO:73 | artsgRA35 scaffold |
| SEQ ID NO:74 | artsgRA36 scaffold |
| SEQ ID NO:75 | artsgRA37 scaffold |
| SEQ ID NO:76 | CCR5 site 10 |
| SEQ ID NO:77 | CCR5 site 28 |
| SEQ ID NO:78 | DNMT1 site 16 |
| SEQ ID NO:79 | RNF2 site 8 |
| SEQ ID NO:80 | Site 5 |

## Claims

1. A genome editing system for site-directed modification of a target sequence in the genome of a cell, comprising at least one of the following i) to v):
i) a C2c1 protein or variant thereof, and a guide RNA;
ii) an expression construct comprising a nucleotide sequence encoding a C2c1 protein or variant thereof, and a guide RNA;
iii) a C2c1 protein or variant thereof, and an expression construct comprising a nucleotide sequence encoding a guide RNA;
iv) an expression construct comprising a nucleotide sequence encoding a C2c1 protein or variant thereof, and an expression construct comprising a nucleotide sequence encoding a guide RNA;
v) an expression construct comprising a nucleotide sequence encoding a C2c1 protein or variant thereof and a nucleotide sequence encoding a guide RNA;
wherein the guide RNA is capable of forming complex with the C2c1 protein or variant thereof and targeting the C2c1 protein ortholog or variant thereof to the target sequence in the cell genome.

2. The system of claim 1, wherein the C2c1 protein is AaC2c1 protein derived from Alicyclobacillus acidiphilus, AkC2c1 protein derived from Alicyclobacillus kakegawensis, AmC2c1 protein derived from Alicyclobacillus macrosporangiidus, BhC2c1 protein derived from Bacillus hisashii, BsC2c1 protein derived from the genus Bacillus, Bs3C2c1 protein derived from the genus Bacillus, DiC2c1 protein derived from Desulfovibrio inopinatus, LsC2c1 protein derived from Laceyella sediminis, SbC2c1 protein derived from Spirochaetes bacterium, or TcC2c1 protein derived from Tuberibacillus calidus.

3. The system of claim 1, wherein the C2c1 protein is the AaC2c1 protein derived from Alicyclobacillus acidiphilus NBRC 100859, the AkC2c1 protein derived from Alicyclobacillus kakegawensis NBRC 103104, the AmC2c1 protein derived from Alicyclobacillus macrosporangiidus strain DSM 17980, the BhC2c1 protein derived from Bacillus hisashii strain C4, the BsC2c1 protein derived from genus Bacillus NSP2.1, the Bs3C2c1 protein derived from genus Bacillus V3-13 contig _40, the DiC2c1 protein derived from Desulfovibrio inopinatus DSM 10711, the LsC2c1 protein derived from Laceyella sediminis strain RHA1, the SbC2c1 protein derived from Spirochaetes bacterium GWB1_27_13, or the TcC2c1 protein derived from Tuberibacillus calidus DSM 17572.

4. The system of any one of claims 1-3, wherein the C2c1 protein comprises the amino acid sequence set forth in any one of SEQ ID NOs: 1-10, or the variant of the C2c1 protein comprises amino acid sequence having at least 80%, at least 85%, at least 90%, at least 95%, at least 96%, at least 97%, at least 98%, at least 99% sequence identity with the amino acid sequence set forth in any one of SEQ ID NOs: 1-10.

5. The system of any one of claims 1-4, wherein the guide RNA is an sgRNA.

6. The system of claim 5, the sgRNA comprises an sgRNA scaffold sequence encoded by the nucleotide sequence selected from SEQ ID NOs: 31-38 or 39-75.

7. The system of any one of claims 1-6, wherein the C2c1 protein is a C2c1 protein whose natural locus does not have a CRISPR array.

8. The system of any one of claims 1-7, wherein the nucleotide sequence encoding a C2c1 protein or variant thereof is codon optimized.

9. The system of claim 8, wherein the nucleotide sequence encoding the C2c1 protein or variant thereof is selected from SEQ ID NOs: 11-20.

10. A method of site-directed modifying a target sequence in the genome of a cell, comprising introducing the system of any one of claims 1-9 into the cell.

11. The method of claim 10, wherein the cell is derived from mammals such as human, mouse, rat, monkey, dog, pig, sheep, cattle, cat; poultry such as chicken, duck, goose; plants including monocots and dicots, such as rice, corn, wheat, sorghum, barley, soybean, peanut and Arabidopsis thaliana and so on.

12. The method of claim 10 or 11, wherein the system is introduced into the cell by a method selected from calcium phosphate transfection, protoplast fusion, electroporation, lipofection, microinjection, viral infection (e.g., baculovirus, vaccinia virus, adenovirus, adeno-associated virus, lentivirus and other viruses), gene gun method, PEG-mediated protoplast transformation, Agrobacterium-mediated transformation.

13. A method of treating a disease in a subject in need thereof, comprising delivering to the subject an effective amount of the genome editing system of any one of claims 1-9 to modify a gene related to the disease in the subject.

14. Use of the genome editing system of any one of claims 1-9 for the preparation of a pharmaceutical composition for treating a disease in a subject in need thereof, wherein the genome editing system is for modifying a gene related to the disease in the subject.

15. A pharmaceutical composition for treating a disease in a subject in need thereof, comprising the genome editing system of any one of claims 1-9 and a pharmaceutically acceptable carrier, wherein the genome editing system is for modifying a gene related to the disease in the subject.

16. The method, use or pharmaceutical composition of any one of claims 13-15, wherein the subject is a mammal, such as a human.

17. The method, use or pharmaceutical composition of claim 16, wherein the disease is selected from tumors, inflammation, Parkinson's disease, cardiovascular disease, Alzheimer's disease, autism, drug addiction, age-related macular degeneration, schizophrenia, and hereditary diseases.
